# EUROPEAN PATENT APPLICATION

(11) **EP 1 398 032 A1**
(43) Date of publication of application: **17.03.2004**
(21) Application number: 03020417.6
(22) Date of filing: 10.09.2003
(51) Int. Cl.: A61K 31/517, C07D 239/95, C07D 401/12, C07D 405/12, A61P 3/06

(54) **4-Oxo-quinazolines as LXR nuclear receptor binding compounds**

(30) Priority: 10.09.2002 EP 02020255
(71) Applicant: PheneX Pharmaceuticals AG, 69120 Heidelberg (DE)
(72) Inventor: Kober, Ingo, 69251 Gaiberg (DE); Albers, Michael, 69221 Dossenheim (DE); Koegl, Manfred, 69214 Eppelheim (DE); Blume, Beatrix, 69221 Dossenheim (DE); Deuschle, Ulrich, 69120 Heidelberg (DE); Kremoser, Claus, 69121 Heidelberg (DE)
(74) Representative: Huhn, Michael, Dr.

(57) **Abstract**

The present invention relates to 4-oxo-quinazolines which bind to the Liver X receptors (LXR receptors, LXRalpha/NR1 H3 and LXRbeta/NR1H2) and act as selective agonists of the LXR receptors. The invention further relates to the treatment of diseases and/or conditions through binding of said nuclear receptors and selective agonistic effects by said compounds and the production of medicaments using said compounds. In particular the compounds are useful in the treatment of hypercholesterimia, obesity or other diseases associated with elevated lipoprotein (LDL) levels.

## Description

Liver X Receptor (LXR) is a prototypical type 2 nuclear receptor which activates genes upon binding to promoter region of target genes in a prototypical heterodimeric fashion with Retinoid X Receptor (hereinafter RXR, Forman et al., Cell, 81, 687-93, 1995). The term LXR includes all subtypes of this receptor. Specifically LXR includes LXRa (also known as LXRalpha, RLD-1 and NR1H3) and LXRb (also known as LXRbeta, NER, NER1, UR, OR-1, R1P15 and NH1H2) and ligands of LXR should be understood to include ligands of LXRa or LXRb. The relevant physiological ligands of LXR seem to be oxidized derivatives of cholesterol, including 22-hydroxycholesterol and 24,25(S)-epoxycholesterol (Lehmann, et al., Biol. Chem. 272(6), 3137-40, 1997). The oxysterol ligands bound to LXR were found to regulate the expression of several genes that participate in cholesterol metabolism (Janowski, et al., Nature, 383, 728-31, 1996).

LXR is proposed to be a hepatic oxysterol sensor. Upon activation (e.g. binding of oxysterols) it influences the conversion of dietary cholesterol into bile acids by upregulating the transcription of key genes which are involved in bile acid synthesis such as CYP7A1. Hence, activation of LXR in the liver could result in an increased synthesis of bile acids from cholesterol which could lead to decreased levels of hepatic cholesterol. This proposed LXR function in hepatic cholesterol metabolism was experimentally confirmed using knockout mice. Mice lacking the receptor LXRa lost their ability to respond normally to an increase in dietary cholesterol and did not induce transcription of the gene encoding CYP7A1. This resulted in accumulation of large quantities of cholesterol in the livers and impaired hepatic function (Peet, et al., Cell, 93, 693-704, 1998).

Besides its important function in liver, LXR plays an important role in the regulation of cholesterol homeostasis in macrophages and intestinal mucosa cells where it upregulates cholesterol transporters from the ABC (=ATP binding cassette) family of membrane proteins (Repa, et al., J Biol Chem. 2002 May 24;277(21):18793-800).

These transporters are believed to be crucially involved in the uptake of cholesterol from the diet since mutations in their genes leads to diseases such as sitosterolemia (Berge, et al., Science (2000);290(5497):1771-5.).

Other members of the ABC transporter family seem to be responsible for the efflux of cholesterol from loaded macrophages, a process which is thought to prevent the generation of atherosclerotic lesions. Stimulation of LXR by synthetic ligands might result in an increased cholesterol efflux from macrophages and a decreased building up of cholesterol loaded atherosclerotic plaques (Venkateswaran, et al., PNAS (2000) 24;97(22):12097-102; Sparrow, et al., J Biol Chem (2002) 277(12):10021-7; Joseph, et al., PNAS (2002);99(11):7604-9). Direct evidence that synthetic LXR ligands inhibit the development of atherosclerosis has been provided in two animal models of atherosclerosis: A significant reduction in the formation of atherosclerotic plaques were shown in two studies in animal models using full LXR agonists Joseph et al. PNAS (2002) 99:7604-9 and Terasaka et al. (2003) Terasaka et al. FEBS Lett. (2003) 536:6-11. In addition, two recent reports have highlighted the potential use of LXR agonists in diabetes (Cao et al., (2003) J Biol Chem. 278:1131-6 and inflammatory disorders (Joseph et al., (2003) Nat Med. 9:213-9.

However, in animal studies it was observed that activation of LXR in the liver by full agonists like T0901317 does not only increase bile acid synthesis but also stimulates the de novo synthesis of fatty acids and triglycerids through the upregulation of key enzymes such as Fatty Acid Synthase (FAS) or Stearyl-CoA Desaturase (SCD-1) (Schultz, et al., Genes Dev (2000) 14(22):2831-8). Elevation of serum triglyceride levels is an indendent risk factor for atherosclerosis (for review see Miller (1999) Hosp Pract (Off Ed) 34: 67-73.).

Thus, LXR activity needs to be selectively modulated for therapeutic benefit. In particular, compounds need to be found that stimulate reverse cholesterol transport, but do not significantly increase trigclyceride levels. This might be particular relevant for the usage of such compounds in diabetic patients since a even more severe lopogenic effect was reported for the full agonist T0901317 in db/db mice which serve as an animal model for diabetes (Chisholm et al. (2003) J.Lipid Res (epub August 16)).

Therefore, an ideal synthetic LXR binding compound should have properties that retain the agonistic activity on hepatic bile acid formation and ABC-transporter-mediated decrease in cholesterol uptake from the diet and increased cholesterol efflux from macrophages. In parallel such a compound should lack the hyperlipidemic potential which is exerted through increased fatty acid and triclyceride synthesis.

To date only few compounds have been described which bind the LXR receptor and thus show utility for treating diseases or conditions which are due to or influenced by said nuclear receptor (Collins, et al., J Med Chem. (2002) 45(10):1963-6; Schultz, et al., Genes Dev (2000) 14(22):2831-8; Sparrow, et al., J Biol Chem (2002) 277(12):10021-7). No non-steroidal compounds have so far been described which show selectivity regarding the induction of ABC transporter genes without simultaneous induction of lipogenic genes like FAS and SREBP-1c (Kaneko et al. (2003) J Biol Chem (epub July 7).

It is thus an object of the invention to provide for compounds which by means of binding the LXR receptor act as partial agonists of said receptor with a selective property regarding the upregulation of genes like the ABC transporters in macrophages and/or other cell types and a stronlgy reduced liability to increase the expression of genes involved in triglyceride synthetic pathways (like FAS and SREBP-1c). These compounds should show utility for treating diseases or conditions which are due to or influenced by said nuclear receptor.

It is further an object of the invention to provide for compounds that may be used for the manufacture of a medicament for the treatment of cholesterol associated conditions or diseases. It is still a further object of the invention to provide for compounds that lower serum cholesterol and/or increase High Density Lipoproteins (HDL) and/or decrease Low Density Lipoproteins (LDL). It is also an object of the invention to provide for compounds that may be used for the treatment of lipid disorders including hypercholesterolemia, atherosclerosis, Alzheimer's disease, skin disorders, inflammation, obesity and diabetes.

The present invention provides, inter alia, novel LXR nuclear receptor protein binding compounds according to the general formula (I) shown below. Said compounds are also binders of mammalian homologues of said receptor. Further the object of the invention was solved by providing for amongst the LXR nuclear receptor protein binding compounds according to the general formula (I) such compounds which act as partial agonists or mixed agonists / antagonists of the human LXR receptor or a mammalian homologue thereof. Further the object of the invention was solved by providing for amongst the LXR receptor protein binding compounds according to the general formula (I) such compounds which act as partial agonists of the human LXR receptor resulting therefore in the induction of ABC transporter proteins such as ABCA1 or ABCG1 in cell types such as macrophages but lacking a strong potential to induce genes involved in triglyceride synthetic pathways such as fatty acid synthase (FAS) or SREBP1c.

The invention provides for LXR agonists that may be used for the manufacture of a medicament for the treatment of cholesterol associated conditions or diseases. In a preferred embodiment compounds are provided that lower serum cholesterol and/or increase High Density lipoproteins (HDL) and/or decrease Low Density Lipoproteins (LDL). Also compounds are provided that may be used for the treatment of lipid disorders including hypercholesterolemia, atherosclerosis, Alzheimer's disease, skin disorders, inflammation, obesity and diabetes.

The invention provides for a compound of the formula (I), or pharmaceutical acceptable salts or solvates thereof, hereinafter also referred to as the "compounds according to the invention" including particular and preferred embodiments thereof. 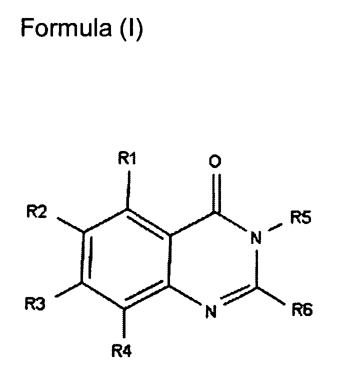 The compounds of the invention can also exist as solvates and hydrates. Thus, these compounds may crystallize with, for example, waters of hydration, or one, a number of, or any fraction thereof of molecules of the mother liquor solvent. The solvates and hydrates of such compounds are included within the scope of this invention.

In one embodiment of the invention in formula (I) above, R₁, R₂, R₃, R₄, - independent from each other - H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N-(phenylsulfonyl)amino or substituted or unsubstituted phenyl, for example a biphenyl,
R₅ is H, C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl
R₆ is H, a secondary or a tertiary amine, C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl

Formula (II) shows another embodiment of the invention wherein 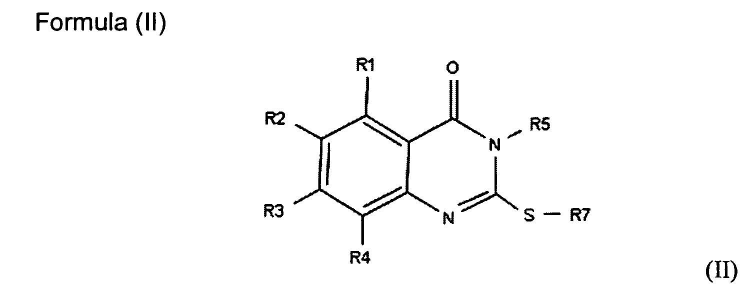 wherein R₇ is C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl

Another embodiment of the invention is described in formula (III) 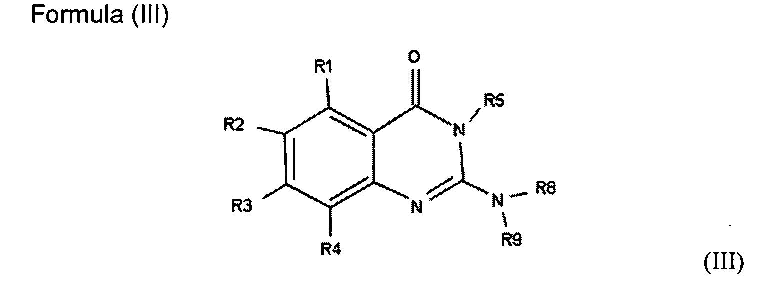
R₁, R₂, R₃, R₄, is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N-(phenylsulfonyl)amino or phenyl,
R₅ in formula (III) is H, C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl,
R₈ in formula (III) is H, C₁ to C₈ alkyl, C₁ to C₈ substituted alkyl, C₃ to C₈ cycloalkyl, C₃ to C₈ substituted cycloalkyl, C₁ to C₇ acyl, C₁ to C₇ substituted acyl, aryl, substituted aryl, C1 to C6 alkylphenyl or C₇ to C₁₂ substituted phenylalkyl, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₉ in formula (III) is H, C₁ to C₈ alkyl, C₁ to C₈ substituted alkyl, C₃ to C₈ cycloalkyl, C₃ to C₈ substituted cycloalkyl, C₁ to C₇ acyl, C₁ to C₇ substituted acyl, aryl, substituted aryl, C1 to C6 alkylphenyl or C₇ to C₁₂ substituted phenylalkyl, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, or
R₈ and R₉ in formula (III) are taken together with the N in formula (III) to form a ring of the following types:
   C₃ to C₈ mono, di- or tri- heteroatom-substituted cycloalkyl such as but not limited to piperidino, morpholino, thiomorpholino, piperazino, or C₁ to C₆ alkyl-, C₁ to C₆ substituted alkyl subtituted- or benzocondensated derivatives thereof
   C₄ to C₁₀ mono, di- or tri- heteroatom-substituted bi- or tricycloalkyl or C₁ to C₆ alkyl-, C₁ to C₆ substituted alkyl subtituted- or benzocondensated derivatives thereof
   C₃ to C₈ mono, di- or tri- heteroatom-substituted heteroaryl such as but not limited to pyrrolo, pyrazolo, oxazolo, thiazolo, pyridino, pyridazino, pyrimidino, pyrazino, triazino, indolo, quinolino, quinazolino or C₁ to C₆ alkyl-, C₁ to C₆ substituted alkyl subtituted- or benzocondensated derivatives thereof

In a preferred embodiment of the invention according to formula (III)
R₁ and R₃ are preferrably H,
R₂ and R₄ are preferrably H, or a halogen such as iodo, bromo, chloro or fluoro or hydroxy methyl, trifluoromethyl, methoxy, amino, alkylated amino, or nitro R₅ is H, C₁ to C₈ alkyl, preferrably isopropyl, vinyl, 2-isobutyl, or cyclohexyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl,
R₈ and R₉ in formula (III) are taken together with the N in formula (III) to form a ring of the following types:
   C₃ to C₈ mono, di- or tri- heteroatom-substituted cycloalkyl such as but not limited to piperidino, morpholino, thiomorpholino, piperazino, or C₁ to C₆ alkyl-, C₁ to C₆ substituted alkyl subtituted- or benzocondensated derivatives thereof, preferrably a 4-aryl- or 4-heteroaryl- or 4-alkyl-substituted piperazino or a 4-alkyl-substituted piperidino
   C₄ to C₁₀ mono, di- or tri- heteroatom-substituted bi- or tricycloalkyl or C₁ to C₆ alkyl-, C₁ to C₆ substituted alkyl subtituted- or benzocondensated derivatives thereof
   C₃ to C₈ mono, di- or tri- heteroatom-substituted heteroaryl such as but not limited to pyrrolo, pyrazolo, oxazolo, thiazolo, pyridino, pyridazino, pyrimidino, pyrazino, triazino, indolo, quinolino, quinazolino or C₁ to C₆ alkyl-, C₁ to C₆ substituted alkyl subtituted- or benzocondensated derivatives thereof

In another preferred embodiment of the invention according to formula (III)
R₁ and R₃ are preferrably H,
R₂ and R₄ are preferrably H, or a halogen such as iodo, bromo, chloro or fluoro or hydroxy methyl, trifluoromethyl, methoxy, amino, alkylated amino, or nitro
R₅ is H, C₁ to C₈ alkyl, preferrably isopropyl, vinyl, 2-isobutyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl,
R₈ is a C₁ to C₈ alkyl, C₁ to C₈ substituted alkyl, C₃ to C₈ cycloalkyl, C₃ to C₈ substituted cycloalkyl, C₁ to C₆ alkylphenyl or C₇ to C₁₂ substituted phenylalkyl, preferrably benzyl or (furan-2-yl)-methyl or (thiophen-2-yl)-methyl
and R₉ in formula is C₁ to C₈ alkyl, C₁ to C₈ substituted alkyl, C₃ to C₈ cycloalkyl, C₃ to C₈ substituted cycloalkyl, C₁ to C₆ alkylphenyl or C₇ to C₁₂ substituted phenylalkyl, preferably, however, (ethyl)-propion-1-yl

The symbol "H" denotes a hydrogen atom.

The term "C₁ to C₇ acyl" encompasses groups such as formyl, acetyl, propionyl, butyryl, pentanoyl, pivaloyl, hexanoyl, heptanoyl, benzoyl and the like. Preferred acyl groups are acetyl and benzoyl.

The term "C₁ to C₇ substituted acyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, C₁ to C₆ alkyl ester, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted acyl groups may be substituted once or more, and preferably once or twice, with the same or with different substituents.

The term "substituted phenyl" specifies a phenyl group substituted with one or more, and preferably one or two, moieties chosen from the groups consisting of halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, wherein the phenyl is substituted or unsubstituted, such that, for example, a biphenyl results.

Examples of the term "substituted phenyl" includes a mono- or di(halo)phenyl group such as 2, 3 or 4-chlorophenyl, 2,6-dichlorophenyl, 2,5-dichlorophenyl, 3,4-dichlorophenyl, 2, 3 or 4-bromophenyl, 3,4-dibromophenyl, 3-chloro-4-fluorophenyl, 2, 3 or 4-fluorophenyl and the like; a mono or di(hydroxy)phenyl group such as 2, 3 or 4-hydroxyphenyl, 2,4-dihydroxyphenyl, the protected-hydroxy derivatives thereof and the like; a nitrophenyl group such as 2, 3 or 4-nitrophenyl; a cyanophenyl group, for example, 2, 3 or 4-cyanophenyl; a mono- or di(alkyl)phenyl group such as 2, 3 or 4-methylphenyl, 2,4-dimethylphenyl, 2, 3 or 4-(iso-propyl)phenyl, 2, 3 or 4-ethylphenyl, 2, 3 or 4-(n-propyl)phenyl and the like; a mono or di(alkoxyl)phenyl group, for example, 2,6-dimethoxyphenyl, 2, 3 or 4-methoxyphenyl, 2, 3 or 4-ethoxyphenyl, 2, 3 or 4-(isopropoxy)phenyl, 2, 3 or 4-(t-butoxy)phenyl, 3-ethoxy-4-methoxyphenyl and the like; 2, 3 or 4-trifluoromethylphenyl; a mono- or dicarboxyphenyl or (protected carboxy)phenyl group such as 2, 3 or 4-carboxyphenyl or 2,4-di(protected carboxy)phenyl; a mono-or di(hydroxymethyl)phenyl or (protected hydroxymethyl)phenyl such as 2, 3, or 4-(protected hydroxymethyl)phenyl or 3,4-di(hydroxymethyl)phenyl; a mono- or di(aminomethyl)phenyl or (protected aminomethyl)phenyl such as 2, 3 or 4-(aminomethyl)phenyl or 2,4-(protected aminomethyl)phenyl; or a mono- or di(N-(methylsulfonylamino))phenyl such as 2, 3 or 4-(N-(methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy 4-chlorophenyl and the like.

The term "heteroaryl" means a heterocyclic aromatic derivative which is a five-membered or six-membered ring system having from 1 to 4 heteroatoms, such as oxygen, sulfur and/or nitrogen, in particular nitrogen, either alone or in conjunction with sulfur or oxygen ring atoms. Examples of heteroaryls include pyridinyl, pyrimidinyl, and pyrazinyl, pyridazinyl, pyrrolo, furano, thiopheno, oxazolo, isoxazolo, phthalimido, thiazolo and the like.

The term "substituted heteroaryl" means the above-described heteroaryl is substituted with, for example, one or more, and preferably one or two, substituents which are the same or different which substituents can be halogen, hydroxy, protected hydroxy, cyano, nitro, C₁ to C₆ alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino groups.

The term "substituted naphthyl" specifies a naphthyl group substituted with one or more, and preferably one or two, moieties either on the same ring or on different rings chosen from the groups consisting of halogen, hydroxy, protected hydroxy, cyano, nitro, C₁ to C₆ alkyl, C₁ to C₇ alkoxy, C₁ to C₇ acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino.

Examples of the term "substituted naphthyl" includes a mono or di(halo)naphthyl group such as 1, 2, 3, 4, 5, 6, 7 or 8-chloronaphthyl, 2, 6-dichloronaphthyl, 2, 5-dichloronaphthyl, 3, 4-dichloronaphthyl, 1, 2, 3, 4, 5, 6, 7 or 8-bromonaphthyl, 3, 4-dibromonaphthyl, 3-chloro-4-fluoronaphthyl, 1, 2, 3, 4, 5, 6, 7 or 8-fluoronaphthyl and the like; a mono or di(hydroxy)naphthyl group such as 1, 2, 3, 4, 5, 6, 7 or 8-hydroxynaphthyl, 2, 4-dihydroxynaphthyl, the protected-hydroxy derivatives thereof and the like; a nitronaphthyl group such as 3- or 4-nitronaphthyl; a cyanonaphthyl group, for example, 1, 2, 3, 4, 5, 6, 7 or 8-cyanonaphthyl; a mono- or di(alkyl)naphthyl group such as 2, 3, 4, 5, 6, 7 or 8-methylnaphthyl, 1, 2, 4-dimethylnaphthyl, 1, 2, 3, 4, 5, 6, 7 or 8-(isopropyl)naphthyl, 1, 2, 3, 4, 5, 6, 7 or 8-ethylnaphthyl, 1, 2, 3, 4, 5, 6, 7 or 8-(n-propyl)naphthyl and the like; a mono or di(alkoxy)naphthyl group, for example, 2, 6-dimethoxynaphthyl, 1, 2, 3, 4, 5, 6, 7 or 8-methoxynaphthyl, 1, 2, 3, 4, 5, 6, 7 or 8-ethoxynaphthyl, 1, 2, 3, 4, 5, 6, 7 or 8-(isopropoxy)naphthyl, 1, 2, 3, 4, 5, 6, 7 or 8-(t-butoxy)naphthyl, 3-ethoxy-4-methoxynaphthyl and the like; 1, 2, 3, 4, 5, 6, 7 or 8-trifluoromethylnaphthyl; a mono-or dicarboxynaphthyl or (protected carboxy)naphthyl group such as 1, 2, 3, 4, 5, 6, 7 or 8-carboxynaphthyl or 2, 4-di(-protected carboxy)naphthyl; a mono-or di(hydroxymethyl)naphthyl or (protected hydroxymethyl)naphthyl such as 1, 2, 3, 4, 5, 6, 7 or 8-(protected hydroxymethyl)naphthyl or 3, 4-di(hydroxymethyl)naphthyl; a mono- or di(amino)naphthyl or (protected amino)naphthyl such as 1, 2, 3, 4, 5, 6, 7 or 8-(amino)naphthyl or 2, 4-(protected amino)-naphthyl, a mono- or di(aminomethyl)naphthyl or (protected aminomethyl)naphthyl such as 2, 3, or 4-(aminomethyl)naphthyl or 2, 4-(protected aminomethyl)-naphthyl; or a mono- or di-(N-methylsulfonylamino) naphthyl such as 1, 2, 3, 4, 5, 6, 7 or 8-(N-methylsulfonylamino)naphthyl. Also, the term "substituted naphthyl" represents disubstituted naphthyl groups wherein the substituents are different, for example, 3-methyl-4-hydroxynaphth-1-yl, 3-chloro-4-hydroxynaphth-2-yl, 2-methoxy-4-bromonaphth-1-yl, 4-ethyl-2-hydroxynaphth-1-yl, 3-hydroxy-4-nitronaphth-2-yl, 2-hydroxy-4-chloronaphth-1-yl, 2-methoxy-7-bromonaphth-1-yl, 4-ethyl-5-hydroxynaphth-2-yl, 3-hydroxy-8-nitronaphth-2-yl, 2-hydroxy-5-chloronaphth-1-yl and the like.

The term "C₁ to C₈ alkyl" denotes such radicals as methyl, ethyl, n-propyl, isopropyl, n-butyl, iso-butyl, sec-butyl, tert-butyl, amyl, tert-amyl, hexyl , n-heptyl, 2-heptyl, 3-heptyl, 4-heptyl, 2-methyl-1hexyl, 2-methyl-2hexyl, 2-methyl-3-hexyl, n-octyl and the like.

The term "C₂ to C₆ alkenyl" denotes such radicals as propenyl or butenyl.

Examples of the above substituted alkyl groups include the 2-oxo-prop-1-yl, 3-oxo-but-1-yl, cyanomethyl, nitromethyl, chloromethyl, hydroxymethyl, tetrahydropyranyloxymethyl, trityloxymethyl, propionyloxymethyl, amino, methylamino, aminomethyl, dimethylamino, carboxymethyl, allyloxycarbonylmethyl, allyloxycarbonylaminomethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichloro(n-butyl), 2-aminopropyl, 1-chloroethyl, 2-chloroethyl, 1-bromoethyl, 2-chloroethyl, 1-fluoroethyl, 2-fluoroethyl, 1- iodoethyl, 2-iodoethyl, 1-chloropropyl, 2-chloropropyl, 3- chloropropyl, 1-bromopropyl, 2-bromopropyl, 3-bromopropyl, 1-fluoropropyl, 2-fluoropropyl, 3-fluoropropyl, 1- iodopropyl, 2-iodopropyl, 3-iodopropyl, 2-aminoethyl, 1- aminoethyl, N-benzoyl-2-aminoethyl, N-acetyl-2-aminoethyl, N-benzoyl-1-aminoethyl, N-acetyl-1-aminoethyl and the like.

The term "C₁ to C₈ substituted alkyl" denotes that the above C₁ to C₈ alkyl groups are substituted by one or more, and preferably one or two, halogen, hydroxy, protected hydroxy, oxo, protected oxo, C₃ to C₇ cycloalkyl, naphthyl, amino, protected amino, (monosubstituted)amino, protected (monosubstituted)amino, (disubstituted)amino, guanidino, protected guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted alkyl groups may be substituted once or more, and preferably once or twice, with the same or with different substituents.

The term "C₇ to C₁₂ phenylalkyl" denotes a C₁ to C₆ alkyl group substituted at any position by a phenyl, substituted phenyl, heteroaryl or substituted heteroaryl. Examples of such a group include benzyl, 2-phenylethyl, 3-phenyl(n-propyl), 4-phenylhexyl, 3-phenyl(n-amyl), 3-phenyl(sec-butyl) and the like. Preferred C₇ to C₁₂ phenylalkyl groups are the benzyl and the phenylethyl groups.
The term "C₇ to C₁₂ substituted phenylalkyl" denotes a C₇ to C₁₂ phenylalkyl group substituted on the C₁ to C₆ alkyl portion with one or more, and preferably one or two, groups chosen from halogen, hydroxy, protected hydroxy, oxo, protected oxo, amino, protected amino, (monosubstituted)amino, protected (monosubstituted)amino, (disubstituted)amino, guanidino, protected guanidino, heterocyclic ring, substituted heterocyclic ring, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N, N-(C₁ to C₆ dialkyl)carboxamide, cyano, N-(C₁ to C₆ alkylsulfonyl)amino, thiol, C₁ to C₄ alkylthio, C₁ to C₄ alkylsulfonyl groups; and/or the phenyl group may be substituted with one or more, and preferably one or two, substituents chosen from halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl) carboxamide, protected N-(C₁ to C₆ alkyl) carboxamide, N, N-di(C₁ to C₆ alkyl)carboxamide, trifluoromethyl, N-((C₁ to C₆ alkyl)sulfonyl)amino, N-(phenylsulfonyl)amino, cyclic C₂ to C₇ alkylene or a phenyl group, substituted or unsubstituted, for a resulting biphenyl group. The substituted alkyl or phenyl groups may be substituted with one or more, and preferably one or two, substituents which can be the same or different.

Examples of the term "C₇ to C₁₂ substituted phenylalkyl" include groups such as 2-phenyl-1-chloroethyl, 2-(4-methoxyphenyl)ethyl, 4-(2,6-dihydroxy phenyl)n-hexyl, 2-(5-cyano-3-methoxyphenyl)n-pentyl, 3-(2,6-dimethylphenyl)n-propyl, 4-chloro-3-aminobenzyl, 6-(4-methoxyphenyl)-3-carboxy(n-hexyl), 5-(4-aminomethylphenyl)- 3-(aminomethyl)n-pentyl, 5-phenyl-3-oxo-n-pent-1-yl and the like.

The term "heterocycle" or "heterocyclic ring" denotes optionally substituted five-membered to eight-membered rings that have 1 to 4 heteroatoms, such as oxygen, sulfur and/or nitrogen, in particular nitrogen, either alone or in conjunction with sulfur or oxygen ring atoms. These five-membered to eight-membered rings may be saturated, fully unsaturated or partially unsaturated, with fully saturated rings being preferred. Preferred heterocyclic rings include morpholino, piperidinyl, piperazinyl, 2-amino-imidazoyl, tetrahydrofurano, pyrrolo, tetrahydrothiophen-yl, hexylmethyleneimino and heptylmethyleneimino.

The term "substituted heterocycle" or "substituted heterocyclic ring" means the above-described heterocyclic ring is substituted with, for example, one or more, and preferably one or two, substituents which are the same or different which substituents can be halogen, hydroxy, protected hydroxy, cyano, nitro, C₁ to C₁₂ alkyl, C₁ to C₁₂ alkoxy, C₁ to C₁₂ substituted alkoxy, C₁ to C₁₂ acyl, C₁ to C₁₂ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino carboxamide, protected carboxamide, N-(C₁ to C₁₂ alkyl)carboxamide, protected N-(C₁ to C₁₂ alkyl)carboxamide, N, N-di(C₁ to C₁₂ alkyl)carboxamide, trifluoromethyl, N-((C₁ to C₁₂ alkyl)sulfonyl)amino, N-(phenylsulfonyl)amino, heterocycle or substituted heterocycle groups.

The term "C₁ to C₈ alkoxy" as used herein denotes groups such as methoxy, ethoxy, n-propoxy, isopropoxy, n-butoxy, t-butoxy and like groups. A preferred alkoxy is methoxy. The term "C₁ to C₈ substituted alkoxy" means the alkyl portion of the alkoxy can be substituted in the same manner as in relation to C₁ to C₈ substituted alkyl.

The term "C₁ to C₈ aminoacyl" encompasses groups such as formyl, acetyl, propionyl, butyryl, pentanoyl, pivaloyl, hexanoyl, heptanoyl, octanoyl, benzoyl and the like.

The term "C₁ to C₈ substituted aminoacyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₁₂ alkoxy, C₁ to C₁₂ acyl, C₁ to C₁₂ acyloxy, nitro, C₁ to C₁₂ alkyl ester, carboxy, protected carboxy, carbamoyl, carboxamide, protected carboxamide, N-(C₁ to C₁₂ alkyl)carboxamide, protected N-(C₁ to C₁₂ alkyl)carboxamide, N,N-di(C₁ to C₁₂ alkyl)carboxamide, cyano, methylsulfonylamino, thiol, C₁ to C₁₀ alkylthio or C₁ to C₁₀ alkylsulfonyl groups. The substituted acyl groups may be substituted once or more, and preferably once or twice, with the same or with different substituents.

Examples of C₁ to C₈ substituted acyl groups include 4-phenylbutyroyl, 3-phenylbutyroyl, 3-phenylpropanoyl, 2- cyclohexanylacetyl, cyclohexanecarbonyl, 2-furanoyl and 3-dimethylaminobenzoyl.

This invention also provides a pharmaceutical composition comprising an effective amount of a compound according to the invention. Such pharmaceuticals compositions can be administered by various routes, for example oral, subcutaneous, intramuscular, intravenous or intracerebral. The preferred route of administration would be oral at daily doses of the compound for adult human treatment of about 0.01-5000 mg, preferably 1-1500 mg per day. The appropriate dose may be administered in a single dose or as divided doses presented at appropriate intervals for example as two, three four or more subdoses per day.

For preparing pharmaceutical compositions containing compounds of the invention, inert, pharmaceutically acceptable carriers are used. The pharmaceutical carrier can be either solid or liquid. Solid form preparations include, for example, powders, tablets, dispersible granules, capsules, cachets, and suppositories.

A solid carrier can be one or more substances which can also act as diluents, flavoring agents, solubilizers, lubricants, suspending agents, binders, or tablet disintegrating agents; it can also be an encapsulating material.

In powders, the carrier is generally a finely divided solid which is in a mixture with the finely divided active component. In tablets, the active compound is mixed with the carrier having the necessary binding properties in suitable proportions and compacted in the shape and size desired.

For preparing pharmaceutical composition in the form of suppositories, a low-melting wax such as a mixture of fatty acid glycerides and cocoa butter is first melted and the active ingredient is dispersed therein by, for example, stirring. The molten homogeneous mixture is then poured into convenient-sized molds and allowed to cool and solidify.

Powders and tablets preferably contain between about 5% to about 70% by weight of the active ingredient, preferably comprising (especially consisting of) one or more of the compounds according to this invention. Suitable carriers include, for example, magnesium carbonate, magnesium stearate, talc, lactose, sugar, pectin, dextrin, starch, tragacanth, methyl cellulose, sodium carboxymethyl cellulose, a low-melting wax, cocoa butter and the like.

The pharmaceutical compositions can include the formulation of the active compound with encapsulating material as a carrier providing a capsule in which the active component (with or without other carriers) is surrounded by a carrier, which is thus in association with it. In a similar manner, cachets are also included. Tablets, powders, cachets, and capsules can be used as solid dosage forms suitable for oral administration.

Liquid pharmaceutical compositions include, for example, solutions suitable for oral or parenteral administration, or suspensions, and emulsions suitable for oral administration. Sterile water solutions of the active component or sterile solutions of the active component in solvents comprising water, ethanol, or propylene glycol are examples of liquid compositions suitable for parenteral administration.

Sterile solutions can be prepared by dissolving the active component in the desired solvent system, and then passing the resulting solution through a membrane filter to sterilize it or, alternatively, by dissolving the sterile compound in a previously sterilized solvent under sterile conditions.

In a preferred embodiment of the invention in the compounds claimed, or the pharmaceutical acceptable salts or solvates thereof, R₁, R₃ and R₄ are H, R₂ is halogen and preferably iodine over bromine and chlorine and R₅ is H, C₁ to C₈ alkyl or C₁ to C₈ substituted alkyl.

For the six preferred compounds as shown in formulae (XIII), (XII), (X), (IX), (VII), and (IV) below it was demonstrated that these compounds may act as a full or partial agonist of LXR
Formula (XIII); MOLNAME LN7181 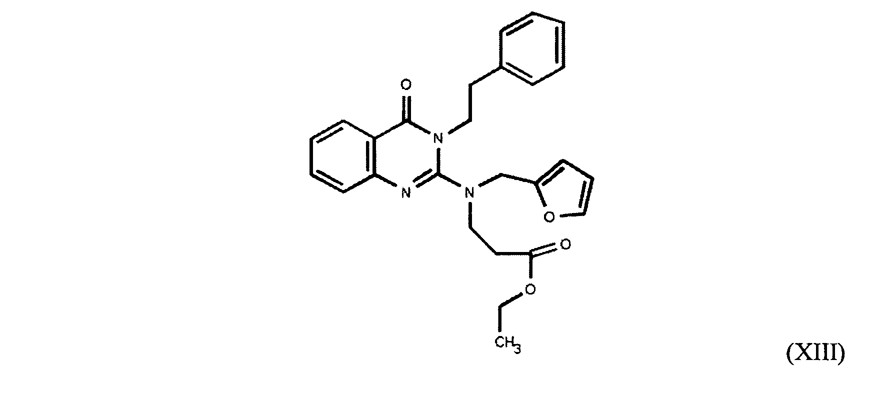 Formula (XII); MOLNAME LN7179 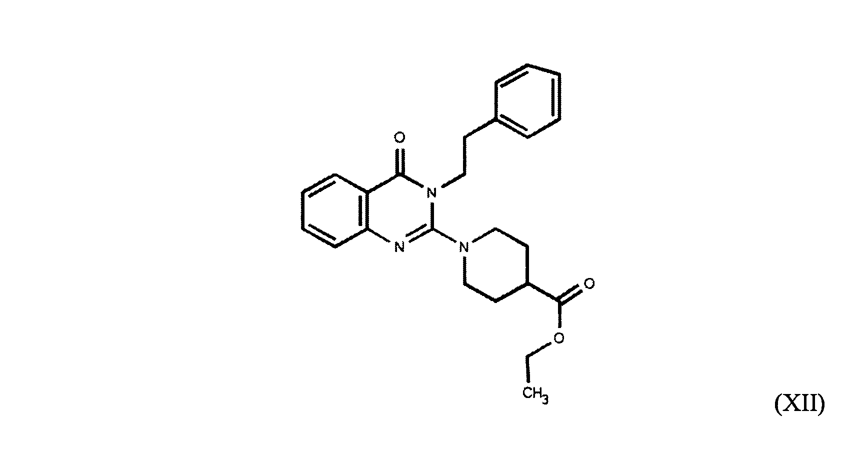 Formula (X); MOLNAME LN7172 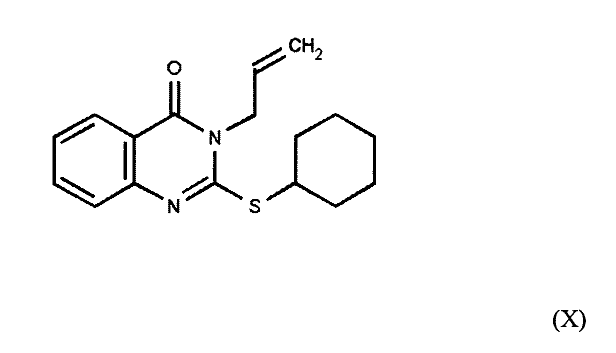 Formula (IX); MOLNAME LN6672 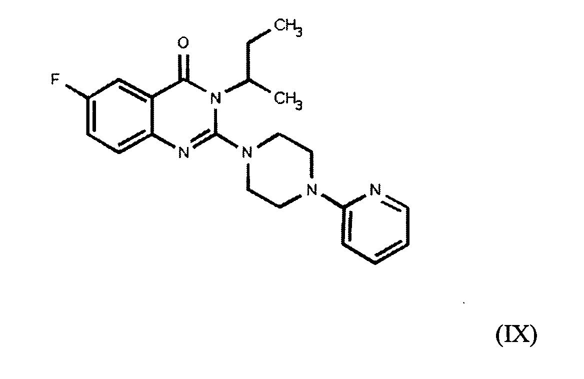 Formula (VII); MOLNAME LN7031 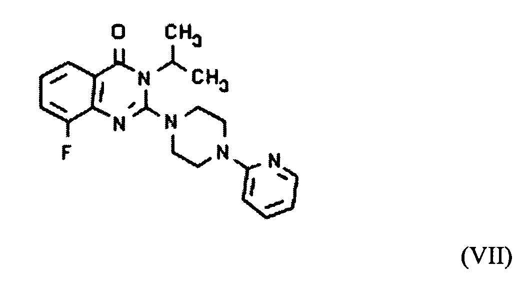 Formula (IV); MOLNAME LN7033

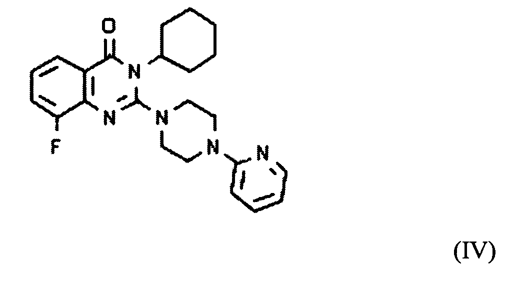

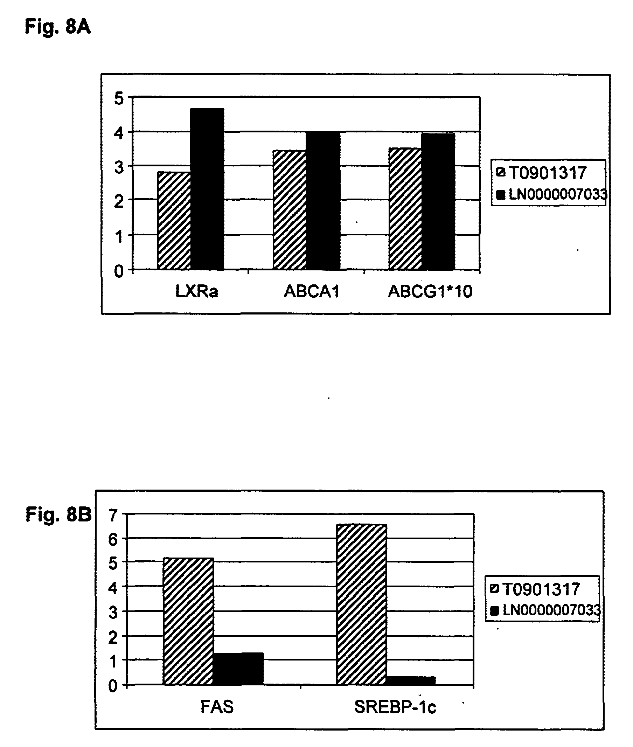

It has also been found that the compound according to formula (IV) (MOLNAME LN7033) to be active as partial agonist of the LXR human nuclear receptors with a selective upregulation of the target genes ABCA1 and ABCG1 in THP-1 cells compared to FAS and SREBP-1c in HepG2 cells (see also Fig. 8A and 8B).

Furthermore, the present invention includes compounds of the following formula:
Formula (V), MOLNAME LN7025 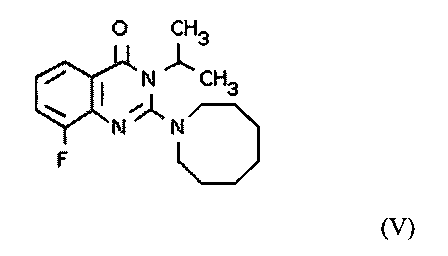 Formula (VI), MOLNAME LN7184 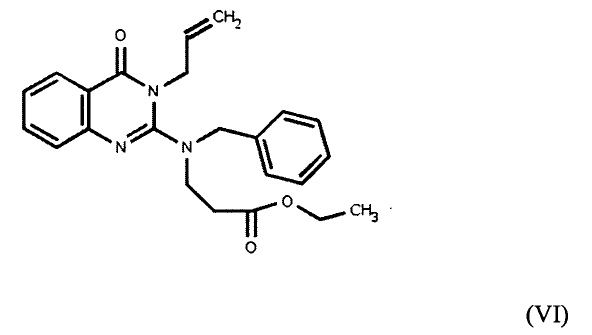 Formula (VIII); MOLNAME LN7032 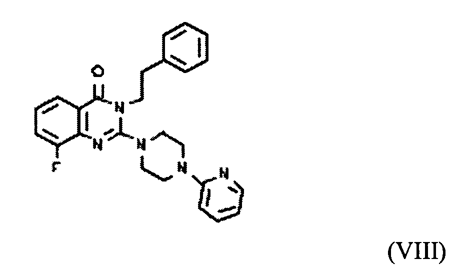 Formula (XI); MOLNAME LN7174

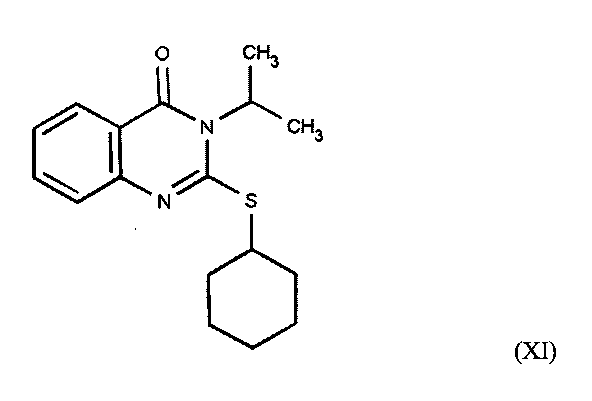

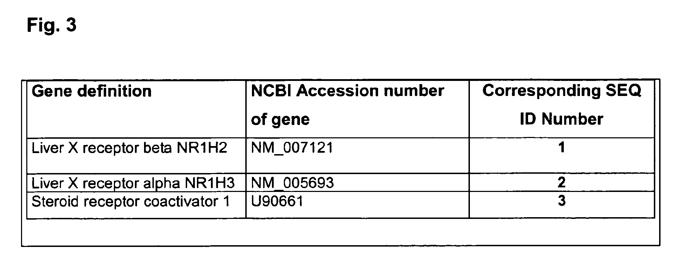

In particular the invention relates to a compound as described above wherein said compounds is capable of binding the LXR receptor protein or a portion thereof encoded by a nucleic acid according to SEQ ID NO:1 or NO:2 (Fig. 3) or a mammalian homologue thereof. This compound can bind to the LXR receptor protein or a portion thereof in a mixture comprising 10-200 ng of LXR receptor protein, a fusion protein containing LXR or a portion thereof, preferably the ligand binding domain, fused to a Tag, 5-100 mM Tris/HCl at pH 6,8-8,3; 60-1000 mM KCI; 0-20 mM MgCl2; 100-1000ng/µl BSA in a total volume of preferably about 25 µl).

A mammalian receptor protein homologue of the protein encoded by a nucleic acid according to SEQ ID NO:1 or 2, as used herein is a protein that performs substantially the same task as LXR does in humans and shares at least 40% sequence identity at the amino acid level, preferably over 50 % sequence identity at the amino acid level more preferably over 65 % sequence identity at the amino acid level, even more preferably over 75 % sequence identity at the amino acid level and most preferably over 85 % sequence identity at the amino acid level.

The invention in particular concerns a method for prevention or treatment of a LXR receptor protein or LXR receptor protein homologue mediated disease or condition in a mammal comprising administration of a therapeutically effective amount of a compound according to the invention wherein the prevention or treatment is directly or indirectly accomplished through the binding of a compound according to the invention to the LXR receptor protein or to the LXR receptor protein homologue.

The term mediated herein means that the physiological pathway in which the LXR receptor protein acts is either directly or indirectly involved in the disease or condition to be treated or prevented. In the case where it is indirectly involved it could be that, e.g. modulating the activity of LXR by a compound according to the invention influences a parameter which has a beneficial effect on a disease or a condition. One such example is that modulation of LXR activity leads to decreased levels of serum cholesterol or certain lipoproteins which in turn have a beneficial effect on the prevention and treatment of atherosclerosis. Herein a condition is a physiological or phenotypic state which is desirably altered. One such example would be obesity which is not necessarily medically harmful but nonetheless a non desirable phenotypic condition. In a preferred embodiment of the invention the method for prevention or treatment of a LXR receptor protein mediated disease or condition is applied to a human. This may be male or female.

Pharmaceutical compositions generally are administered in an amount effective for treatment or prophylaxis of a specific condition or conditions. Initial dosing in human is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, doses is from about 100 µg/kg to about 5 mg/kg body weight, daily.

For administration particularly to mammals, and particularly humans, it is expected that the daily dosage level of active agent will be 0,1 mg/kg to 10 mg/kg and typically around 1 mg/kg.

By "therapeutically effective amount" is meant a symptom-alleviating or symptom-reducing amount, a cholesterol-reducing amount, a cholesterol absorption blocking amount, a protein and/or carbohydrate digestion-blocking amount and/or a de novo cholesterol biosynthesis-blocking amount of a compound according to the invention.

Likewise the invention concerns a method of treating in mammal a disease which is correlated with abnormal cholesterol, triglyceride, or bile acid levels or deposits comprising administering to a mammal in need of such treatment a therapeutically effective amount of a compound according to the invention.

Accordingly, the compounds according to the invention may also be used in a method of prevention or treatment of mammalian atherosclerosis, gallstone disease, lipid disorders, Alzheimer's disease, skin disorders, inflammation, obesity or cardiovascular disorders such as coronary heart disease or stroke.

The invention further concerns a method of blocking in a mammal the cholesterol absorption in the intestine in need of such blocking comprising administering to a mammal in need of such treatment a therapeutically effective amount of a compound according to the invention. The invention may also be used to treat obesity in humans.

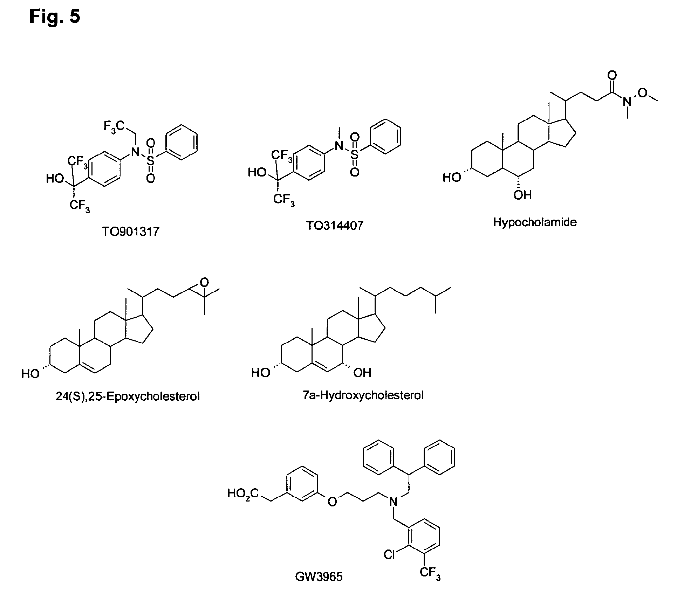

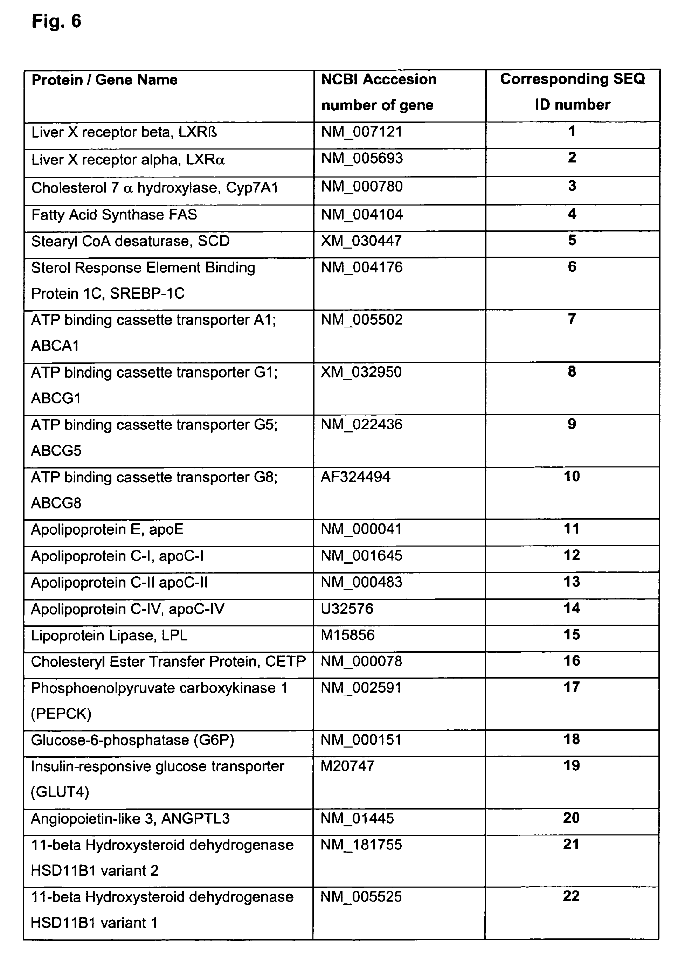

The Liver X Receptor alpha is a prototypical type 2 nuclear receptor meaning that it activates genes upon binding to the promoter region of target genes in a heterodimeric fashion with Retinoid X Receptor. The relevant physiological ligands of LXR are oxysterols. The compounds have been demonstrated to have a high binding efficacy (binding coefficients measured as EC50 in the range of 1-5 µM) as well as agonistic and/or partial agonistic properties. Consequently they may be applied to regulate genes that participate in bile acid, cholesterol and fatty acid homeostasis as well as other downstream regulated genes. Examples of such genes are but are not limited to lipid absorption, cholesterol biosynthesis, cholesterol transport or binding, bile acid transport or binding, proteolysis, amino acid metabolism, glucose biosynthesis, protein translation, electron transport, and hepatic fatty acid metabolism. LXR often functions in vivo as a heterodimer with the Retinoid X Receptor. Published non-steroidal LXR agonists such as the "Tularik" compound "TO901317" (see figure 5) are known to influence the regulation of various liver genes. Genes found to be regulated by T0901317 can be found in figure 6. Thus, the invention also concerns a method of modulating a gene whose expression is regulated by the LXR receptor in a mammal comprising administration of a therapeutically effective amount of a compound according to the invention to said mammal.

A number of direct and indirect LXR target genes have been described whose regulated expression contributes to cholesterol homeostasis and lipogenesis. In this respect the direct regulation of Cyp7A, which was shown to be a direct target gene of LXR at least in the rodent lineage is an important aspect of cholesterol removal by increased metabolism of bile acids (Lehmann et al., J Biol.Chem. 272 (6) 3137-3140; 1007). Gupta et al. (Biochem. Biophys Res.Com, 293; 338-343, 2002) showed that LXR α regulation of Cyp7A is dominant over FXR inhibitory effects on Cyp7A transcription.

A key transcription factor that was also shown to be a direct target gene for the LXR receptor is SREBP-1 C (Repa et al., Genes and Development, 14:2819-2830; 2000: Yoshikawa et al.; Mol.Cell.Biol.21 (9) 2991-3000, 2001). SREBP-1 C itself activates transcription of genes involved in cholesterol and fatty acid synthesis in liver but also other mammalian tissues. Some of the SREBP1c target genes involved in lipogenesis like FAS and SCD have shown to be additionally direct targets of the LXR receptors (Joseph et al.; J Biol Chem. 2002 Mar 29;277(13):11019-25; Liang et al., J Biol Chem. 2002 Mar 15;277(11):9520-8.).

A primary limitation for the applicability of LXR agonists as e.g. anti-atherosclerotic drugs comes from the observation that compounds with full agonistic activity, e.g. T0901317, not only elevate HDL cholesterol levels but do also increase plasma triglyceride levels in mice (Schultz et al., 2000 Genes Dev. 14:2831-8.). Concommitantly, not only genes that are involved in cholesterol efflux such as the cholesterol transporter ABCA1 (Venkateswaran et al., 2000 PNAS. 97:12097-102.), ABCG1 as well as the lipid binding protein Apoliprotein E (Laffite et al. 2001 PNAS 98:507-512) are induced by full LXR agonists, but also genes involved in lipogenesis, including the fatty acid synthase FAS (Joseph et al 2002 J Biol Chem. 277:11019-11025), and SREB P-1c (Yoshikawa et al., 2001 Mol Cell Biol. 21:2991-3000). Elevation of serum triglyceride levels is an indendent risk factor for atherosclerosis (for review see Miller, 1999). Thus, LXR activity needs to be selectively modulated for therapeutic benefit. In particular, compounds need to be found that stimulate reverse cholesterol transport, but do not significantly increase trigclyceride levels.

Another gene that has been shown to be directly regulated by LXRs is the LPL gene, that codes for a key enzyme that is responsible for the hydrolysis of triglycerides in circulating lipoprotein, releasing free fatty acids to peripheral tissues. (Zhang et al. J Biol Chem. 2001 Nov 16;276(46):43018-24.) This enzyme is believed to promote uptake of HDL cholesterol in liver, thereby promoting reverse cholesterol transport. A similar functional involvement in HDL clearance is described for the CETP gene product that facilitated the transfer of HDL cholesterol esters from plasma to the liver. LXR response elements were found in the CETP promoter and direct activation of this gene by LXR was demonstrated (Luo and Tall; J Clin Invest. 2000 Feb;105(4):513-20.).

The regulated transport of cholesterol through biological membranes is an important mechanism in order to maintain cholesterol homeostasis. A pivotal role in these processes in multiple tissues like e.g. macrophages and intestinal mucosa cells is maintained by the ATP-binding cassette transporter proteins (ABC). ABCA1 and ABCG1 were identified as direct LXR target genes (Costet et al.; J Biol Chem. 2000 Sep 8;275(36):28240-5) that mediate cholesterol efflux and prevent thereby e.g. generation of artherogenic plaques in macrophages (Singaraja et al. J Clin Invest. 2002 Jul;110(1):35-42). Other ABC transporters like ABCG5 and ABCG8, primarily expressed in hepatocytes and enterocytes have also been reported to be directly responsive to LXR agonists ( Repa et al., J Biol Chem. 2002 May 24;277(21):18793-800. Kennedy et al., J Biol Chem. 2001 Oct 19;276(42):39438-47) and mediate the secretion of sterols from the liver and efflux of dietary sterols from the gut. Apolipoproteins E, C-I, C-II, and C-IV, that fulfill important roles in lipoprotein/lipid homeostasis have also been shown to be direct targets of the LXR receptor ( Laffitte et al., Proc Natl Acad Sci U S A. 2001 Jan 16;98(2):507-12; Mak et al.; J Biol Chem. 2002 May 24 [epub ahead of print]). These proteins have been found to be crucial components of chylomicrons, VLDL, IDL, and HDL and are among other things associated with hypertriglyceridemia and arteriosclerosis.

Recently the LXRα itself was shown to be regulated by both LXR receptors in human cell types including macrophages suggesting an autoregulatory amplification event in the response to LXR ligands which could e.g. lead to an enhanced stimulation of LXR target genes like e.g. ABCA1 (Bolten et al.; Mol Endocrinol. 2002 Mar;16(3):506-14.; Laffitte et al., Mol Cell Biol. 2001 Nov;21 (22):7558-68; Whitney et al.; J Biol Chem. 2001 Nov 23;276(47):43509-15).

Besides the important function of LXR receptors in tissues like liver and macrophages it has recently been reported that that stimulation of epidermal differentiation is mediated by Liver X receptors in murine epidermis. Differentiation maker genes like involucrin, loricin and profilaggrin have been shown to be upregulated upon LXR ligand treatment (Kömüves et al.; J Invest Dermatol. 2002 Jan;118(1):25-34.).

Another recent report describes the regulation of cholesterol homeostasis (primarily the regulation of ABCA1, ABCG1 and SREBP-1C) by the LXR receptors in the central nervous system suggesting that LXRs may prove benefical in the treatment of CNS diseases such as Alzheimer's and Niemann-Pick disease that are known to be accompanied by dysregulation of cholesterol balance (Whitney et al.; Mol Endocrinol. 2002 Jun;16(6):1378-85).

Activation of LXR by an agonist improves glucose tolerance in a murine model of diet-induced obesity and insulin resistance. Gene expression analysis in LXR agonist-treated mice reveals coordinate regulation of genes involved in glucose metabolism in liver and adipose tissue, e.g. the down-regulation of peroxisome proliferator-activated receptor gamma coactivator-1 alpha (PGC-1), phosphoenolpyruvate carboxykinase (PEPCK), and glucose-6-phosphatase expression and induction of glucokinase in liver. In adipose tissue, activation of LXR led to the transcriptional induction of the insulin-sensitive glucose transporter, GLUT4. LXR agonist may limit hepatic glucose output and improve peripheral glucose uptake (Laffitte et al. (2003) PNAS 100:5419-24).

Therefore one other important embodiment of the invention concerns methods that enhances or suppresses amongst other today yet unknown LXR target genes the above mentioned genes and the associated biological processes and pathways through LXR compounds that are subject of this invention.

The compounds according to the invention may be used as medicaments, in particular for the manufacture of a medicament for the prevention or treatment of a LXR receptor protein or LXR receptor protein homologue mediated disease or condition in a mammal wherein the prevention or treatment is directly or indirectly accomplished through the binding of the compound according to the invention to the LXR receptor protein or LXR receptor protein homologue. These pharmaceutical compositions contain 0,1 % to 99,5 % of the compound according to the invention, more particularly 0,5 % to 90 % of the compound according to the invention in combination with a pharmaceutically acceptable carrier.

The invention concerns also the use of a compound according to the invention for the manufacture of a medicament for the prevention or treatment of a LXR receptor protein mediated disease or condition wherein the mammal described above is a human. The medicament may be used for regulating the cholesterol transport system, for regulating levels of cholesterol, triglyceride, and/or bile acid in a mammal preferentially a human by activating the LXR receptor. The medicament may be used for the treatment of atherosclerosis, gallstone disease, lipid disorders, Alzherimer's disease, skin disorders, obesity or a cardiovascular disorder.

The invention further concerns the use of a compound according to the invention for the manufacture of a medicament capable for blocking in a mammal, preferentially in a human the cholesterol absorption in the intestine. Further the claimed compound may be used for the manufacture of a medicament for treating obesity in humans and for modulating a gene whose expression is regulated by the LXR receptor (see details above and figures).

### EXAMPLES

### EXAMPLE 1

In vitro screening for compounds which influence LXR binding to coactivators.

For screening purposes a GST and 6 x His fusion of the LBD (from amino acids 155 of hLXRalpha to 447) of human LXRalpha is constructed by first cloning a Gateway cassette (Invitrogen) in frame into the Sma I site of the pAGGHLT Polylinker (Pharmingen). Then a PCR fragment specifically amplified from human liver cDNA is cloned into the resulting pACGHLT-GW following the manufacturers instructions for Gateway cloning (Invitrogen) to yield pACGHLT-GW-hLXRalphaLBD.
Primers used for Amplification are: GGGGACAAGTTTGTACAAAAAAGCAGGCTCGCTTCGCAAATGCCGTCAG and GGGGACCACTTTGTACAAGAAAGCTGGGTCCCCTTCTCAGTCTGTTCCACTT . 100 % sequence integrity of all recombinant products is verified by sequencing. Recombinant Baculovirus is constructed from pACGHLT-GW-hLXRalphaLBD using the Pharmingen Baculovirus Expression vector system according to instructions of the manufacturer. Monolayer cultures of SF9 cells are infected by the virus as recommended by Pharmingen or 200ml cultures of 1 x10⁶ cells/ml grown in 2 liter Erlenmeyer flasks on an orbital shaker at 30 rpm are infected by 10ml of same virus stock. In both cases cells are harvested 3 days after infection. All cell growth is performed in Gibco SF900 II with Glutamine (Invitrogen) medium without serum supplementation at 28°C. Since SF9 cells contain significant amounts of endogenous GST, purification is performed via His and not via GST affinity chromatography. To this end instructions of Pharmingen for purification of recombinant His tagged proteins from SF9 cells are followed with the following modifications: All detergents are omitted from the buffers and cells were lysed on ice by 5 subsequent sonication pulses using a sonicator needle at maximum power. All eluates are dialyzed against 20 mM Tris/HCl pH 6,8, 300 mM KCI; 5 mM MgCl₂; 1 mM DTT; 0,2 mM PMSF; 10% Glycerol. A typical dialyzed eluate fraction contains the fusion protein at a purity of more than 80%. Total protein concentration is 0,1-0,3 mg/ml.
For E. coli expression of a NR coactivator, pDest17-hTif2BD expressing a NR interaction domain from amino acids 548-878 of human Tif2 (Acc. No: XM_011633 RefSeq) tagged by 6 N-terminal His residues is constructed. Therefore, a PCR fragment specifically amplified from human liver cDNA is subcloned into pDest 17 (Invitrogen) following the manufacturers instructions for Gateway cloning (Invitrogen).Primers used for Amplification are: GGGGACAAGTTTGTACAAAAAAGCAGGCTCGTTAGGGTCATCGTTGGCTTCACC and 
GGGGACCACTTTGTACAAGAAAGCTGGGTCTCAAAGTTGCCCTGGTCGTGGGT
TA
 For E. coli expression plasmid DNA is transformed into chemically competent E. coli BL21 (Invitrogen, USA) and cells are grown to an OD600 of 0.4-0.7 before expression was induced by addition of 0,5 mM IPTG according instructions of the manufacturer (Invitrogen). After induction for 8 hours at 30°C cells are harvested by centrifugation for 10 minutes at 5000 x g. Fusion proteins are affinity purified using Ni-NTA Agarose (QIAGEN) according to the instructions of the manufacturer. Recombinant Tif2 construct is dialyzed against 20 mM Tris/HCL pH 7.9; 60 mM KCI; 5 mM MgCl₂; 1 mM DTT, 0,2 mM PMSF; 10% glycerol. A typical dialyzed eluate fraction contains the fusion protein at a purity of more than 80%. Total protein concentration is 0,1-0,3 mg/ml.
The TIF2 fragment is subsequently biotinylated by addition of 5-40µl/ml Tif2 fraction of a Biotinamidocaproate N-Hydroxysuccinimide-ester (Sigma) solution (20 mg/ml in DMSO). Overhead rotating samples are incubated for 2 hours at room temperature. Unincorporated label is then separated using G25 Gel filtration chromatography (Pharmacia Biotech, Sweden). Protein containing fractions from the column are pooled and tested for activity in the assay as described below.

For screening of compound libraries as provided for by the methods shown below in the examples for substances which influence the LXR/Tif 2 interaction, the Perkin Elmer LANCE technology is applied. This method relies on the binding dependent energy transfer from a donor to an acceptor fluorophore attached to the binding partners of interest. For ease of handling and reduction of background from compound fluorescence LANCE technology makes use of generic fluorophore labels and time resoved detection (for detailed description see Hemmilä I, Blomberg K and Hurskainen P, Time-resolved resonance energy transfer (TR-FRET) principle in LANCE, Abstract of Papers Presented at the 3 rd Annual Conference of the Society for Biomolecular Screening, Sep., California (1997))
For screening, 20-200 ng of biotinylated Tif 2 fragment and 10-200 ng of GST-LXR fragment are combined with 0.5-2 nM LANCE Eu-(W1024) labelled anti-GST antibody (Perkin Elmer) and 0,1-0,5µg of highly fluorescent APC-labelled streptavidin (Perkin Elmer, AD0059) in the presence of 50µM of individual compounds to be screened in a total volume of 25 µl of 20 mM Tris /HCl pH 6,8; 300 mM KCI; 5 mM MgCl2; 100-1000 ng/µl/ BSA DMSO content of the samples is kept below 4%. Samples are incubated for a minimum of 60 minutes in the dark at room temperature in FIA-Plates black 384well med. binding (Greiner).

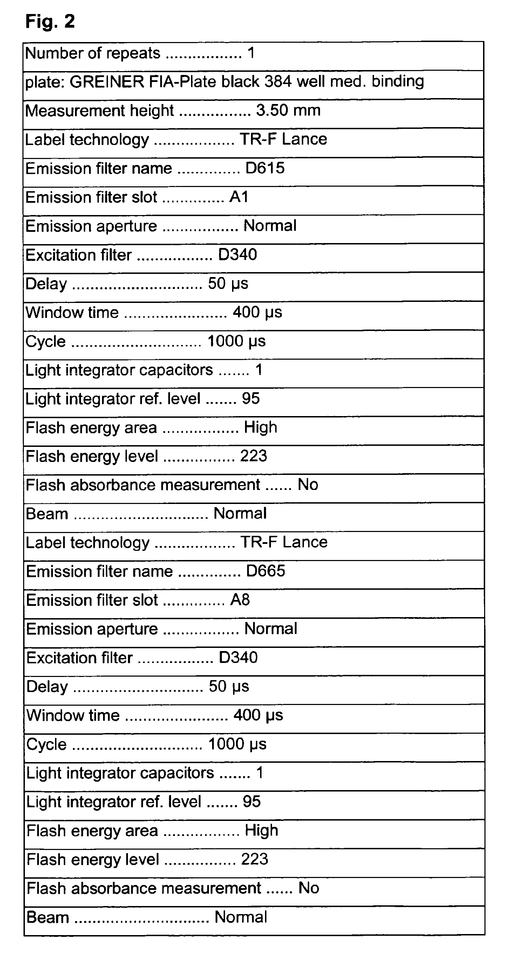

The LANCE signal is detected by a Perkin Elmer VICTOR2V™ Multilabel Counter applying the detection parameters listed in Fig. 2. The results are visualized by plotting the ratio between the emitted light at 665 nm and at 615 nm. For every batch of recombinant proteins amount of proteins, including BSA and labeling reagents giving the most sensitive detection of hits is determined individually by analysis of dose response curves for 22R Hydroxycholesterol and TO 901317

### EXAMPLE 2

Experimental procedure for the preparation of the compounds according to the invention.

### o-AZIDOBENZOIC ACID SYNTHESIS (2)

The anthranilic acid (1, 1 eq., 0.5-1 M) is suspended in 6 M HCl, containing enough AcOH (0-20% dependent upon the anthranilic acid) to facilitate dissolution of the anthranilic acid and/or the intermediate diazonium salt, and cooled to 0 °C. NaNO₂ (1.1 eq., 1.3-2.5 M) dissolved in H₂O is added to the anthranilic acid solution at a rate such that the temperature of the reaction solution remains below 5 °C. The resulting homogeneous solution of the diazonium salt is slowly filtered through a sintered glass funnel into a solution of NaN₃ (1.1 eq., 0.7-1.1 M) and NaOAc (12 eq.) in H₂O. The reaction mixture is stirred/shaken for 30-60 min following cessation of vigorous N₂ evolution. Following acidification of the reaction mixture to pH 1 with concentrated HCl, the mixture was cooled to 0 °C to encourage complete precipitation of the o-azidobenzoic acid. The precipitate is collected by filtration and washed with 6 M HCl (2x) and H₂O (2x). The *o*-azidobenzoic acid product (2) is dried *in vacuo* (500 mtorr, 30°C).

### ACYLATION OF HYDROXYMETHYL RESIN (4)

To hydroxymethyl resin (1.0 eq., 1.3 mmol/g) and the *o*-azidobenzoic acid (1, 2.5 eq.) is added DMF (to give 400 mM *o*-azidobenzoic acid ,1), CsCO₃ (2.0 eq.) and Kl (2.0 eq.). Following agitation of the reaction mixture for 36-48 h, the resin-bound *o*-azidobenzoic acid (4) is washed with MeOH (2 cycles), CH₂Cl₂ (3 cycles), MeOH (3 cycles), DMF (3 cycles), MeOH (3 cycles) and CH₂Cl₂ (3 cycles), and dried *in vacuo.*

### AZA-WITTIG FORMATION (5)

To the resin-bound o-azidobenzoic acid (4,1.0 eq.) is added a solution of PPh₃ (THF, 500 mM, 5.0 eq.). After 6 h, the resin is washed with 3 cycles of the following: THF (3 cycles), toluene (3 cycles), CH₂Cl₂ (3 cycles) and hexanes (3 cycles). Followed by drying *in vacuo* to afford resin bound iminophosphorane (5)

### CARBODIIMIDE FORMATION (6)

To the resin-bound iminophosphorane (5, 1 eq.) is added isocyanate (9 , 5 eq., 450 mM) dissolved in ClCH₂CH₂Cl. The compounds are shaken at ambient temperature for 16 h, washed with 3 cycles of the following: THF (3 cycles), toluene (3 cycles), CH₂Cl₂ (3 cycles) and hexanes (3 cycles), and dried *in vacuo* to afford carbodiimide (6).

### GUANIDINE FORMATION / CYCLIZATION

To the carbodiimide functionalized resin (6) is added secondary amine (10, 0.6 eq., 500 mM) dissolved in ClCH₂CH₂Cl. The reaction mixture is heated to 50 °C in an incubator for 12-72 h to afford 2-aminoquinazoline (8).

All of the final products are analyzed using an Evaporative Light Scattering Detector (ELSD) detection to determine purity.

### EXAMPLE 3

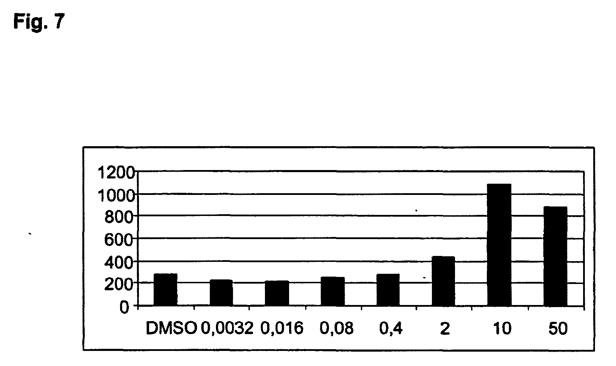

This example illustrates that compounds according to the invention (experiments shown were done with MOLSTRUCTURE LN0000007033 (see formulas (9)) activate luciferase reporter gene expression in a dose dependent manner mediated through GAL4-LXRa-LBD or GAL4-LXRb-LBD constructs in HEK293 cells. LN0000007033 does activate LXR beta LBD truct mediated luciferase activity much stronger than with LXR alpha construct which is in contrast to the similar activation of both LXR alpha and LXR beta LBD containing constructs with T0901317 (see Fig. 7).

HEK293 cells are grown in 96 well plates and co-transfected with pFR-luc (Stratagene) and pCMV-BD-LXRa-LBD or pCMV-BD-LXRb-LBD (each 100 ng of plasmid DNA per well). Transfection is carried using Lipfectamine 2000 (Gibco-BRL) according to the manufacturers protocol. The ligand binding domains (LBD) of LXRa and LXRb are cloned into the pCMV-BD-GW (the Gateway Reading Frame Cassette B is cloned as an EcoRV fragment into Smal site of pCMV-BD) applying the manufacturer protocols for the Gateway™ system (Invitrogen).
Luciferase reporter activity is measured in triplicates from extracts of cells after incubating cells in culture medium (DMEM [Gibco-BRL] + 10% FCS [PAA laboratories]) for 16 hours (5% CO₂, 37°C) containing 0,5% DMSO (control) or 0,5% DMSO with increasing concentrations of LN0000007033. The type of assay used here is a mammalian one hybrid (M1H) assay that is known to those skilled in the art.

Dose-dependent luciferase activities originating from pFR-luc demonstrate the relative activities of the compounds with the LXRa or LXRb LBDs in this mammalian one hybrid type approach.

### EXAMPLE 4

This example shows that described compounds can increase the abundance of mRNA of LXR target genes like ABCA1 and ABCG1 in THP-1 cells which are treated with TPA or FAS and SREBP-1c in HepG2 cells as shown in Fig 8A and Fig. 8B.

THP-1 cells are seeded in 24 well plates at 3 x10⁵ cells per well in RPMI 1640 medium containing 10 % FCS and 100 nM TPA for 24 h. HepG2 cells are seeded in poly-L-Lysine coated 24well plates at 1x10⁶ cells per well in EMEM medium containing 10 % FCS until they are appr. 60% confluent.
Before treatment with LXR compounds, the growth medium is changed to medium containing 10% charcoal/dextran-stripped FCS for 12 h. Treatment is done for 12h (THP-1 cells) and 24h (HepG2 cells), respectively, in medium containing 10% charcoal/dextran-stripped FCS (and 100 nM PMA in the case of THP-1 cells).
LXR compounds are dissolved in DMSO, with the final solvent concentration never exceeding 0.125%. All treatments are done in triplicates and experiments repeated twice. Total RNA is extracted using the Qiagen Rneasy Mini Kit and treated with DNase (DNAfree kit, Ambion). RNA is reverse transcribed with Oligo(dT) primer and real-time reverse transcription PCR (TaqMan) is performed using the ABI Prism 7900HT Sequence Detection System and reagents supplied by Applied Biosystems. mRNA steady state levels are normalised to H3 histone (H3F3A) expression levels. The sequences of forward primers, reverse primers and TaqMan probes are as follows :

FAS : CTGAGACGGAGGCCATATGCT, GCTGCCACACGCTCCTCTAG, FAM-
CAGCAGTTCACGGACATGGAGCACAA-TAMRA

ABCA1 :TCCTGTGGTGTTTCTGGATGAAC, CTTGACAACACTTAGGGCACAATTC,
FAM- ACCACAGGCATGGATCCCAAAGCC-TAMRA

### EXAMPLE 5

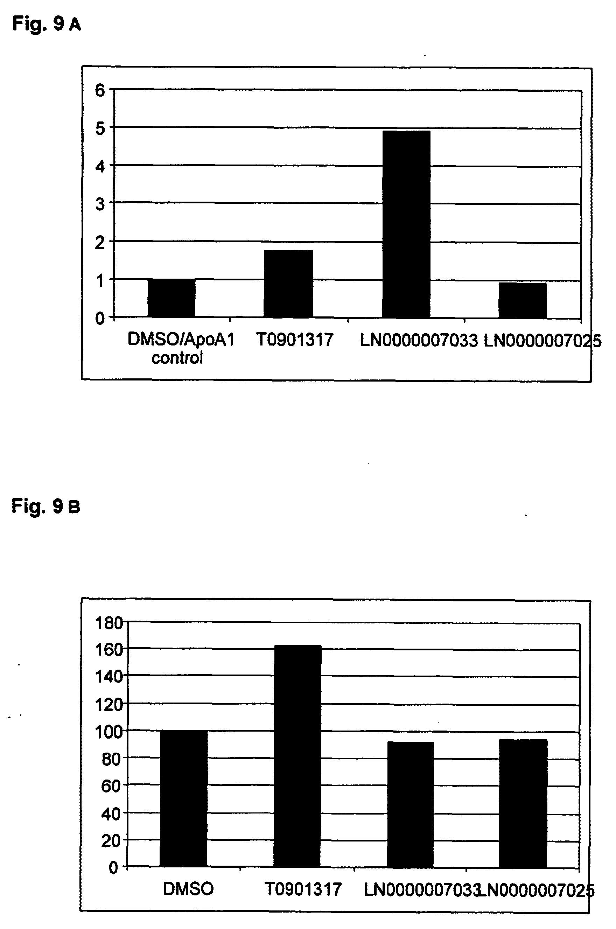

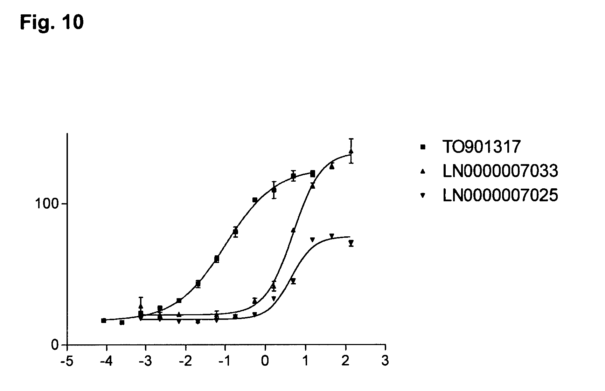

LN0000007033 causes a marked increase in cholesterol export in differentated THP-1 macrophages (see Figure 9 A).
Strikingly, the compound T0901317 causes a marked increase in triyglyceride mass in HepG2 liver cells, while compounds like LN0000007033 and LN0000007025 do not cause a significant increase in triglyceride mass.
This behavior is similar to the selective transcriptional effect of compounds like LN0000007033 and LN0000007025 on the LXR target genes in HepG2 versus THP-1 cells (see Fig 8A and 8B).

Methods: Cultures of the monocyte-macrophage cell line and the hepatocytes HepG2 are obtained from the American Type tissue Culture Collection, Rockville, MD and were grown in RPMI 1640 medium supplemented with 10% fetal bovine serum, 10 mM HEPES, 2 mM Pyruvat, 50 µM β-Mercaptoethanol (THP-1) and Minimum essential medium (Eagle) with 2 mM L-glutamine and Earle's BSS supplemented with 10% fetal bovine serum, 2 mM glutamine, 0.1 mM non-essential amino acids, 1 mM sodium pyruvate (HepG2), respectively, at 37°C in 5% CO2.
THP-1 cells are differentiated into macrophages by addition of 100 nM Phorbol 12-Myristate 13-Acetate (PMA; Sigma P8139) and PMA included in the medium of all subsequent experiments to maintain differentiation.
For cholesterol efflux measurements and triglyceride analysis cells are seeded in 6well plates at 1.8x106 cells (THP-1) and 1x106 cells (HepG2) per well, respectively.

### CHOLESTEROL EFFLUX

THP-1 cells are seeded in 6well plates at 1x10⁶ cells per well in RPMI 1640 medium containing 10 % FCS and 100 nM TPA for 72 h. After washing with PBS cells are incubated 24 h with fresh in RPMI 1640 medium containing 10 % FCS and 100 nM TPA. Cells are washed twice with PBS and RPMI 1640 medium containing 0.15% BSA and 100 nM TPA is added for further 24 h. Treatment with LXR compounds is done for 24 h in RPMI 1640 medium containing 0.15% BSA, 100 nM TPA and 40 µg/ml ApoA1 (Calbiochem # 178452). Medium is collected, centrifuged to remove cell debris and assayed for cholesterol using a commercial fluorometric kit (Molecular Probes A-12216). The remaining cellular proteins are lysed with 0.3 N NaOH and protein content measured with the Biorad Bradford reagent.

### TRIGLYCERIDE ASSAY

HepG2 cells are seeded in poly-L-Lysine coated 6well plates at 1x10⁶ cells per well in EMEM medium containing 10 % FCS until they were appr. 60% confluent. Before treatment with LXR compounds growth medium is changed to medium containing 10% charcoal/dextran-stripped FCS for 12 h. Treatment is done for 24h in medium containing 10% charcoal/dextran-stripped FCS. Cells are washed twice with ice-cold PBS/0.2% BSA and twice with cold PBS and all liquid carefully removed. Triglyceride are extracted with with 1.5 ml hexane/ isopropanol = 3:2 per well with gentle shaking for 2-3 h at RT according to Pan et al. (2002) JBC 277, 4413-4421 and Goti et al. (1998) Biochem J, 332, 57-65. The extraction solution is collected, dried under vacuum and redissolved in isopropanol/ 1.5% triton. The remaining cellular proteins are lysed with 0.3 N NaOH and protein content measured with the Biorad Bradford reagent.
Triglyceride levels are measured as esterified glycerol using a commercial enzymatic colorimetric kit (Sigma 343-25P). In a preliminary assay it is checked by omitting the lipase enzyme that contribution of free glycerol is negligible.

### FIGURE CAPTIONS

### FIG. 1

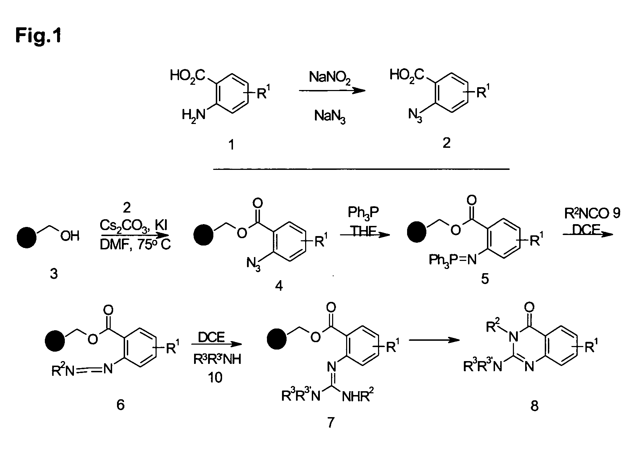

Fig. 1 shows the synthesis of the compounds according to the invention as also described in Example 2.

### FIG. 2

Fig. 2 shows the measurement parameters employed by the Wallace VICTOR2V™ Multilabel Counter which was used for measuring the EC₅₀ values

### FIG. 3

Shows a table with the accession numbers for the key genes

### FIG. 4

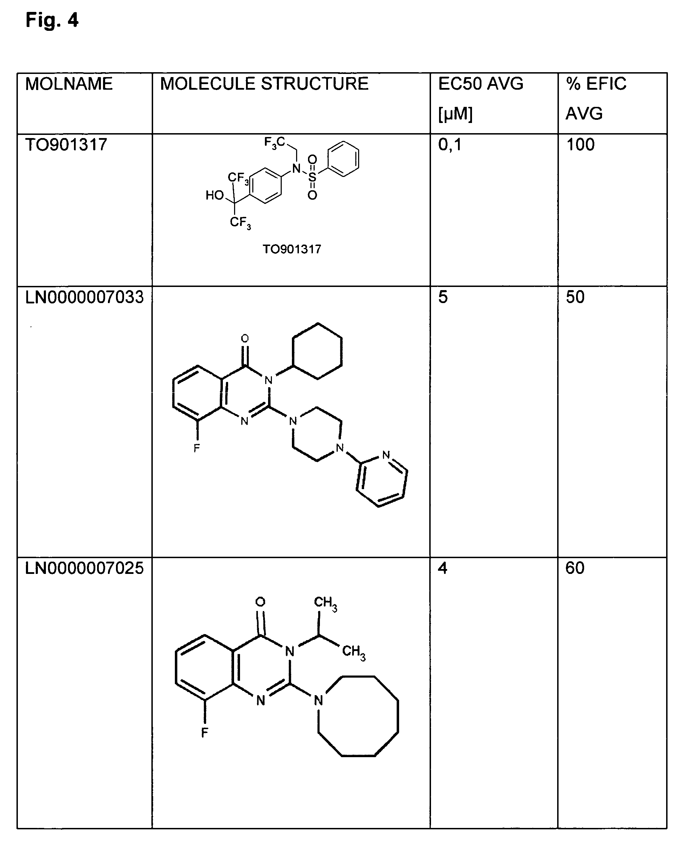

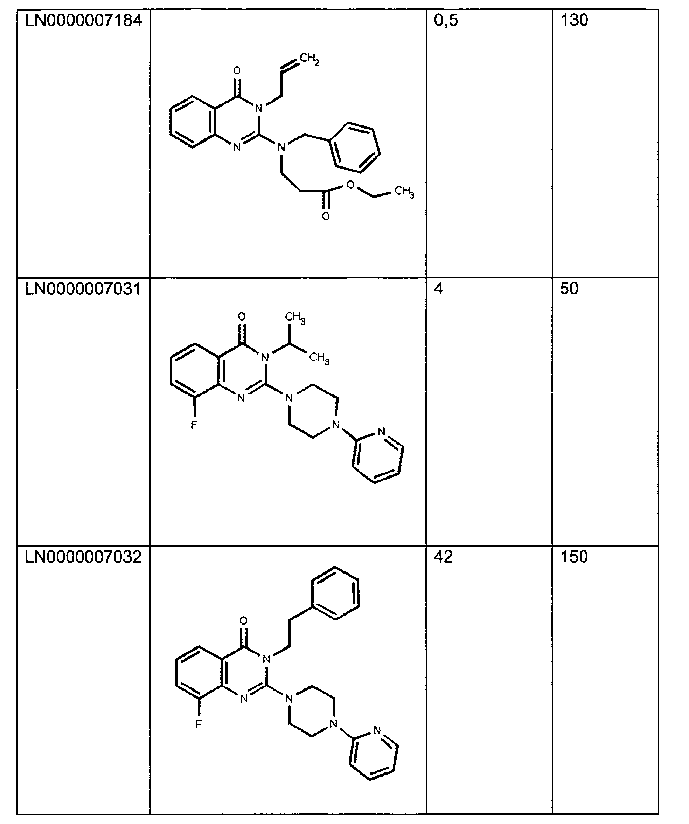

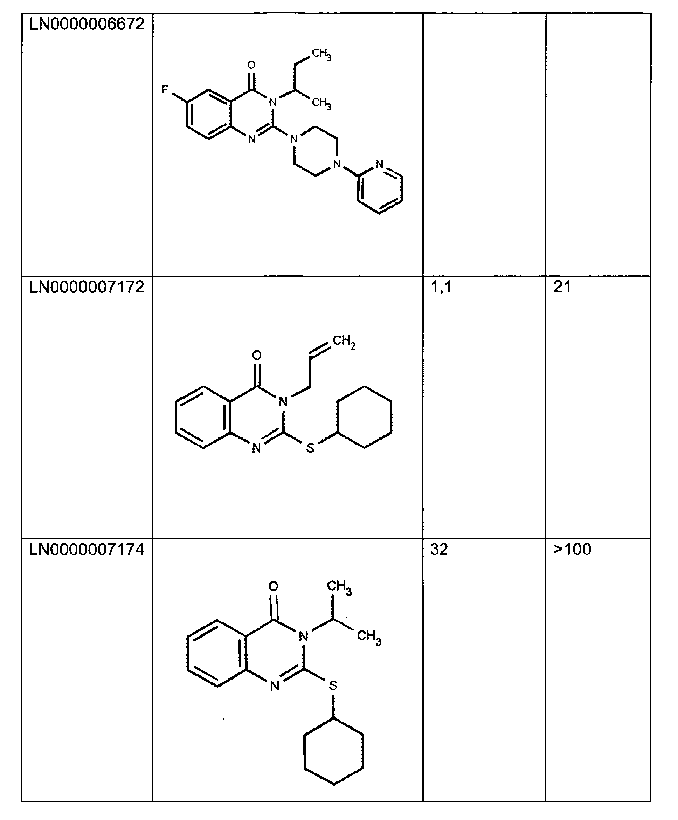

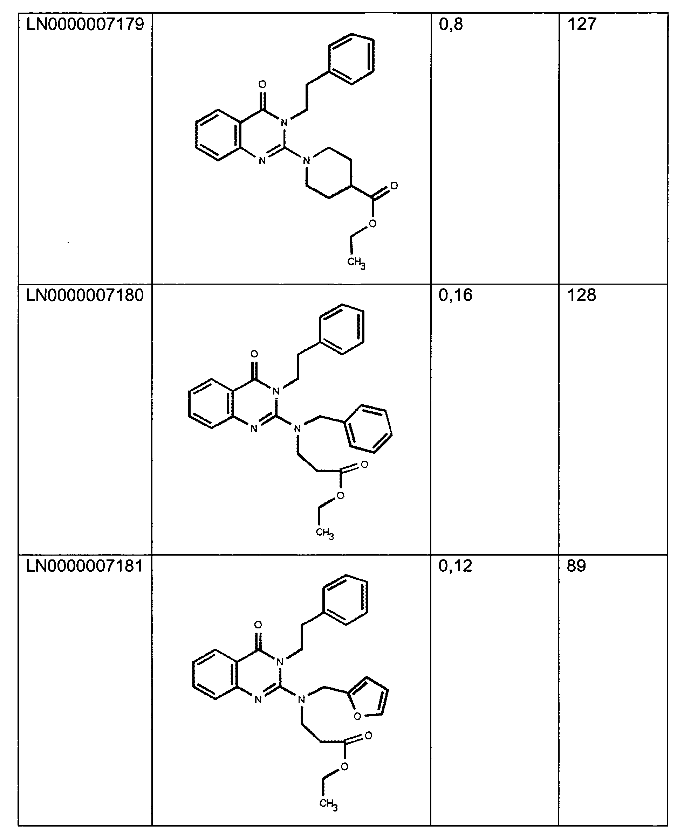

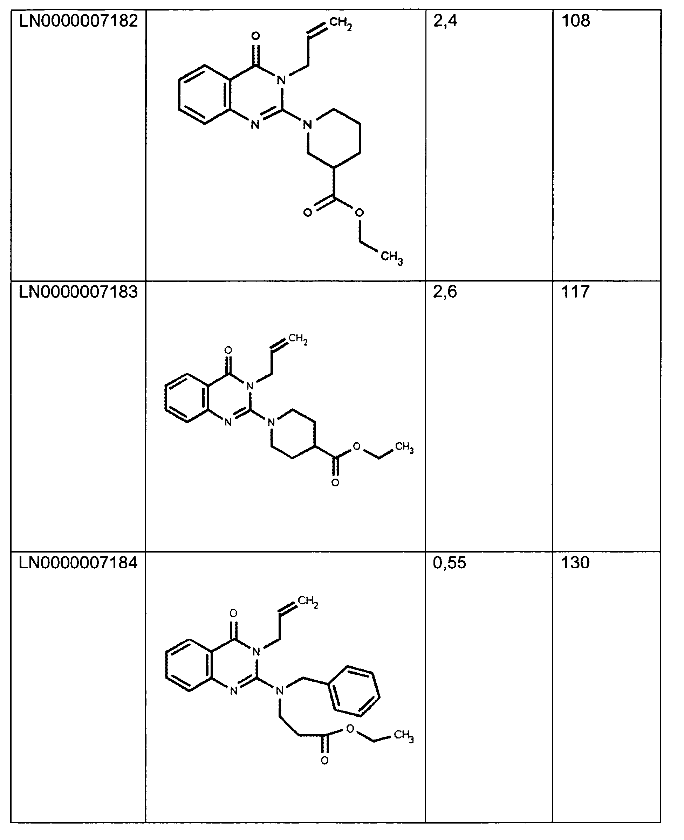

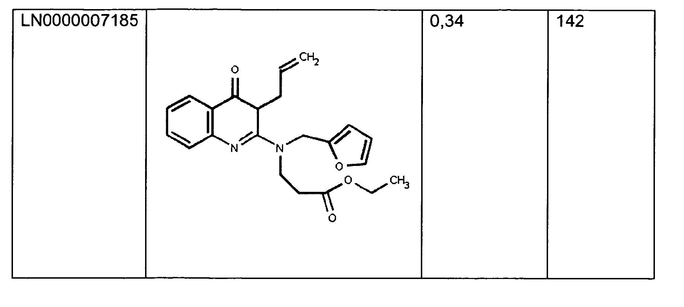

Fig. 4 shows the internal molecular name used by the applicant (MOLNAME) as well as the corresponding structures of preferred compounds according to the invention. The figure further shows their respective EC₅₀ values (EC50 AVG) as established according to the Example 1 in multiple experiments (see above), as well as their respective average efficacy (% activity relative to TO901317 control agonist).

### FIG. 5

Figure 5 shows various known LXR ligands. The compound T0901317 is used as a reference compound here. It is apparent from their structures that the inventors have identified novel compounds which are structurally not related to these known ligands.

### FIG. 6

Figure 6 shows various genes that have been found to be regulated through binding of an LXR agonist to the LXR protein.

### FIG. 7

Fig 7 shows a dose dependence of LN0000007033 with LXR beta LBD containing constructs in mammalian one hybrid (M1H) type assays. The respective µM concentrations of the compounds are given on the x-axis and the relative light units (RLU) are depicted on the y-axis.

### Fig. 8 A and B

Analysis of mRNA content of the indicated genes (LXRalpha, ABCA1, ABCG1, FAS and SREBP-1 c) in total RNA isolated from THP-1 cells (8A, LXRalpha, ABCA1, ABCG1) or HepG2 cells (8B, FAS and SREBP-1 c) treated for 12 or 24 hours with 10 µM on x-axis of T0901317 and LN0000007033. The relative fold induction is depicted on the y-axis and the value depicted with * for ABCG1 has to be multiplicated by a factor of 10.

### Fig. 9

Analysis of relative fold increase in total cholesterol from supernatants of cultivated THP-1 cells (indicated on the y-axis) incubated with ApoA1 and with or without 10µM of the compounds T0901317, LN0000007033 and LN0000007025 as indicated on the X-axis of Fig 9A.
Analysis of relative levels of total triglyceride (TG) content in HepG2 cells (indicated on the y-axis) treated with 25µM of the indicated compounds T0901317, LN0000007033 and LN0000007025 (indicated on the x-axis).

## Claims

1. A compound of the formula (I), or pharmaceutically acceptable salts or solvates thereof, 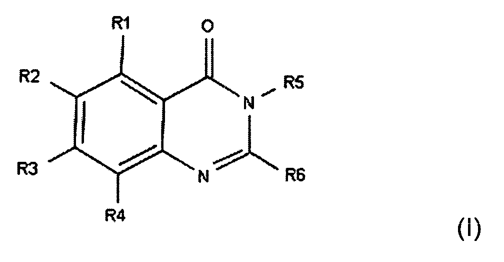 wherein the substitutents and indices have the following meanings:
R₁, R₂, R₃ and R₄, is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N-(phenylsulfonyl)amino or substituted or unsubstituted phenyl,
R₅ is H, C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl
R₆ is H, a secondary or a tertiary amine, C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl

2. A compound according to claim 1, or pharmaceutically acceptable salts or solvates thereof, of formula (II) 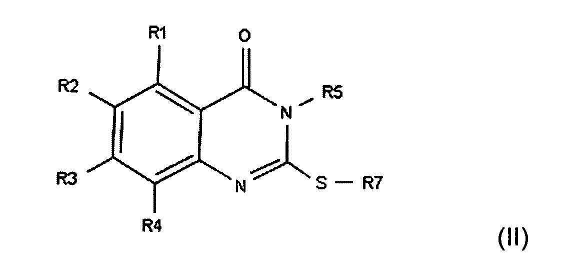 wherein the substitutents and indices have the following meanings:
R₇ is C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl

3. A compound according to claim 1, or pharmaceutically acceptable salts or solvates thereof, of formula (III) 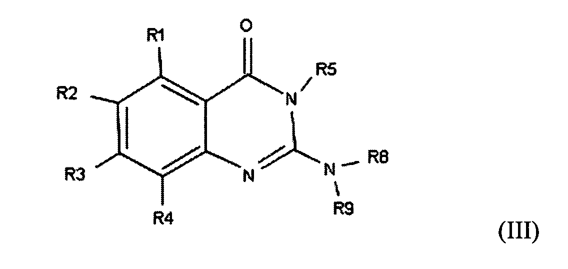 Wherein the substituents and indices have the following meanings:
R₁, R₂, R₃, R₄, is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N-(phenylsulfonyl)amino or phenyl,
R₅ in formula (III) is H, C₁ to C₈ alkyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl,
R₈ in formula (III) is H, C₁ to C₈ alkyl, C₁ to C₈ substituted alkyl, C₃ to C₈ cycloalkyl, C₃ to C₈ substituted cycloalkyl, C₁ to C₇ acyl, C₁ to C₇ substituted acyl, aryl, substituted aryl, C1 to C6 alkylphenyl or C₇ to C₁₂ substituted phenylalkyl, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₉ in formula (III) is H, C₁ to C₈ alkyl, C₁ to C₈ substituted alkyl, C₃ to C₈ cycloalkyl, C₃ to C₈ substituted cycloalkyl, C₁ to C₇ acyl, C₁ to C₇ substituted acyl, aryl, substituted aryl, C1 to C6 alkylphenyl or C₇ to C₁₂ substituted phenylalkyl, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl,
R₈ and R₉ in formula (III) are taken together with the N in formula (III) to form a ring of the following types:
C₃ to C₈ mono, di- or tri- heteroatom-substituted cycloalkyl, preferably piperidino, morpholino, thiomorpholino, piperazino, or C₁ to C₆ alkyl-, C₁ to C₆ substituted alkyl subtituted- or benzocondensated derivatives thereof,
C₄ to C₁₀ mono, di- or tri- heteroatom-substituted bi- or tricycloalkyl derivatives thereof or C₁ to C₆ alkyl-, C₁ to C₆ substituted alkyl subtituted- or benzocondensated derivatives thereof,
C₃ to C₈ mono, di- or tri- heteroatom-substituted heteroaryl, preferably pyrrolo, pyrazolo, oxazolo, thiazolo, pyridino, pyridazino, pyrimidino, pyrazino, triazino, indolo, quinolino, quinazolino, or C₁ to C₆ alkyl-, C₁ to C₆ substituted alkyl subtituted- or benzocondensated derivatives thereof

4. A compound according to claim 3 wherein
R₁ and R₃ are H,
R₂ and R₄ are H, or iodo, bromo, chloro or fluoro or hydroxy methyl, trifluoromethyl, methoxy, amino, alkylated amino, or nitro
R₅ is H, C₁ to C₈ alkyl, preferrably isopropyl, vinyl, 2-isobutyl, or cyclohexyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl,
R₈ and R₉ in formula (III) are taken together with the N in formula (III) to form a ring of the following types:
C₃ to C₈ mono, di- or tri- heteroatom-substituted cycloalkyl, preferably piperidino, morpholino, thiomorpholino, piperazino, or C₁ to C₆ alkyl-C₁-C₆ substituted or benzocondensated derivatives thereof, more preferrably a 4-aryl- or 4-heteroaryl- or 4-alkyl-substituted piperazino or a 4-alkyl-substituted piperidino
C₄ to C₁₀ mono, di- or tri- heteroatom-substituted bi- or tricycloalkyl or C₁ to C₆ alkyl-, C₁-C₆ substituted alkyl substituted- or benzocondensated derivatives thereof
C₃ to C₈ mono, di- or tri- heteroatom-substituted heteroaryl, preferably pyrrolo, pyrazolo, oxazolo, thiazolo, pyridino, pyridazino, pyrimidino, pyrazino, triazino, indolo, quinolino, quinazolino or C₁ to C₆ alkyl- C₁ to C₆ substituted alkyl substituted- or benzocondensated derivatives thereof

5. A compound according to any of claims 1 to 3 wherein
R₁ and R₃ are H,
R₂ and R₄ are H, or iodo, bromo, chloro or fluoro or hydroxy methyl, trifluoromethyl, methoxy, amino, alkylated amino, or nitro
R₅ is H, C₁ to C₈ alkyl, preferrably isopropyl, vinyl, 2-isobutyl, C₁ to C₈ substituted alkyl, C₃ to C₈ cycloalkyl, C₃ to C₈ substituted cycloalkyl, aryl, substituted aryl, C1 to C6 alkylphenyl or C₇ to C₁₂ substituted phenylalkyl,
R₈ is a C₁ to C₈ alkyl, C₁ to C₈ substituted alkyl, C₃ to C₈ cycloalkyl, C₃ to C₈ substituted cycloalkyl, C₁ to C₆ alkylphenyl or C₇ to C₁₂ substituted phenylalkyl, preferrably benzyl or (furan-2-yl)-methyl or (thiophen-2-yl)-methyl
and R₉ in formula is C₁ to C₈ alkyl, C₁ to C₈ substituted alkyl, C₃ to C₈ cycloalkyl, C₃ to C₈ substituted cycloalkyl, C₁ to C₆ alkylphenyl or C₇ to C₁₂ substituted phenylalkyl, preferably (ethyl)-propion-1-yl

6. A compound according to any of claims 1 to 5 being

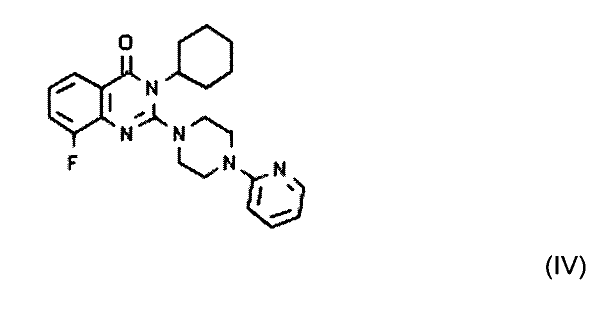

7. A compound according to any of claims 1 to 5 being

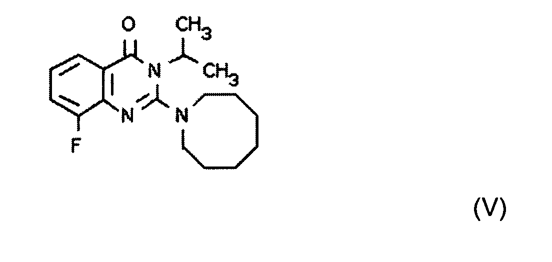

8. A compound according to any of claims 1 to 5 being

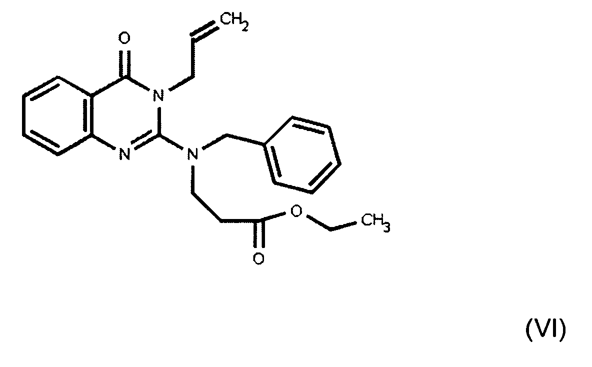

9. A compound according to any of claims 1 and 5 being

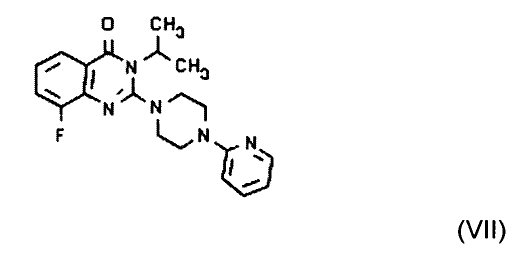

10. A compound according to any of claims 1 and 5 being

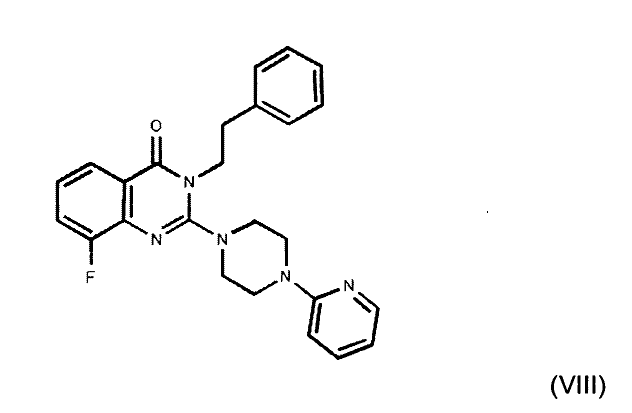

11. A compound according to any of claims 1 and 5 being

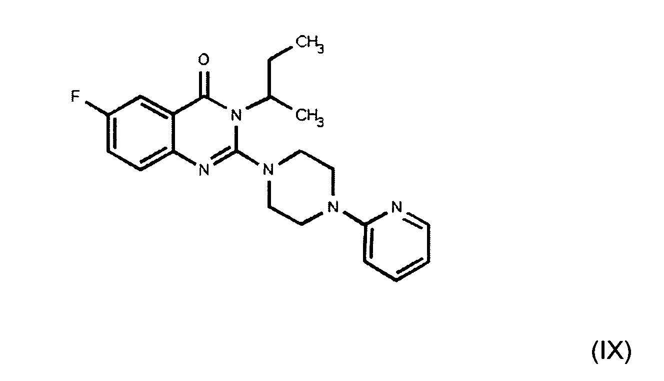

12. A compound according to any of claims 1 and 5 being

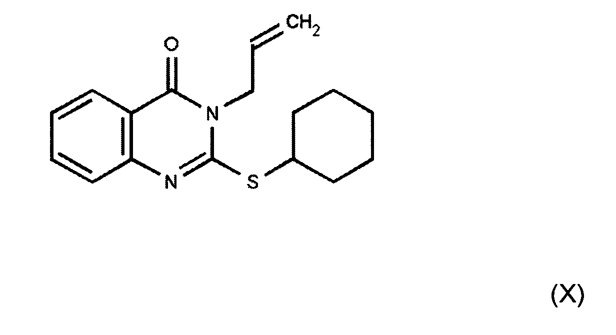

13. A compound according to any of claims 1 and 5 being

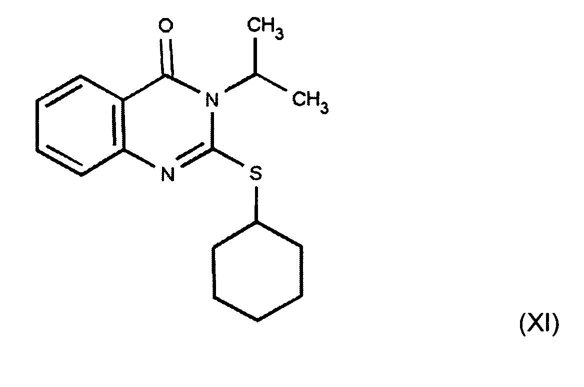

14. A compound according to any of claims 1 and 5 being

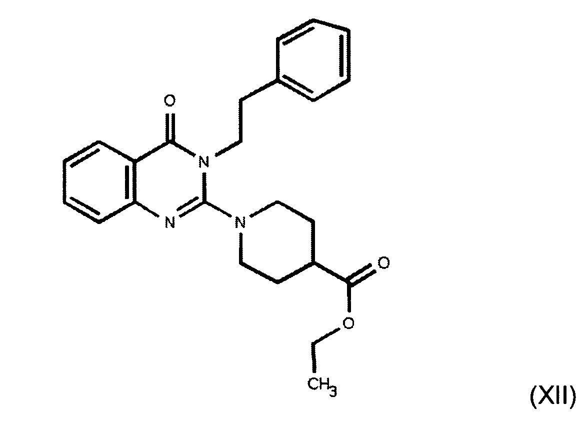

15. A compound according to any of claims 1 and 5 being

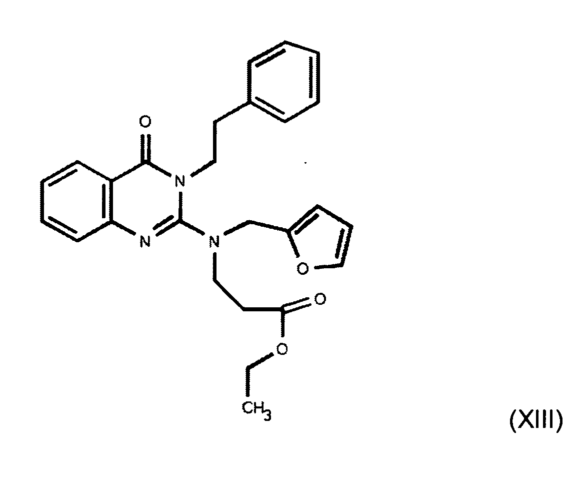

16. A compound according to any of claims 1 to 15 wherein said compound is capable of binding the NR1H3 receptor protein encoded by a nucleic acid comprising SEQ ID NO:2 or a portion thereof or a mammalian homologue thereof.

17. A compound according to any of claims 1 to 15 wherein said compound is capable of binding the NR1H2 receptor protein encoded by a nucleic acid comprising SEQ ID NO:1 or a portion thereof or a mammalian homologue thereof.

18. A compound according to any of claims 1 to 15 for use as a medicament.

19. A method for prevention or treatment of a NR1H3 and/or NR1H2 receptor protein mediated disease or NR1H3 and/or NR1H2 receptor protein homologue mediated disease or condition in a mammal comprising administration of a therapeutically effective amount of a compound according to claims 1 to 15 wherein the prevention or treatment is directly or indirectly accomplished through the binding of the compound according claims 1 to 15 to the NR1H3 and/or NR1H2 receptor proteins or to the NR1H3 and/or NR1H2 receptor protein homologues.

20. A method for regulating the cholesterol synthesis or transport in a mammal which comprises activating the NR1H3 and/or NR1H2 receptors with a therapeutically effective amount of a compound according to claims 1 to 15.

21. A method of treating in mammal a disease which is affected by cholesterol, triglyceride, bile acid, glucose or glucocorticoid levels comprising administering to a mammal in need of such treatment a therapeutically effective amount of a compound according to claims 1 to 15.

22. A method of treating in a mammal Atherosclerosis, Alzheimers disease, Type II diabetes, lipid disorders, obesity, an inflammatory or a cardiovascular disorder comprising administering to a mammal in need of such treatment a therapeutically effective amount of a compound according to claims 1 to 15.

23. A method according to any of claims 19 to 22, wherein the expression of one or more of the genes out of the group comprising ABCA1, ABCG1, ABCG5, ABCG8, apolipoprotein-CI, -CII, -CIV, -E, LPL (lipoprotein lipase), CETP (cholesteryl ester transfer protein) or other genes that positively regulate cholesterol homeostasis is increased upon compound administration.

24. A method according to any of claims 19 to 22, wherein the expression of one or more of the genes out of the group comprising 11-β-HSD (11-β hydroxysteroid dehydrogenase), PEPCK (phosphoenolpyruvat carboxykinase), G-6-P (glucose-6-phosphatase) is reduced upon compound administration.

25. A method according to any of claims 19 to 22, wherein the expression of one or more of the genes out of the group comprising FAS (Fatty Acid Synthase), SREBP-1c (Sterol-response element binding protein), SCD-1 (Stearoyl-CoA Desaturase), Angiopoietin like protein 3 (Angptl3) or other genes which are relevant for controlling serum triglyceride or glucose levels are not or more weakly increased in liver and or other organs compared to administration of a full agonist like T0901317.

26. A method of blocking in a mammal the cholesterol or fatty acid absorption in the intestine of a mammal in need of such blocking comprising administering to a mammal in need of such treatment a therapeutically effective amount of a compound as defined in any of claims 1 to 15.

27. A method for treating obesity in a mammal comprising administring a therapeutically effective amount of a compound as defined in any of claims 1 to 15.

28. A method of modulating a gene whose expression is regulated by the NR1H3 and/or NR1H2 receptor in a mammal comprising administering a therapeutically effective amount of a compound as defined in any claims 1 to 15.

29. A method according to any of claims 19 to 28 wherein the expression of ABCA1 and/or ABCG1 and/or ABCG5 and/or ABCG8 is increased.

30. Use of a compound as defined in any of claims 1 to 15 in a method according to claims 19 to 29 wherein the mammal is a human.

31. Use of a compound as defined in any of claims 1 to 15 for the manufacture of a medicament for the prevention or treatment of a NR1H3 and/or NR1H2 receptor protein or NR1H3 and/or NR1H2 receptor protein homologue mediated disease or condition in a mammal wherein the prevention or treatment is directly or indirectly accomplished through the binding of the compound to the NR1H3 and/or NR1H2 receptor protein or NR1H3 and/or NR1H2 receptor protein homologue.

32. Use of a compound as defined in any of claims 1 to 15 for the manufacture of a medicament for prevention or treatment of a NR1H3 and/or NR1H2 receptor protein mediated disease or condition wherein the mammal is a human.

33. Use of a compound as defined in any of claims 1 to 15 for the manufacture of a medicament for regulating the cholesterol transport system in a mammal by activating the NR1H3 and/or NR1H2 receptor.

34. Use of a compound as defined in any of claims 1 to 15 for the manufacture of a medicament for regulating levels of cholesterol, triglyceride, and/or bile acid.

35. Use of a compound as defined in any of claims 1 to 15 for the manufacture of a medicament for treating in a mammal atherosclerosis, Alzheimer disease, gallstone disease, lipid disorders, inflammatory disorder, type II diabetis, obesity or a cardiovascular disorder.

36. Use of a compound as defined in any of claims 1 to 15 for the manufacture of a medicament capable for blocking in a mammal the cholesterol and/or fatty acid absorption in the intestine.

37. Use of a compound as defined in any of claims 1 to 15 for the manufacture of a medicament for treating obesity in a mammal.

38. Use of a compound as defined in any of claims 1 to 15 for the manufacture of a medicament for modulating a gene whose expression is regulated by the NR1H3 and/or NR1H2 receptor.

39. Use of a compound as defined in any of claims 1 to 15 in a mammal for the selective up-regulation of one or more genes selected from the group comprising ABCA1, ABCG1, ABCG5, ABCG8, apolipoprotein-CI, -CII, -CIV, -E, LPL (lipoprotein lipase), CETP (cholesteryl ester transfer protein) or other genes that positively regulate cholesterol homeostasis are increased and a weaker regulation of one or more of the genes selected from the group comprising FAS and SREBP-1c or other genes that positively regulate lipogenesis, said compound showing a larger difference in regulation of the two groups of genes when compared with the regulatory behaviour of a full agonist like T0901317 on both groups of genes.

40. The use according to any of claims 31 to 39, wherein the mammal is human.


SEQUENCE LISTING

<110>  Phenex Pharmaceuticals AG

<120>  Novel selective LXR Nuclear Receptor Binding Compounds with partial
agonistic properties

<130>  PX62425EP

<160>  22

<170>  PatentIn version 3.1

<210>  1
<211>  2010
<212>  DNA
<213>  Homo sapiens

<400>  1
caagaagtgg cgaagttacc tttgagggta tttgagtagc ggcggtgtgt caggggctaa      60
agaggaggac gaagaaaagc agagcaaggg aacccagggc aacaggagta gttcactccg     120
cgagaggccg tccacgagac ccccgcgcgc aggcatgagc cccgcccccc acgcatgagc     180
cccgcccccc gctgttgctt ggagaggggc gggacctgga gagaggctgc tccgtgaccc     240
caccatgtcc tctcctacca cgagttccct ggataccccc ctgcctggaa atggcccccc     300
tcagcctggc gccccttctt cttcacccac tgtaaaggag gagggtccgg agccgtggcc     360
cgggggtccg gaccctgatg tcccaggcac tgatgaggcc agctcagcct gcagcacaga     420
ctgggtcatc ccagatcccg aagaggaacc agagcgcaag cgaaagaagg gcccagcccc     480
gaagatgctg ggccacgagc tttgccgtgt ctgtggggac aaggcctccg gcttccacta     540
caacgtgctc agctgcgaag gctgcaaggg cttcttccgg cgcagtgtgg tccgtggtgg     600
ggccaggcgc tatgcctgcc ggggtggcgg aacctgccag atggacgctt tcatgcggcg     660
caagtgccag cagtgccggc tgcgcaagtg caaggaggca gggatgaggg agcagtgcgt     720
cctttctgaa gaacagatcc ggaagaagaa gattcggaaa cagcagcagc aggagtcaca     780
gtcacagtcg cagtcacctg tggggccgca gggcagcagc agctcagcct ctgggcctgg     840
ggcttcccct ggtggatctg aggcaggcag ccagggctcc ggggaaggcg agggtgtcca     900
gctaacagcg gctcaagaac taatgatcca gcagttggtg gcggcccaac tgcagtgcaa     960

caaacgctcc ttctccgacc agcccaaagt cacgccctgg cccctgggcg cagaccccca 1020
gtcccgagat gcccgccagc aacgctttgc ccacttcacg gagctggcca tcatctcagt 1080
ccaggagatc gtggacttcg ctaagcaagt gcctggtttc ctgcagctgg gccgggagga 1140
ccagatcgcc ctcctgaagg catccactat cgagatcatg ctgctagaga cagccaggcg 1200
ctacaaccac gagacagagt gtatcacctt cttgaaggac ttcacctaca gcaaggacga 1260
cttccaccgt gcaggcctgc aggtggagtt catcaacccc atcttcgagt tctcgcgggc 1320
catgcggcgg ctgggcctgg acgacgctga gtacgccctg ctcatcgcca tcaacatctt 1380
ctcggccgac cggcccaacg tgcaggagcc gggccgcgtg gaggcgttgc agcagcccta 1440
cgtggaggcg ctgctgtcct acacgcgcat caagaggccg caggaccagc tgcgcttccc 1500
gcgcatgctc atgaagctgg tgagcctgcg cacgctgagc tctgtgcact cggagcaggt 1560
cttcgccttg cggctccagg acaagaagct gccgcctctg ctgtcggaga tctgggacgt 1620
ccacgagtga ggggctggcc acccagcccc acagccttgc ctgaccaccc tccagcagat 1680
agacgccggc accccttcct cttcctaggg tggaaggggc cctgggcgag cctgtagacc 1740
tatcggctct catcccttgg gataagcccc agtccaggtc caggaggctc cctccctgcc 1800
cagcgagtct tccagaaggg gtgaaagggt tgcaggtccc gaccactgac ccttcccggc 1860
tgccctccct ccccagctta cacctcaagc ccagcacgca gcgtaccttg aacagaggga 1920
ggggaggacc catggctctc cccccctagc ccgggagacc aggggccttc ctcttcctct 1980
gcttttattt aataaaaata aaaacagaaa 2010

<210> 2
<211> 1528
<212> DNA
<213> Homo sapiens

<400> 2
   cagtgccttg gtaatgacca gggctccaga aagagatgtc cttgtggctg gggccccctg 60
   tgcctgacat tcctcctgac tctgcggtgg agctgtggaa gccaggcgca caggatgcaa 120
   gcagccaggc ccagggaggc agcagctgca tcctcagaga ggaagccagg atgccccact 180
   ctgctggggg tactgcaggg gtggggctgg aggctgcaga gcccacagcc ctgctcacca 240
   gggcagagcc cccttcagaa cccacagaga tccgtccaca aaagcggaaa aaggggccag 300
   cccccaaaat gctggggaac gagctatgca gcgtgtgtgg ggacaaggcc tcgggcttcc 360
   actacaatgt tctgagctgc gagggctgca agggattctt ccgccgcagc gtcatcaagg 420
   gagcgcacta catctgccac agtggcggcc actgccccat ggacacctac atgcgtcgca 480
   agtgccagga gtgtcggctt cgcaaatgcc gtcaggctgg catgcgggag gagtgtgtcc 540
   tgtcagaaga acagatccgc ctgaagaaac tgaagcggca agaggaggaa caggctcatg 600
   ccacatcctt gccccccagg cgttcctcac ccccccaaat cctgccccag ctcagcccgg 660
   aacaactggg catgatcgag aagctcgtcg ctgcccagca acagtgtaac cggcgctcct 720
   tttctgaccg gcttcgagtc acgccttggc ccatggcacc agatccccat agccgggagg 780
   cccgtcagca gcgctttgcc cacttcactg agctggccat cgtctctgtg caggagatag 840
   ttgactttgc taaacagcta cccggcttcc tgcagctcag ccgggaggac cagattgccc 900
   tgctgaagac ctctgcgatc gaggtgatgc ttctggagac atctcggagg tacaaccctg 960
   ggagtgagag tatcaccttc ctcaaggatt tcagttataa ccgggaagac tttgccaaag 1020
   cagggctgca agtggaattc atcaacccca tcttcgagtt ctccagggcc atgaatgagc 1080
   tgcaactcaa tgatgccgag tttgccttgc tcattgctat cagcatcttc tctgcagacc 1140
   ggcccaacgt gcaggaccag ctccaggtgg agaggctgca gcacacatat gtggaagccc 1200
   tgcatgccta cgtctccatc caccatcccc atgaccgact gatgttccca cggatgctaa 1260
   tgaaactggt gagcctccgg accctgagca gcgtccactc agagcaagtg tttgcactgc 1320
   gtctgcagga caaaaagctc ccaccgctgc tctctgagat ctgggatgtg cacgaatgac 1380
   tgttctgtcc ccatattttc tgttttcttg gccggatggc tgaggcctgg tggctgcctc 1440
   ctagaagtgg aacagactga gaagggcaaa cattcctggg agctgggcaa ggagatcctc 1500
   ccgtggcatt aaaagagagt caaagggt 1528

<210> 3

<211> 2877

<212> DNA

<213> Homo sapiens

<400> 3
gtggcatcct tccctttcta atcagagatt ttcttcctca gagattttgg cctagatttg 60

caaaatgatg accacatctt tgatttgggg gattgctata gcagcatgct gttgtctatg 120

gcttattctt ggaattagga gaaggcaaac gggtgaacca cctctagaga atggattaat 180

tccatacctg ggctgtgctc tgcaatttgg tgccaatcct cttgagttcc tcagagcaaa 240

tcaaaggaaa catggtcatg tttttacctg caaactaatg ggaaaatatg tccatttcat 300

cacaaatccc ttgtcatacc ataaggtgtt gtgccacgga aaatattttg attggaaaaa 360

atttcacttt gctacttctg cgaaggcatt tgggcacaga agcattgacc cgatggatgg 420

aaataccact gaaaacataa acgacacttt catcaaaacc ctgcagggcc atgccttgaa 480

ttccctcacg gaaagcatga tggaaaacct ccaacgtatc atgagacctc cagtctcctc 540

taactcaaag accgctgcct gggtgacaga agggatgtat tctttctgct accgagtgat 600

gtttgaagct gggtatttaa ctatctttgg cagagatctt acaaggcggg acacacagaa 660

agcacatatt ctaaacaatc ttgacaactt caagcaattc gacaaagtct ttccagccct 720

ggtagcaggc ctccccattc acatgttcag gactgcgcac aatgcccggg agaaactggc 780
agagagcttg aggcacgaga acctccaaaa gagggaaagc atctcagaac tgatcagcct 840
gcgcatgttt ctcaatgaca ctttgtccac ctttgatgat ctggagaagg ccaagacaca 900
cctcgtggtc ctctgggcat cgcaagcaaa caccattcca gcgactttct ggagtttatt 960
tcaaatgatt aggaacccag aagcaatgaa agcagctact gaagaagtga aaagaacatt 1020
agagaatgct ggtcaaaaag tcagcttgga aggcaatcct atttgtttga gtcaagcaga 1080
actgaatgac ctgccagtat taaatagtat aatcaaggaa tcgctgaggc tttccagtgc 1140
ctccctcaac atccggacag ctaaggagga tttcactttg caccttgagg acggttccta 1200
caacatccga aaagatagca tcatagctct ttacccacag ttaatgcact tagatccaga 1260
aatctaccca gaccctttga cttttaaata tgataggtat cttgatgaaa acgggaagac 1320
aaagactacc ttctattgta atggactcaa gttaaagtat tactacatgc cctttggatc 1380
gggagctaca atatgtcctg gaagattgtt cgctatccac gaaatcaagc aatttttgat 1440
tctgatgctt tcttattttg aattggagct tatagagggc caagctaaat gtccaccttt 1500
ggaccagtcc cgggcaggct tgggcatttt gccgccattg aatgatattg aatttaaata 1560
taaattcaag catttgtgaa tacatggctg gaataagagg acactagatg atattacagg 1620
actgcagaac accctcacca cacagtccct ttggacaaat gcatttagtg gtggtagaaa 1680
tgattcacca ggtccaatgt tgttcaccag tgcttgcttg tgaatcttaa cattttggtg 1740
acagtttcca gatgctatca cagactctgc tagtgaaaag aactagtttc taggagcaca 1800
ataatttgtt ttcatttgta taagtccatg aatgttcata tagccaggga ttgaagttta 1860
ttattttcaa aggaaaacac ctttatttta ttttttttca aaatgaagat acacattaca 1920
gccaggtgtg gtagcaggca cctgtagtct tagctactcg agaggccaaa gaaggaggat 1980
ggcttgagcc caggagttca agaccagcct ggacagctta gtgagatccc gtctccgaag 2040
aaaagatatg tattctaatt ggcagattgt tttttcctaa ggaaactgct ttatttttat 2100
aaaactgcct gacaattatg aaaaaatgtt caaattcacg ttctagtgaa actgcattat 2160
ttgttgacta gatggtgggg ttcttcgggt gtgatcatat atcataaagg atatttcaaa 2220
tgattatgat tagttatgtc ttttaataaa aaggaaatat ttttcaactt cttctatatc 2280
caaaattcag ggctttaaac atgattatct tgatttccca aaaacactaa aggtggtttt 2340
attttccctt catgttttaa cttattgttg ctgaaaactc tatgtccggc tttaactatc 2400
ttctctatat ttttatttca ttcacattaa tgagaagagt tttctcagag attaaaaaag 2460
gtagtttttc tgtcattgtt aaatacacat tatcactgaa aaaatgtagc ttttatgatg 2520
tatgttttaa agttaaaact ggatggaaat agccatttgg aagctttggt tatgaaacat 2580
gtggagtgta ttaagtgcag cttgacatta tgttttattt aaatgctttt tatcgctaaa 2640
tgacttgcag atgaaaaaaa ctaaggtgac tcgagtgttt aaatgcctgt gtacaacaat 2700
gctttgataa aatattttaa ggtatgagtt atcagctcta tgtcaattga tatttctgtg 2760

tagtatttat atttaaatta tatttacctt tttgcttatt ttacaaatat taagaaaata 2820
ttctaacatt tgataatttt gaaatgattc atctttcaga aataaaagta tgaatct 2877

<210> 4
<211> 8461
<212> DNA
<213> Homo sapiens

<400> 4
gagggagcca gagagacggc agcggccccg gcctccctct ccgccgcgct tcagcctccc 60
gctccgccgc gctccagcct cgctctccgc cgcccgcacc gccgcccgcg ccctcaccag 120
agcagccatg gaggaggtgg tgattgccgg catgtccggg aagctgccag agtcggagaa 180
cttgcaggag ttctgggaca acctcatcgg cggtgtggac atggtcacgg acgatgaccg 240
tcgctggaag gcggggctct acggcctgcc ccggcggtcc ggcaagctga aggacctgtc 300
taggtttgat gcctccttct tcggagtcca ccccaagcag gcacacacga tggaccctca 360
gctgcggctg ctgctggaag tcacctatga agccatcgtg gacggaggca tcaacccaga 420
ttcactccga ggaacacaca ctggcgtctg ggtgggcgtg agcggctctg agacctcgga 480
ggccctgagc cgagaccccg agacactcgt gggctacagc atggtgggct gccagcgagc 540
gatgatggcc aaccggctct ccttcttctt cgacttcaga gggcccagca tcgcactgga 600
cacagcctgc tcctccagcc tgatggccct gcagaacgcc taccaggcca tccacagcgg 660
gcagtgccct gccgccatcg tggggggcat caatgtcctg ctgaagccca acacctccgt 720
gcagttcttg aggctgggga tgctcagccc cgagggcacc tgcaaggcct tcgacacagc 780
ggggaatggg tactgccgct cggagggtgt ggtggccgtc ctgctgacca agaagtccct 840
ggcccggcgg gtgtacgcca ccatcctgaa cgccggcacc aatacagatg gcttcaagga 900
gcaaggcgtg accttcccct caggggatat ccaggagcag ctcatccgct cgttgtacca 960
gtcggccgga gtggcccctg agtcatttga atacatcgaa gcccacggca caggcaccaa 1020
ggtgggcgac ccccaggagc tgaatggcat cacccgagcc ctgtgcgcca cccgccagga 1080
gccgctgctc atcggctcca ccaagtccaa catggggcac ccggagccag cctcggggct 1140
ggcagccctg gccaaggtgc tgctgtccct ggagcacggg ctctgggccc ccaacctgca 1200
cttccatagc cccaaccctg agatcccagc gctgttggat gggcggctgc aggtggtgga 1260
ccagcccctg cccgtccgtg gcggcaacgt gggcatcaac tcctttggct tcgggggctc 1320
caacgtgcac atcatcctga ggcccaacac gcagccgccc cccgcacccg ccccacatgc 1380
caccctgccc cgtctgctgc gggccagcgg acgcacccct gaggccgtgc agaagctgct 1440
ggagcagggc ctccggcaca gccaggacct ggctttcctg agcatgctga acgacatcgc 1500
ggctgtcccc gccaccgcca tgcccttccg tggctacgct gtgctgggtg gtgagcgcgg 1560

tggcccagag gtgcagcagg tgcccgctgg cgagcgcccg ctctggttca tctgctctgg 1620
gatgggcaca cagtggcgcg ggatggggct gagcctcatg cgcctggacc gcttccgaga 1680
ttccatccta cgctccgatg aggctgtgaa gccattcggc ctgaaggtgt cacagctgct 1740
gctgagcaca gacgagagca cctttgatga catcgtccat tcgtttgtga gcctgactgc 1800
catccagata ggcctcatag acctgctgag ctgcatgggg ctgaggccag atggcatcgt 1860
cggccactcc ctgggggagg tggcctgtgg ctacgccgac ggctgcctgt cccaggagga 1920
ggccgtcctc gctgcctact ggaggggaca gtgcatcaaa gaagcccatc tcccgccggg 1980
cgccatggca gccgtgggct tgtcctggga ggagtgtaaa cagcgctgcc ccccggcggt 2040
ggtgcccgcc tgccacaact ccaaggacac agtcaccatc tcgggacctc aggccccggt 2100
gtttgagttc gtggagcagc tgaggaagga gggtgtgttt gccaaggagg tgcggaccgg 2160
cggtatggcc ttccactcct acttcatgga ggccatcgca cccccactgc tgcaggagct 2220
caagaaggtg atccgggagc cgaagccacg ttcagcccgc tggctcagca cctctatccc 2280
cgaggcccag tggcacagca gcctggcacg cacgtcctcc gccgagtaca atgtcaacaa 2340
cctggtgagc cctgtgctgt tccaggaggc cctgtggcac gtgcctgagc acgcggtggt 2400
gctggagatc gcgccccacg ccctgctgca ggctgtcctg aagcgtggcc tgaagccgag 2460
ctgcaccatc atccccctga tgaagaagga tcacagggac aacctggagt tcttcctggc 2520
cggcatccgg aggctgcacc tctcaggcat cgacgccaac cccaatgcct tgttcccacc 2580
tgtggagttc ccagctcccc gaggaactcc cctcatctcc ccactcatca agtgggacca 2640
cagcctggcc tgggacgtgc cggccgccga ggacttcccc aacggttcag gttccccctc 2700
agccgccatc tacaacatcg acaccagctc cgagtctcct gaccactacc tggtggacca 2760
caccctcgac ggtcgcgtcc tcttccccgc cactggctac ctgagcatag tgtggaagac 2820
gctggcccga cccctgggcc tgggcgtcga gcagctgcct gtggtgtttg aggatgtggt 2880
gctgcaccag gccaccatcc tgcccaagac tgggacagtg tccctggagg tacggctcct 2940
ggaggcctcc cgtgccttcg aggtgtcaga gaacggcaac ctggtagtga gtgggaaggt 3000
gtaccagtgg gatgaccctg accccaggct cttcgaccac ccggaaagcc ccacccccaa 3060
ccccacggag cccctcttcc tggcccaggc tgaagtttac aaggagctgc gtctgcgtgg 3120
ctacgactac ggccctcatt tccagggcat cctggaggcc agcctggaag gtgactcggg 3180
gaggctgctg tggaaggata actgggtgag cttcatggac accatgctgc agatgtccat 3240
cctgggctcg gccaagcacg gcctgtacct gcccacccgt gtcaccgcca tccacatcga 3300
ccctgccacc cacaggcaga agctgtacac actgcaggac aaggcccaag tggctgacgt 3360
ggtggtgagc aggtggctga gggtcacagt ggccggaggc gtccacatct ccgggctcca 3420
cactgagtcg gccccgcggc ggcagcagga gcagcaggtg cccatcctgg agaagttttg 3480
cttcactccc cacacggagg aggggtgcct gtctgagcgc gctgccctgc aggaggagct 3540
gcaactgtgc aaggggctgg tgcaggcact gcagaccaag gtgacccagc aggggctgaa 3600

gatggtggtg cccggactgg atggggccca gatcccccgg gacccctcac agcaggaact 3660
gccccggctg ttgtcggctg cctgcaggct tcagctcaac gggaacctgc agctggagct 3720
ggcgcaggtg ctggcccagg agaggcccaa gctgccagag gaccctctgc tcagcggcct 3780
cctggactcc ccggcactca aggcctgcct ggacactgcc gtggagaaca tgcccagcct 3840
gaagatgaag gtggtggagg tgctggccgg ccacggtcac ctgtattccc gcatcccagg 3900
cctgctcagc ccccatcccc tgctgcagct gagctacacg gccaccgacc gccaccccca 3960
ggccctggag gctgcccagg ccgagctgca gcagcacgac gttgcccagg gccagtggga 4020
tcccgcagac cctgccccca gcgccctggg cagcgccgac ctcctggtgt gcaactgtgc 4080
tgtggctgcc ctcggggacc cggcctcagc tctcagcaac atggtggctg ccctgagaga 4140
agggggcttt ctgctcctgc acacactgct ccgggggcac ccctcgggac atgtggcctt 4200
cctcacctcc actgagccgc agtatggcca gggcatcctg agccaggacg cgtgggagag 4260
cctcttctcc agggtgtccg tgcgcctggt gggcctgaag aagtccttct acggctccac 4320
gctcttcctg tgccgccggc ccaccccgca ggacagcccc atcttcctgc cggtggacga 4380
taccagcttc cgctgggtgg agtctctgaa gggcatcctg gctgacgaag actcttcccg 4440
gcctgtgtgg ctgaaggcca tcaactgtgc cacctcgggc gtggtgggct tggtgaactg 4500
tctccgccga gagcccggcg gaacgctccg gtgtgtgctg ctctccaacc tcagcagcac 4560
ctcccacgtc ccggaggtgg acccgggctc cgcagaactg cagaaggtgt tgcagggaga 4620
cctggtgatg aacgtctacc gcgacggggc ctggggggct ttccgccact tcctgctgga 4680
ggaggacaag cctgaggagc cgacggcaca tgcctttgtg agcaccctca cccgggggga 4740
cctgtcctcc atccgctggg tctgctcctc gctgcgccat gcccagccca cctgccctgg 4800
cgcccagctc tgcacggtct actacgcctc cctcaacttc cgcgacatca tgctggccac 4860
tggcaagctg tcccctgatg ccatcccagg gaagtggacc tcccaggaca gcctgctagg 4920
tatggagttc tcgggccgag acgccagcgg caagcgtgtg atgggactgg tgcctgccaa 4980
gggcctggcc acctctgtcc tgctgtcacc ggacttcctc tgggatgtgc cttccaactg 5040
gacgctggag gaggcggcct cggtgcctgt cgtctacagc acggcctact acgcgctggt 5100
ggtgcgtggg cgggtgcgcc ccggggagac gctgctcatc cactcgggct cgggcggcgt 5160
gggccaggcc gccatcgcca tcgccctcag tctgggctgc cgcgtcttca ccaccgtggg 5220
gtcggctgag aagcgggcgt acctccaggc caggttcccc cagctcgaca gcaccagctt 5280
cgccaactcc cgggacacat ccttcgagca gcatgtgctg tggcacacgg gcgggaaggg 5340
cgttgacctg gtcttgaact ccttggcgga agagaagctg caggccagcg tgaggtgctt 5400
ggctacgcac ggtcgcttcc tggaaattgg caaattcgac ctttctcaga accacccgct 5460
cggcatggct atcttcctga agaacgtgac attccacggg gtcctactgg atgcgttctt 5520
caacgagagc agtgctgact ggcgggaggt gtgggcgctt gtgcaggccg gcatccggga 5580
tggggtggta cggcccctca agtgcacggt gttccatggg gcccaggtgg aggacgcctt 5640

ccgctacatg gcccaaggga agcacattgg caaagtcgtc gtgcaggtgc ttgcggagga 5700
gccggaggca gtgctgaagg gggccaaacc caagctgatg tcggccatct ccaagacctt 5760
ctgcccggcc cacaagagct acatcatcgc tggtggtctg ggtggcttcg gcctggagtt 5820
ggcgcagtgg ctgatacagc gtggggtgca gaagctcgtg ttgacttctc gctccgggat 5880
ccggacaggc taccaggcca agcaggtccg ccggtggagg cgccagggcg tacaggtgca 5940
ggtgtccacc agcaacatca gctcactgga gggggcccgg ggcctcattg ccgaggcggc 6000
gcagcttggg cccgtgggcg gcgtcttcaa cctggccgtg gtcttgagag atggcttgct 6060
ggagaaccag accccagagt tcttccagga cgtctgcaag cccaagtaca gcggcaccct 6120
gaacctggac agggtgaccc gagaggcgtg ccctgagctg gactactttg tggtcttctc 6180
ctctgtgagc tgcgggcgtg gcaatgcggg acagagcaac tacggctttg ccaattccgc 6240
catggagcgt atctgtgaga aacgccggca cgaaggcctc ccaggcctgg ccgtgcagtg 6300
gggcgccatc ggcgacgtgg gcattttggt ggagacgatg agcaccaacg acacgatcgt 6360
cagtggcacg ctgccccagc gcatggcgtc ctgcctggag gtgctggacc tcttcctgaa 6420
ccagccccac atggtcctga gcagctttgt gctggctgag aaggctgcgg cctataggga 6480
cagggacagc cagcgggacc tggtggaggc cgtggcacac atcctgggca tccgcgactt 6540
ggctgctgtc aacctggaca gctcactggc ggacctgggc ctggactcgc tcatgagcgt 6600
ggaggtgcgc cagacgctgg agcgtgagct caacctggtg ctgtccgtgc gcgaggtgcg 6660
gcaactcacg ctccggaaac tgcaggagct gtcctcaaag gcggatgagg ccagcgagct 6720
ggcatgcccc acgcccaagg aggatggtct ggcccagcag cagactcagc tgaacctgcg 6780
ctccctgctg gtgaacccgg agggccccac cctgatgcgg ctcaactccg tgcagagctc 6840
ggagcggccc ctgttcctgg tgcacccaat cgagggctcc accaccgtgt tccacagcct 6900
ggcctcccgg ctcagcatcc ccacctatgg cctgcagtgc acccgagctg cgccccttga 6960
cagcatccac agcctggctg cctactacat cgactgcatc aggcaggtgc agcccgaggg 7020
cccctaccgc gtggccggct actcctacgg ggcctgcgtg gcctttgaaa tgtgctccca 7080
gctgcaggcc cagcagagcc cagcccccac ccacaacagc ctcttcctgt tcgacggctc 7140
gcccacctac gtactggcct acacccagag ctaccgggca aagctgaccc caggctgtga 7200
ggctgaggct gagacggagg ccatatgctt cttcgtgcag cagttcacgg acatggagca 7260
caacagggtg ctggaggcgc tgctgccgct gaagggccta gaggagcgtg tggcagccgc 7320
cgtggacctg atcatcaaga gccaccaggg cctggaccgc caggagctga gctttgcggc 7380
ccggtccttc tactacaagc tgcgtgccgc tgagcagtac acacccaagg ccaagtacca 7440
tggcaacgtg atgctactgc gcgccaagac gggtggcgcc tacggcgagg acctgggcgc 7500
ggactacaac ctctcccagg tatgcgacgg gaaagtatcc gtccacgtca tcgagggtga 7560
ccaccgcacg ctgctggagg gcagcggcct ggagtccatc atcagcatca tccacagctc 7620
cctggctgag ccacgcgtga gcgtgcggga gggctaggcc cgtgcccccg cctgccaccg 7680

gaggtcactc caccatcccc accccacccc accccacccc cgccatgcaa cgggattgaa 7740
gggtcctgcc ggtgggaccc tgtccggccc agtgccactg ccccccgagg ctgctagacg 7800
taggtgttag gcatgtccca cccacccgcc gcctcccacg gcacctcggg gacaccagag 7860
ctgccgactt ggagactcct ggtctgtgaa gagccggtgg tgcccgtgcc cgcaggaact 7920
gggctgggcc tcgtgcgccc gtggggtctg cgcttggtct ttctgtgctt ggatttgcat 7980
atttattgca ttgctggtag agacccccag gcctgtccac cctgccaaga ctcctcaggc 8040
agcgtgtggg tcccgcactc tgcccccatt tccccgatgt cccctgcggg cgcgggcagc 8100
cacccaagcc tgctggctgc ggccccctct cggccaggca ttggctcagc ccgctgagtg 8160
gggggtcgtg ggccagtccc cgaggagctg ggcccctgca caggcacaca gggcccggcc 8220
acacccagcg gccccccgca cagccacccg tggggtgctg cccttatgcc cggcgccggg 8280
caccaactcc atgtttggtg tttgtctgtg tttgtttttc aagaaatgat tcaaattgct 8340
gcttggattt tgaaatttac tgtaactgtc agtgtacacg tctggacccc gtttcatttt 8400
tacaccaatt tggtaaaaat gctgctctca gcctcccaca attaaaccgc atgtgatctc 8460
c 8461

<210> 5
<211> 1444
<212> DNA
<213> Homo sapiens

<400> 5
acggtcaccc gttgccagct ctagccttta aattcccggc tcggggacct ccacgcaccg 60
cggctagcgc cgacaaccag ctagcgtgca aggcgccgcg gctcagcgcg taccggcggg 120
cttcgaaacc gcagtcctcc ggcgaccccg aactccgctc cggagcctca gccccctgga 180
aagtgatccc ggcatccgag agccaagatg ccggcccact tgctgcagga cgatatctct 240
agctcctata ccaccaccac caccattaca gcgcctccct ccagggtcct gcagaatgga 300
ggagataagt tggagacgat gcccctctac ttggaagacg acattcgccc tgatataaaa 360
gatgatatat atgaccccac ctacaaggat aaggaaggcc caagccccaa ggttgaatat 420
gtctggagaa acatcatcct tatgtctctg ctacacttgg gagccctgta tgggatcact 480
ttgattccta cctgcaagtt ctacacctgg ctttgggggg tattctacta ttttgtcagt 540
gccctgggca taacagcagg agctcatcgt ctgtggagcc accgctctta caaagctcgg 600
ctgcccctac ggctctttct gatcattgcc aacacaatgg cattccagaa tgatgtctat 660
gaatgggctc gtgaccaccg tgcccaccac aagttttcag aaacacatgc tgatcctcat 720
aattcccgac gtggcttttt cttctctcac gtgggttggc tgcttgtgcg caaacaccca 780
gctgtcaaag agaaggggag tacgctagac ttgtctgacc tagaagctga gaaactggtg 840

atgttccaga ggaggtacta caaacctggc ttgctgatga tgtgcttcat cctgcccacg 900
cttgtgccct ggtatttctg gggtgaaact tttcaaaaca gtgtgttcgt tgccactttc 960
ttgcgatatg ctgtggtgct taatgccacc tggctggtga acagtgctgc ccacctcttc 1020
ggatatcgtc cttatgacaa gaacattagc ccccgggaga atatcctggt ttcacttgga 1080
gctgtgggtg agggcttcca caactaccac cactcctttc cctatgacta ctctgccagt 1140
gagtaccgct ggcacatcaa cttcaccaca ttcttcattg attgcatggc cgccctcggt 1200
ctggcctatg accggaagaa agtctccaag gccgccatct tggccaggat taaaagaacc 1260
ggagatggaa actacaagag tggctgagtt tggggtccct caggttcctt tttcaaaaac 1320
cagccaggca gaggttttaa tgtctgttta ttaactactg aataatgcta ccaggatgct 1380
aaagatgatg atgttaaccc attccagtac agtattcttt taaaattcaa aagtattgaa 1440
agcc 1444

<210> 6
<211> 4154
<212> DNA
<213> Homo sapiens

<400> 6
taacgaggaa cttttcgccg gcgccgggcc gcctctgagg ccagggcagg acacgaacgc 60
gcggagcggc ggcggcgact gagagccggg gccgcggcgg cgctccctag gaagggccgt 120
acgaggcggc gggcccggcg ggcctcccgg aggaggcggc tgcgccatgg acgagccacc 180
cttcagcgag gcggctttgg agcaggcgct gggcgagccg tgcgatctgg acgcggcgct 240
gctgaccgac atcgaagaca tgcttcagct tatcaacaac caagacagtg acttccctgg 300
cctatttgac ccaccctatg ctgggagtgg ggcagggggc acagaccctg ccagccccga 360
taccagctcc ccaggcagct tgtctccacc tcctgccaca ttgagctcct ctcttgaagc 420
cttcctgagc gggccgcagg cagcgccctc acccctgtcc cctccccagc ctgcacccac 480
tccattgaag atgtacccgt ccatgcccgc tttctcccct gggcctggta tcaaggaaga 540
gtcagtgcca ctgagcatcc tgcagacccc caccccacag cccctgccag gggccctcct 600
gccacagagc ttcccagccc cagccccacc gcagttcagc tccacccctg tgttaggcta 660
ccccagccct ccgggaggct tctctacagg aagccctccc gggaacaccc agcagccgct 720
gcctggcctg ccactggctt ccccgccagg ggtcccgccc gtctccttgc acacccaggt 780
ccagagtgtg gtcccccagc agctactgac agtcacagct gcccccacgg cagcccctgt 840
aacgaccact gtgacctcgc agatccagca ggtcccggtc ctgctgcagc cccacttcat 900
caaggcagac tcgctgcttc tgacagccat gaagacagac ggagccactg tgaaggcggc 960
aggtctcagt cccctggtct ctggcaccac tgtgcagaca gggcctttgc cgaccctggt 1020

gagtggcgga accatcttgg caacagtccc actggtcgta gatgcggaga agctgcctat 1080
caaccggctc gcagctggca gcaaggcccc ggcctctgcc cagagccgtg gagagaagcg 1140
cacagcccac aacgccattg agaagcgcta ccgctcctcc atcaatgaca aaatcattga 1200
gctcaaggat ctggtggtgg gcactgaggc aaagctgaat aaatctgctg tcttgcgcaa 1260
ggccatcgac tacattcgct ttctgcaaca cagcaaccag aaactcaagc aggagaacct 1320
aagtctgcgc actgctgtcc acaaaagcaa atctctgaag gatctggtgt cggcctgtgg 1380
cagtggaggg aacacagacg tgctcatgga gggcgtgaag actgaggtgg aggacacact 1440
gaccccaccc ccctcggatg ctggctcacc tttccagagc agccccttgt cccttggcag 1500
caggggcagt ggcagcggtg gcagtggcag tgactcggag cctgacagcc cagtctttga 1560
ggacagcaag gcaaagccag agcagcggcc gtctctgcac agccggggca tgctggaccg 1620
ctcccgcctg gccctgtgca cgctcgtctt cctctgcctg tcctgcaacc ccttggcctc 1680
cttgctgggg gcccgggggc ttcccagccc ctcagatacc accagcgtct accatagccc 1740
tgggcgcaac gtgctgggca ccgagagcag agatggccct ggctgggccc agtggctgct 1800
gcccccagtg gtctggctgc tcaatgggct gttggtgctc gtctccttgg tgcttctctt 1860
tgtctacggt gagccagtca cacggcccca ctcaggcccc gccgtgtact tctggaggca 1920
tcgcaagcag gctgacctgg acctggcccg gggagacttt gcccaggctg cccagcagct 1980
gtggctggcc ctgcgggcac tgggccggcc cctgcccacc tcccacctgg acctggcttg 2040
tagcctcctc tggaacctca tccgtcacct gctgcagcgt ctctgggtgg gccgctggct 2100
ggcaggccgg gcagggggcc tgcagcagga ctgtgctctg cgagtggatg ctagcgccag 2160
cgcccgagac gcagccctgg tctaccataa gctgcaccag ctgcacacca tggggaagca 2220
cacaggcggg cacctcactg ccaccaacct ggcgctgagt gccctgaacc tggcagagtg 2280
tgcaggggat gccgtgtctg tggcgacgct ggccgagatc tatgtggcgg ctgcattgag 2340
agtgaagacc agtctcccac gggccttgca ttttctgaca cgcttcttcc tgagcagtgc 2400
ccgccaggcc tgcctggcac agagtggctc agtgcctcct gccatgcagt ggctctgcca 2460
ccccgtgggc caccgtttct tcgtggatgg ggactggtcc gtgctcagta ccccatggga 2520
gagcctgtac agcttggccg ggaacccagt ggaccccctg gcccaggtga ctcagctatt 2580
ccgggaacat ctcttagagc gagcactgaa ctgtgtgacc cagcccaacc ccagccctgg 2640
gtcagctgat ggggacaagg aattctcgga tgccctcggg tacctgcagc tgctgaacag 2700
ctgttctgat gctgcggggg ctcctgccta cagcttctcc atcagttcca gcatggccac 2760
caccaccggc gtagacccgg tggccaagtg gtgggcctct ctgacagctg tggtgatcca 2820
ctggctgcgg cgggatgagg aggcggctga gcggctgtgc ccgctggtgg agcacctgcc 2880
ccgggtgctg caggagtctg agagacccct gcccagggca gctctgcact ccttcaaggc 2940
tgcccgggcc ctgctgggct gtgccaaggc agagtctggt ccagccagcc tgaccatctg 3000
tgagaaggcc agtgggtacc tgcaggacag cctggctacc acaccagcca gcagctccat 3060

tgacaaggcc gtgcagctgt tcctgtgtga cctgcttctt gtggtgcgca ccagcctgtg 3120
gcggcagcag cagcccccgg ccccggcccc agcagcccag ggcaccagca gcaggcccca 3180
ggcttccgcc cttgagctgc gtggcttcca acgggacctg agcagcctga ggcggctggc 3240
acagagcttc cggcccgcca tgcggagggt gttcctacat gaggccacgg cccggctgat 3300
ggcgggggcc agccccacac ggacacacca gctcctcgac cgcagtctga ggcggcgggc 3360
aggccccggt ggcaaaggag gcgcggtggc ggagctggag ccgcggccca cgcggcggga 3420
gcacgcggag gccttgctgc tggcctcctg ctacctgccc cccggcttcc tgtcggcgcc 3480
cgggcagcgc gtgggcatgc tggctgaggc ggcgcgcaca ctcgagaagc ttggcgatcg 3540
ccggctgctg cacgactgtc agcagatgct catgcgcctg ggcggtggga ccactgtcac 3600
ttccagctag accccgtgtc cccggcctca gcacccctgt ctctagccac tttggtcccg 3660
tgcagcttct gtcctgcgtc gaagctttga aggccgaagg cagtgcaaga gactctggcc 3720
tccacagttc gacctgcggc tgctgtgtgc cttcgcggtg gaaggcccga ggggcgcgat 3780
cttgacccta agaccggcgg ccatgatggt gctgacctct ggtggccgat cggggcactg 3840
caggggccga gccattttgg ggggcccccc tccttgctct gcaggcacct tagtggcttt 3900
tttcctcctg tgtacaggga agagaggggt acatttccct gtgctgacgg aagccaactt 3960
ggctttcccg gactgcaagc agggctctgc cccagaggcc tctctctccg tcgtgggaga 4020
gagacgtgta catagtgtag gtcagcgtgc ttagcctcct gacctgaggc tcctgtgcta 4080
ctttgccttt tgcaaacttt attttcatag attgagaagt tttgtacaga gaattaaaaa 4140
tgaaattatt tata 4154

<210> 7
<211> 10412
<212> DNA
<213> Homo sapiens

<400> 7
gtaattgcga gcgagagtga gtggggccgg gacccgcaga gccgagccga cccttctctc 60
ccgggctgcg gcagggcagg gcggggagct ccgcgcacca acagagccgg ttctcagggc 120
gctttgctcc ttgttttttc cccggttctg ttttctcccc ttctccggaa ggcttgtcaa 180
ggggtaggag aaagagacgc aaacacaaaa gtggaaaaca gttaatgacc agccacggcg 240
tccctgctgt gagctctggc cgctgccttc cagggctccc gagccacacg ctgggggtgc 300
tggctgaggg aacatggctt gttggcctca gctgaggttg ctgctgtgga agaacctcac 360
tttcagaaga agacaaacat gtcagctgct gctggaagtg gcctggcctc tatttatctt 420
cctgatcctg atctctgttc ggctgagcta cccaccctat gaacaacatg aatgccattt 480
tccaaataaa gccatgccct ctgcaggaac acttccttgg gttcagggga ttatctgtaa 540

tgccaacaac ccctgtttcc gttacccgac tcctggggag gctcccggag ttgttggaaa 600
ctttaacaaa tccattgtgg ctcgcctgtt ctcagatgct cggaggcttc ttttatacag 660
ccagaaagac accagcatga aggacatgcg caaagttctg agaacattac agcagatcaa 720
gaaatccagc tcaaacttga agcttcaaga tttcctggtg gacaatgaaa ccttctctgg 780
gttcctgtat cacaacctct ctctcccaaa gtctactgtg gacaagatgc tgagggctga 840
tgtcattctc cacaaggtat ttttgcaagg ctaccagtta catttgacaa gtctgtgcaa 900
tggatcaaaa tcagaagaga tgattcaact tggtgaccaa gaagtttctg agctttgtgg 960
cctaccaagg gagaaactgg ctgcagcaga gcgagtactt cgttccaaca tggacatcct 1020
gaagccaatc ctgagaacac taaactctac atctcccttc ccgagcaagg agctggctga 1080
agccacaaaa acattgctgc atagtcttgg gactctggcc caggagctgt tcagcatgag 1140
aagctggagt gacatgcgac aggaggtgat gtttctgacc aatgtgaaca gctccagctc 1200
ctccacccaa atctaccagg ctgtgtctcg tattgtctgc gggcatcccg agggaggggg 1260
gctgaagatc aagtctctca actggtatga ggacaacaac tacaaagccc tctttggagg 1320
caatggcact gaggaagatg ctgaaacctt ctatgacaac tctacaactc cttactgcaa 1380
tgatttgatg aagaatttgg agtctagtcc tctttcccgc attatctgga aagctctgaa 1440
gccgctgctc gttgggaaga tcctgtatac acctgacact ccagccacaa ggcaggtcat 1500
ggctgaggtg aacaagacct tccaggaact ggctgtgttc catgatctgg aaggcatgtg 1560
ggaggaactc agccccaaga tctggacctt catggagaac agccaagaaa tggaccttgt 1620
ccggatgctg ttggacagca gggacaatga ccacttttgg gaacagcagt tggatggctt 1680
agattggaca gcccaagaca tcgtggcgtt tttggccaag cacccagagg atgtccagtc 1740
cagtaatggt tctgtgtaca cctggagaga agctttcaac gagactaacc aggcaatccg 1800
gaccatatct cgcttcatgg agtgtgtcaa cctgaacaag ctagaaccca tagcaacaga 1860
agtctggctc atcaacaagt ccatggagct gctggatgag aggaagttct gggctggtat 1920
tgtgttcact ggaattactc caggcagcat tgagctgccc catcatgtca agtacaagat 1980
ccgaatggac attgacaatg tggagaggac aaataaaatc aaggatgggt actgggaccc 2040
tggtcctcga gctgacccct ttgaggacat gcggtacgtc tgggggggct tcgcctactt 2100
gcaggatgtg gtggagcagg caatcatcag ggtgctgacg ggcaccgaga agaaaactgg 2160
tgtctatatg caacagatgc cctatccctg ttacgttgat gacatctttc tgcgggtgat 2220
gagccggtca atgcccctct tcatgacgct ggcctggatt tactcagtgg ctgtgatcat 2280
caagggcatc gtgtatgaga aggaggcacg gctgaaagag accatgcgga tcatgggcct 2340
ggacaacagc atcctctggt ttagctggtt cattagtagc ctcattcctc ttcttgtgag 2400
cgctggcctg ctagtggtca tcctgaagtt aggaaacctg ctgccctaca gtgatcccag 2460
cgtggtgttt gtcttcctgt ccgtgtttgc tgtggtgaca atcctgcagt gcttcctgat 2520
tagcacactc ttctccagag ccaacctggc agcagcctgt gggggcatca tctacttcac 2580

gctgtacctg ccctacgtcc tgtgtgtggc atggcaggac tacgtgggct tcacactcaa 2640
gatcttcgct agcctgctgt ctcctgtggc ttttgggttt ggctgtgagt actttgccct 2700
ttttgaggag cagggcattg gagtgcagtg ggacaacctg tttgagagtc ctgtggagga 2760
agatggcttc aatctcacca cttcggtctc catgatgctg tttgacacct tcctctatgg 2820
ggtgatgacc tggtacattg aggctgtctt tccaggccag tacggaattc ccaggccctg 2880
gtattttcct tgcaccaagt cctactggtt tggcgaggaa agtgatgaga agagccaccc 2940
tggttccaac cagaagagaa tatcagaaat ctgcatggag gaggaaccca cccacttgaa 3000
gctgggcgtg tccattcaga acctggtaaa agtctaccga gatgggatga aggtggctgt 3060
cgatggcctg gcactgaatt tttatgaggg ccagatcacc tccttcctgg gccacaatgg 3120
agcggggaag acgaccacca tgtcaatcct gaccgggttg ttccccccga cctcgggcac 3180
cgcctacatc ctgggaaaag acattcgctc tgagatgagc accatccggc agaacctggg 3240
ggtctgtccc cagcataacg tgctgtttga catgctgact gtcgaagaac acatctggtt 3300
ctatgcccgc ttgaaagggc tctctgagaa gcacgtgaag gcggagatgg agcagatggc 3360
cctggatgtt ggtttgccat caagcaagct gaaaagcaaa acaagccagc tgtcaggtgg 3420
aatgcagaga aagctatctg tggccttggc ctttgtcggg ggatctaagg ttgtcattct 3480
ggatgaaccc acagctggtg tggaccctta ctcccgcagg ggaatatggg agctgctgct 3540
gaaataccga caaggccgca ccattattct ctctacacac cacatggatg aagcggacgt 3600
cctgggggac aggattgcca tcatctccca tgggaagctg tgctgtgtgg gctcctccct 3660
gtttctgaag aaccagctgg gaacaggcta ctacctgacc ttggtcaaga aagatgtgga 3720
atcctccctc agttcctgca gaaacagtag tagcactgtg tcatacctga aaaaggagga 3780
cagtgtttct cagagcagtt ctgatgctgg cctgggcagc gaccatgaga gtgacacgct 3840
gaccatcgat gtctctgcta tctccaacct catcaggaag catgtgtctg aagcccggct 3900
ggtggaagac atagggcatg agctgaccta tgtgctgcca tatgaagctg ctaaggaggg 3960
agcctttgtg gaactctttc atgagattga tgaccggctc tcagacctgg gcatttctag 4020
ttatggcatc tcagagacga ccctggaaga aatattcctc aaggtggccg aagagagtgg 4080
ggtggatgct gagacctcag atggtacctt gccagcaaga cgaaacaggc gggccttcgg 4140
ggacaagcag agctgtcttc gcccgttcac tgaagatgat gctgctgatc caaatgattc 4200
tgacatagac ccagaatcca gagagacaga cttgctcagt gggatggatg gcaaagggtc 4260
ctaccaggtg aaaggctgga aacttacaca gcaacagttt gtggcccttt tgtggaagag 4320
actgctaatt gccagacgga gtcggaaagg attttttgct cagattgtct tgccagctgt 4380
gtttgtctgc attgcccttg tgttcagcct gatcgtgcca ccctttggca agtaccccag 4440
cctggaactt cagccctgga tgtacaacga acagtacaca tttgtcagca atgatgctcc 4500
tgaggacacg ggaaccctgg aactcttaaa cgccctcacc aaagaccctg gcttcgggac 4560
ccgctgtatg gaaggaaacc caatcccaga cacgccctgc caggcagggg aggaagagtg 4620

gaccactgcc ccagttcccc agaccatcat ggacctcttc cagaatggga actggacaat 4680
gcagaaccct tcacctgcat gccagtgtag cagcgacaaa atcaagaaga tgctgcctgt 4740
gtgtccccca ggggcagggg ggctgcctcc tccacaaaga aaacaaaaca ctgcagatat 4800
ccttcaggac ctgacaggaa gaaacatttc ggattatctg gtgaagacgt atgtgcagat 4860
catagccaaa agcttaaaga acaagatctg ggtgaatgag tttaggtatg gcggcttttc 4920
cctgggtgtc agtaatactc aagcacttcc tccgagtcaa gaagttaatg atgccatcaa 4980
acaaatgaag aaacacctaa agctggccaa ggacagttct gcagatcgat ttctcaacag 5040
cttgggaaga tttatgacag gactggacac caaaaataat gtcaaggtgt ggttcaataa 5100
caagggctgg catgcaatca gctctttcct gaatgtcatc aacaatgcca ttctccgggc 5160
caacctgcaa aagggagaga accctagcca ttatggaatt actgctttca atcatcccct 5220
gaatctcacc aagcagcagc tctcagaggt ggctctgatg accacatcag tggatgtcct 5280
tgtgtccatc tgtgtcatct ttgcaatgtc cttcgtccca gccagctttg tcgtattcct 5340
gatccaggag cgggtcagca aagcaaaaca cctgcagttc atcagtggag tgaagcctgt 5400
catctactgg ctctctaatt ttgtctggga tatgtgcaat tacgttgtcc ctgccacact 5460
ggtcattatc atcttcatct gcttccagca gaagtcctat gtgtcctcca ccaatctgcc 5520
tgtgctagcc cttctacttt tgctgtatgg gtggtcaatc acacctctca tgtacccagc 5580
ctcctttgtg ttcaagatcc ccagcacagc ctatgtggtg ctcaccagcg tgaacctctt 5640
cattggcatt aatggcagcg tggccacctt tgtgctggag ctgttcaccg acaataagct 5700
gaataatatc aatgatatcc tgaagtccgt gttcttgatc ttcccacatt tttgcctggg 5760
acgagggctc atcgacatgg tgaaaaacca ggcaatggct gatgccctgg aaaggtttgg 5820
ggagaatcgc tttgtgtcac cattatcttg ggacttggtg ggacgaaacc tcttcgccat 5880
ggccgtggaa ggggtggtgt tcttcctcat tactgttctg atccagtaca gattcttcat 5940
caggcccaga cctgtaaatg caaagctatc tcctctgaat gatgaagatg aagatgtgag 6000
gcgggaaaga cagagaattc ttgatggtgg aggccagaat gacatcttag aaatcaagga 6060
gttgacgaag atatatagaa ggaagcggaa gcctgctgtt gacaggattt gcgtgggcat 6120
tcctcctggt gagtgctttg ggctcctggg agttaatggg gctggaaaat catcaacttt 6180
caagatgtta acaggagata ccactgttac cagaggagat gctttcctta acaaaaatag 6240
tatcttatca aacatccatg aagtacatca gaacatgggc tactgccctc agtttgatgc 6300
catcacagag ctgttgactg ggagagaaca cgtggagttc tttgcccttt tgagaggagt 6360
cccagagaaa gaagttggca aggttggtga gtgggcgatt cggaaactgg gcctcgtgaa 6420
gtatggagaa aaatatgctg gtaactatag tggaggcaac aaacgcaagc tctctacagc 6480
catggctttg atcggcgggc ctcctgtggt gtttctggat gaacccacca caggcatgga 6540
tcccaaagcc cggcggttct tgtggaattg tgccctaagt gttgtcaagg aggggagatc 6600
agtagtgctt acatctcata gtatggaaga atgtgaagct ctttgcacta ggatggcaat 6660

catggtcaat ggaaggttca ggtgccttgg cagtgtccag catctaaaaa ataggtttgg 6720
agatggttat acaatagttg tacgaatagc agggtccaac ccggacctga agcctgtcca 6780
ggatttcttt ggacttgcat ttcctggaag tgttctaaaa gagaaacacc ggaacatgct 6840
acaataccag cttccatctt cattatcttc tctggccagg atattcagca tcctctccca 6900
gagcaaaaag cgactccaca tagaagacta ctctgtttct cagacaacac ttgaccaagt 6960
atttgtgaac tttgccaagg accaaagtga tgatgaccac ttaaaagacc tctcattaca 7020
caaaaaccag acagtagtgg acgttgcagt tctcacatct tttctacagg atgagaaagt 7080
gaaagaaagc tatgtatgaa gaatcctgtt catacggggt ggctgaaagt aaagaggaac 7140
tagactttcc tttgcaccat gtgaagtgtt gtggagaaaa gagccagaag ttgatgtggg 7200
aagaagtaaa ctggatactg tactgatact attcaatgca atgcaattca atgcaatgaa 7260
aacaaaattc cattacaggg gcagtgcctt tgtagcctat gtcttgtatg gctctcaagt 7320
gaaagacttg aatttagttt tttacctata cctatgtgaa actctattat ggaacccaat 7380
ggacatatgg gtttgaactc acactttttt tttttttttt gttcctgtgt attctcattg 7440
gggttgcaac aataattcat caagtaatca tggccagcga ttattgatca aaatcaaaag 7500
gtaatgcaca tcctcattca ctaagccatg ccatgcccag gagactggtt tcccggtgac 7560
acatccattg ctggcaatga gtgtgccaga gttattagtg ccaagttttt cagaaagttt 7620
gaagcaccat ggtgtgtcat gctcactttt gtgaaagctg ctctgctcag agtctatcaa 7680
cattgaatat cagttgacag aatggtgcca tgcgtggcta acatcctgct ttgattccct 7740
ctgataagct gttctggtgg cagtaacatg caacaaaaat gtgggtgtct ccaggcacgg 7800
gaaacttggt tccattgtta tattgtccta tgcttcgagc catgggtcta cagggtcatc 7860
cttatgagac tcttaaatat acttagatcc tggtaagagg caaagaatca acagccaaac 7920
tgctggggct gcaagctgct gaagccaggg catgggatta aagagattgt gcgttcaaac 7980
ctagggaagc ctgtgcccat ttgtcctgac tgtctgctaa catggtacac tgcatctcaa 8040
gatgtttatc tgacacaagt gtattatttc tggctttttg aattaatcta gaaaatgaaa 8100
agatggagtt gtattttgac aaaaatgttt gtacttttta atgttatttg gaattttaag 8160
ttctatcagt gacttctgaa tccttagaat ggcctctttg tagaaccctg tggtatagag 8220
gagtatggcc actgccccac tatttttatt ttcttatgta agtttgcata tcagtcatga 8280
ctagtgccta gaaagcaatg tgatggtcag gatctcatga cattatattt gagtttcttt 8340
cagatcattt aggatactct taatctcact tcatcaatca aatatttttt gagtgtatgc 8400
tgtagctgaa agagtatgta cgtacgtata agactagaga gatattaagt ctcagtacac 8460
ttcctgtgcc atgttattca gctcactggt ttacaaatat aggttgtctt gtggttgtag 8520
gagcccactg taacaatact gggcagcctt tttttttttt tttttaattg caacaatgca 8580
aaagccaaga aagtataagg gtcacaagtc taaacaatga attcttcaac agggaaaaca 8640
gctagcttga aaacttgctg aaaaacacaa cttgtgttta tggcatttag taccttcaaa 8700

taattggctt tgcagatatt ggatacccca ttaaatctga cagtctcaaa tttttcatct 8760
cttcaatcac tagtcaagaa aaatataaaa acaacaaata cttccatatg gagcattttt 8820
cagagttttc taacccagtc ttatttttct agtcagtaaa catttgtaaa aatactgttt 8880
cactaatact tactgttaac tgtcttgaga gaaaagaaaa atatgagaga actattgttt 8940
ggggaagttc aagtgatctt tcaatatcat tactaacttc ttccactttt tccagaattt 9000
gaatattaac gctaaaggtg taagacttca gatttcaaat taatctttct atatttttta 9060
aatttacaga atattatata acccactgct gaaaaagaaa aaaatgattg ttttagaagt 9120
taaagtcaat attgatttta aatataagta atgaaggcat atttccaata actagtgata 9180
tggcatcgtt gcattttaca gtatcttcaa aaatacagaa tttatagaat aatttctcct 9240
catttaatat ttttcaaaat caaagttatg gtttcctcat tttactaaaa tcgtattcta 9300
attcttcatt atagtaaatc tatgagcaac tccttacttc ggttcctctg atttcaaggc 9360
catattttaa aaaatcaaaa ggcactgtga actattttga agaaaacaca acattttaat 9420
acagattgaa aggacctctt ctgaagctag aaacaatcta tagttataca tcttcattaa 9480
tactgtgtta ccttttaaaa tagtaatttt ttacattttc ctgtgtaaac ctaattgtgg 9540
tagaaatttt taccaactct atactcaatc aagcaaaatt tctgtatatt ccctgtggaa 9600
tgtacctatg tgagtttcag aaattctcaa aatacgtgtt caaaaatttc tgcttttgca 9660
tctttgggac acctcagaaa acttattaac aactgtgaat atgagaaata cagaagaaaa 9720
taataagccc tctatacata aatgcccagc acaattcatt gttaaaaaac aaccaaacct 9780
cacactactg tatttcatta tctgtactga aagcaaatgc tttgtgacta ttaaatgttg 9840
cacatcattc attcactgta tagtaatcat tgactaaagc catttgtctg tgttttcttc 9900
ttgtggttgt atatatcagg taaaatattt tccaaagagc catgtgtcat gtaatactga 9960
accactttga tattgagaca ttaatttgta cccttgttat tatctactag taataatgta 10020
atactgtaga aatattgctc taattctttt caaaattgtt gcatccccct tagaatgttt 10080
ctatttccat aaggatttag gtatgctatt atcccttctt ataccctaag atgaagctgt 10140
ttttgtgctc tttgttcatc attggccctc attccaagca ctttacgctg tctgtaatgg 10200
gatctatttt tgcactggaa tatctgagaa ttgcaaaact agacaaaagt ttcacaacag 10260
atttctaagt taaatcattt tcattaaaag gaaaaaagaa aaaaaatttt gtatgtcaat 10320
aactttatat gaagtattaa aatgcatatt tctatgttgt aatataatga gtcacaaaat 10380
aaagctgtga cagttctgtt ggtctacaga aa 10412

<210> 8
<211> 3473
<212> DNA
<213> Homo sapiens

<400> 8
ttctttccaa gggtctctgg gtgaggcccg tgaccttccc aagcctctcc ctgtcttgtg 60
aaacctgggc gtgatatacc tcccttttag ggctgctgcg atcatttagg cagattaaac 120
ctcataagtg gtttcccata caagaaagat gctagcagtg caacagacag aacacttacc 180
tgcctgccct cccgccagga ggtggtcttc caacttttgc ccggagtcta cagagggtgg 240
gccctctctg ctggggctcc gggacatggt caggagaggt tggtctgtct gtaccgccat 300
tctcttggcc agactgtggt gtctggtccc tactcacacc ttcctgtcag agtatccaga 360
ggccgcagag tatccacacc ctggctgggt gtactggcta cagatggctg tggctccagg 420
tcacctgcgt gcctgggtga tgagaaataa tgtcacaaca aatatcccat ctgcattctc 480
tgggacactg acccatgaag agaaagcagt tctcacagtt tttacaggca cagccacagc 540
cgtgcatgta caggtggcag ctttagcttc tgctaaactg gagagctcag tgtttgtgac 600
agactgcgtg tcctgcaaaa tcgaaaatgt ctgtgattca gctcttcagg gaaaaagggt 660
gccgatgtct ggcctacagg gctcaagcat tgtcatcatg cccccatcca accgtccact 720
cgccagtgcg gcatcctgca cgtggtcagt ccaagtccag ggagggcccc atcacctggg 780
ggtggtcgct atcagtggca aagtcttgtc agcagctcat ggggcaggaa gggcctatgg 840
ttgggggttt cctggcgatc ccatggagga aggatacaag accctcctga aaggaatttc 900
cgggaagttc aatagtggtg agttggtggc cattatgggt ccttccgggg ccgggaagtc 960
cacgctgatg aacatcctgg ctggatacag ggagacgggc atgaaggggg ccgtcctcat 1020
caacggcctg ccccgggacc tgcgctgctt ccggaaggtg tcctgctaca tcatgcagga 1080
tgacatgctg ctgccgcatc tcactgtgca ggaggccatg atggtgtcgg cacatctgaa 1140
gcttcaggag aaggatgaag gcagaaggga aatggtcaag gagatactga cagcgctggg 1200
cttgctgtct tgcgccaaca cgcggaccgg gagcctgtca ggtggtcagc gcaagcgcct 1260
ggccatcgcg ctggagctgg tgaacaaccc tccagtcatg ttcttcgatg agcccaccag 1320
cggcctggac agcgcctcct gcttccaggt ggtctcgctg atgaaagggc tcgctcaagg 1380
gggtcgctcc atcatttgca ccatccacca gcccagcgcc aaactcttcg agctgttcga 1440
ccagctttac gtcctgagtc aaggacaatg tgtgtaccgg ggaaaagtct gcaatcttgt 1500
gccatatttg agggatttgg gtctgaactg cccaacctac cacaacccag cagattttgt 1560
catggaggtt gcatccggcg agtacggtga tcagaacagt cggctggtga gagcggttcg 1620
ggagggcatg tgtgactcag accacaagag agacctcggg ggtgatgccg aggtgaaccc 1680
ttttctttgg caccggccct ctgaagaggt aaagcagaca aaacgattaa aggggttgag 1740
aaaggactcc tcgtccatgg aaggctgcca cagcttctct gccagctgcc tcacgcagtt 1800
ctgcatcctc ttcaagagga ccttcctcag catcatgagg gactcggtcc tgacacacct 1860
gcgcatcacc tcgcacattg ggatcggcct cctcattggc ctgctgtact tggggatcgg 1920
gaacgaagcc aagaaggtct tgagcaactc cggcttcctc ttcttctcca tgctgttcct 1980

catgttcgcg gccctcatgc ctactgttct gacatttccc ctggagatgg gagtctttct 2040

tcgggaacac ctgaactact ggtacagcct gaaggcctac tacctggcca agaccatggc 2100

agacgtgccc tttcagatca tgttcccagt ggcctactgc agcatcgtgt actggatgac 2160

gtcgcagccg tccgacgccg tgcgctttgt gctgtttgcc gcgctgggca ccatgacctc 2220

cctggtggca cagtccctgg gcctgctgat cggagccgcc tccacgtccc tgcaggtggc 2280

cactttcgtg ggcccagtga cagccatccc ggtgctcctg ttctcggggt tcttcgtcag 2340

cttcgacacc atccccacgt acctacagtg gatgtcctac atctcctatg tcaggtatgg 2400

gttcgaaggg gtcatcctct ccatctatgg cttagaccgg gaagatctgc actgtgacat 2460

cgacgagacg tgccacttcc agaagtcgga ggccatcctg cgggagctgg acgtggaaaa 2520

tgccaagctg tacctggact tcatcgtact cgggattttc ttcatctccc tccgcctcat 2580

tgcctatttt gtcctcaggt acaaaatccg ggcagagagg taaaacacct gaatgccagg 2640

aaacaggaag attagacact gtggccgagg gcacgtctag aatcgaggag gcaagcctgt 2700

gcccgaccga cgacacagag actcttctga tccaacccct agaaccgcgt tgggtttgtg 2760

ggtgtctcgt gctcagccac tctgcccagc tgggttggat cttctctcca ttcccctttc 2820

tagctttaac taggaagatg taggcagatt ggtggttttt ttttttttaa catacagaat 2880

tttaaatacc acaactgggg cagaatttaa agctgcaaca cagctggtga tgagaggctt 2940

cctcagtcca gtcgctcctt agcaccaggc accgtgggtc ctggatgggg aactgcaagc 3000

agcctctcag ctgatggctg cacagtcaga tgtctggtgg cagagagtcc gagcatggag 3060

cgattccatt ttatgactgt tgtttttcac attttcatct ttctaaggtg tgtctctttt 3120

ccaatgagaa gtcatttttg caagccaaaa gtcgatcaat cgcattcatt ttaagaaatt 3180

ataccttttt agtacttgct gaagaatgat tcagggtaaa tcacatactt tgtttagaga 3240

ggcgaggggt ttaaccgagt cacccagctg gtctcataca tagacagcac ttgtgaagga 3300

ttgaatgcag gttccaggtg gagggaagac gtggacacca tctccactga gccatgcaga 3360

catttttaaa agctatacaa aaaattgtga gaagacattg gccaactctt tcaaagtctt 3420

tctttttcca cgtgcttctt attttaagcg aaatatattg tttgtttctt cct 3473

<210> 9

<211> 2740

<212> DNA

<213> Homo sapiens

<400> 9
   aagtcccagt cctgctgtcc caagggactc cggggtcagg tggagcaggc agggcagtct 60
   gccacgggct ccccaactga agccactctg gggagggtcc ggccaccaga aaatttgccc 120
   agctttgctg cctgttggcc atgggtgacc tctcatcttt gacccccgga gggtccatgg 180
   gtctccaagt aaacagaggc tcccagagct ccctggaggg ggctcctgcc accgccccgg 240
   agcctcacag cctgggcatc ctccatgcct cctacagcgt cagccaccgc gtgaggccct 300
   ggtgggacat cacatcttgc cggcagcagt ggaccaggca gatcctcaaa gatgtctcct 360
   tgtacgtgga gagcgggcag atcatgtgca tcctaggaag ctcaggctcc gggaaaacca 420
   cgctgctgga cgccatgtcc gggaggctgg ggcgcgcggg gaccttcctg ggggaggtgt 480
   atgtgaacgg ccgggcgctg cgccgggagc agttccagga ctgcttctcc tacgtcctgc 540
   agagcgacac cctgctgagc agcctcaccg tgcgcgagac gctgcactac accgcgctgc 600
   tggccatccg ccgcggcaat cccggctcct tccagaagaa ggtggaggcc gtcatggcag 660
   agctgagtct gagccatgtg gcagaccgac tgattggcaa ctacagcttg gggggcattt 720
   ccacgggtga gcggcgccgg gtctccatcg cagcccagct gctccaggat cctaaggtca 780
   tgctgtttga tgagccaacc acaggcctgg actgcatgac tgctaatcag attgtcgtcc 840
   tcctggtgga actggctcgc aggaaccgaa ttgtggttct caccattcac cagccccgtt 900
   ctgagctttt tcagctcttt gacaaaattg ccatcctgag cttcggagag ctgatttctt 960
   gtggcacgcc agcggaaatg cttgatttct tcaatgactg cggttaccct tgtcctgaac 1020
   attcaaaccc ttttgacttc tatatggacc tgacgtcagt ggatacccaa agcaaggaac 1080
   gggaaataga aacctccaag agagtccaga tgatagaatc tgcctacaag aaatcagcaa 1140
   tttgtcataa aactttgaag aatattgaaa gaatgaaaca cctgaaaacg ttaccaatgg 1200
   ttcctttcaa aaccaaagat tctcctggag ttttctctaa actgggtgtt ctcctgagga 1260
   gagtgacaag aaacttggtg agaaataagc tggcagtgat tacgcgtctc cttcagaatc 1320
   tgatcatggg tttgttcctc cttttcttcg ttctgcgggt ccgaagcaat gtgctaaagg 1380
   gtgctatcca ggaccgcgta ggtctccttt accagtttgt gggcgccacc ccgtacacag 1440
   gcatgctgaa cgctgtgaat ctgtttcccg tgctgcgagc tgtcagcgac caggagagtc 1500
   aggacggcct ctaccagaag tggcagatga tgctggccta tgcactgcac gtcctcccct 1560
   tcagcgttgt tgccaccatg attttcagca gtgtgtgcta ctggacgctg ggcttacatc 1620
   ctgaggttgc ccgatttgga tatttttctg ctgctctctt ggccccccac ttaattggtg 1680
   aatttctaac tcttgtgcta cttggtatcg tccaaaatcc aaatatagtc aacagtgtag 1740
   tggctctgct gtccattgcg ggggtgcttg ttggatctgg attcctcaga aacatacaag 1800
   aaatgcccat tccttttaaa atcatcagtt attttacatt ccaaaaatat tgcagtgaga 1860
   ttcttgtagt caatgagttc tacggactga atttcacttg tggcagctca aatgtttctg 1920
   tgacaactaa tccaatgtgt gccttcactc aaggaattca attcattgag aaaacctgcc 1980
   caggtgcaac atctagattc acaatgaact ttctgatttt gtattcattt attccagctc 2040
   ttgtcatcct aggaatagtt gttttcaaaa taaggatcat tctcattagc aggtagtgaa 2100
   agccatggct gggaaaatgg aagtgaagct gccgactgtg catgactgct ctgaacgtct 2160
   gaaatgagag tgccatgtat ttctttcttg acaggacatc tcaagtcttt taaccattaa 2220
   gactccattt gtgcctcttg gatccaagca ggccttgaat gcaatggaag tggtttatag 2280
   tcccttgctc ttacaacttg cagggacatg tggttatttg gaaattgtga ctgagcggac 2340
   ccaagaatgt aaataatatt cataaaccta tgggagactc gtgtgactat tttttttcct 2400
   tgttctaggc acagaaaaaa ataggtcagc ttaaaaatat gtttacattg gataaaggat 2460
   taggcaaaaa taaaatgttt caaggattcc tgaccataag tgacagagaa agagagttgt 2520
   gggtttagat gaagcaaggt tatcatgcag aattgggtaa gaatgcttct gttcctggaa 2580
   gacccagagt taaatgcaga tgtccacacg aggggtcgga gttacctgat cacatcgaga 2640
   gagtgctggg cagatggatg gtgagcacca ctgctacaga gcacccagtg attttactga 2700
   ggattaaaat aaaaaaccgt aggaatgggc tcaacagtga 2740

<210> 10
<211> 2679
<212> DNA
<213> Homo sapiens

<400> 10
ctccaggaaa cagagtgaag acactggccc tggcaggcag cagctgggtc taagagagct 60
gcagcccagg gtcacagacc tgtgggcccc atggccggga aggcggcaga ggagagaggg 120
ctgccgaaag gggccactcc ccaggatacc tcgggcctcc aggatagatt gttctcctct 180
gaaagtgaca acagcctgta cttcacctac agtggccagc ccaacaccct ggaggtcaga 240
gacctcaact gccaggtgga cctggcctct caggtccctt ggtttgagca gctggctcag 300
ttcaagatgc cctggacatc tcccagctgc cagaattctt gtgagctggg catccagaac 360
ctaagcttca aagtgagaag tgggcagatg ctggccatca tagggagctc aggttgtggg 420
agagcctcct tgctagatgt gatcactggc cgaggtcacg gcggcaagat caagtcaggc 480
cagatctgga tcaatgggca gcccagctcg cctcagctgg tgaggaagtg tgtggcccac 540
gtgcgccagc acaaccagct gctccccaac ttgactgtgc gagagacctt ggccttcatt 600
gcccagatgc ggctgcccag aaccttctcc caggcccagc gtgacaaaag ggtggaggac 660
gtgatcgcgg agctgcggct taggcagtgc gctgacaccc gcgtgggcaa catgtacgtg 720
cgggggttgt cggggggtga gcgcaggaga gtcagcattg gggtgcagct cctgtggaac 780
ccaggaatcc ttattctcga cgaacccacc tctgggctcg acagcttcac agcccacaac 840
ctggtgaaga ccttgtccag gctggccaaa ggcaaccggc tggtgctcat ctccctccac 900
cagcctcgct ctgacatctt caggctgttt gatctggtcc tcctgatgac gtctggcacc 960
cccatctact taggggcggc ccagcacatg gtccagtatt tcacagccat cggctacccc 1020
tgtcctcgct acagcaatcc tgctgacttc tatgtggacc tgaccagcat tgacaggcgc 1080
agcagagagc aggaattggc caccagggag aaggctcagt cactcgcagc cctgtttcta 1140

gaaaaagtgc gtgacttaga tgactttcta tggaaagcag agacgaagga tcttgacgag 1200
gacacctgtg tggaaagcag cgtgacccca ctagacacca actgcctccc gagtcctacg 1260
aagatgcctg gggcggtgca gcagtttacg acgctgatcc gtcgtcagat ttccaacgac 1320
ttccgagacc tgcccaccct cctcatccat ggggcggagg cctgtctgat gtcaatgacc 1380
atcggcttcc tctattttgg ccatgggagc atccagctct ccttcatgga tacagccgcc 1440
ctcttgttca tgatcggtgc tctcatccct ttcaacgtca ttctggatgt catctccaaa 1500
tgttactcag agagggcaat gctttactat gaactggaag acgggctgta caccactggt 1560
ccatatttct ttgccaagat cctcggggag cttccggagc actgtgccta catcatcatc 1620
tacgggatgc ccacctactg gctggccaac ctgaggccag gcctccagcc cttcctgctg 1680
cacttcctgc tggtgtggct ggtggtcttc tgttgcagga ttatggccct ggccgccgcg 1740
gccctgctcc ccaccttcca catggcctcc ttcttcagca atgccctcta caactccttc 1800
tacctcgccg ggggcttcat gataaacttg agcagcctgt ggacagtgcc cgcgtggatt 1860
tccaaagtgt ccttcctgcg gtggtgtttt gaagggctga tgaagattca gttcagcaga 1920
agaacttata aaatgcctct cgggaacctc accatcgcgg tctcaggaga taaaatcctc 1980
agtgccatgg agctggactc gtaccctctc tacgccatct acctcatcgt cattggcctc 2040
agcggtggct tcatggtcct gtactacgtg tccttaaggt tcatcaaaca gaaaccaagt 2100
caagactggt gattcacgcc agacgtctgc ccgctggtgg gggacctgag cagacccttc 2160
aactgcactc cctcctcagg agccccttcc tggggacagt gaggacaatg accctacaga 2220
tgctcagcta catccggccc agggtgctgc ggtggcacag accagccaca ggatggcagt 2280
agaataaaga cagtcgaaag ggatttctgc tcactggcag gagactgcga tgactgggag 2340
aaaacctgca ctcggtggca cctacaacgt tgctaattta tttccttttg atatgcattt 2400
atataggcaa ctcgatatag gatgggagca aactaggaat gaattgggta gctagactgt 2460
gcaggaattg ttggaacctg gagggaacaa taacagtacc tagcagattt ggcttcatct 2520
tccaggggcc ccacactccg tggtgagcca ccatcaatac agaaagtgac ctaagatgta 2580
ccagcaagat gccatccctt ctttttgtgt ggggtcatgg gctccaaaag ccaacgtgaa 2640
caattaaaaa tgtattgagc atctaaaaaa aaaaaaaaa 2679

<210> 11
<211> 1156
<212> DNA
<213> Homo sapiens

<400> 11
cgcagcggag gtgaaggacg tccttcccca ggagccgact ggccaatcac aggcaggaag 60
atgaaggttc tgtgggctgc gttgctggtc acattcctgg caggatgcca ggccaaggtg 120

gagcaagcgg tggagacaga gccggagccc gagctgcgcc agcagaccga gtggcagagc 180
ggccagcgct gggaactggc actgggtcgc ttttgggatt acctgcgctg ggtgcagaca 240
ctgtctgagc aggtgcagga ggagctgctc agctcccagg tcacccagga actgagggcg 300
ctgatggacg agaccatgaa ggagttgaag gcctacaaat cggaactgga ggaacaactg 360
accccggtgg cggaggagac gcgggcacgg ctgtccaagg agctgcaggc ggcgcaggcc 420
cggctgggcg cggacatgga ggacgtgtgc ggccgcctgg tgcagtaccg cggcgaggtg 480
caggccatgc tcggccagag caccgaggag ctgcgggtgc gcctcgcctc ccacctgcgc 540
aagctgcgta agcggctcct ccgcgatgcc gatgacctgc agaagcgcct ggcagtgtac 600
caggccgggg cccgcgaggg cgccgagcgc ggcctcagcg ccatccgcga gcgcctgggg 660
cccctggtgg aacagggccg cgtgcgggcc gccactgtgg gctccctggc cggccagccg 720
ctacaggagc gggcccaggc ctggggcgag cggctgcgcg cgcggatgga ggagatgggc 780
agccggaccc gcgaccgcct ggacgaggtg aaggagcagg tggcggaggt gcgcgccaag 840
ctggaggagc aggcccagca gatacgcctg caggccgagg ccttccaggc ccgcctcaag 900
agctggttcg agcccctggt ggaagacatg cagcgccagt gggccgggct ggtggagaag 960
gtgcaggctg ccgtgggcac cagcgccgcc cctgtgccca gcgacaatca ctgaacgccg 1020
aagcctgcag ccatgcgacc ccacgccacc ccgtgcctcc tgcctccgcg cagcctgcag 1080
cgggagaccc tgtccccgcc ccagccgtcc tcctggggtg gaccctagtt taataaagat 1140
tcaccaagtt tcacgc 1156

<210> 12
<211> 417
<212> DNA
<213> Homo sapiens

<400> 12
acctcccaac caagccctcc agcaaggatt caggagtgcc cctcgggcct cgccatgagg 60
ctcttcctgt cgctcccggt cctggtggtg gttctgtcga tcgtcttgga aggcccagcc 120
ccagcccagg ggaccccaga cgtctccagt gccttggata agctgaagga gtttggaaac 180
acactggagg acaaggctcg ggaactcatc agccgcatca aacagagtga actttctgcc 240
aagatgcggg agtggttttc agagacattt cagaaagtga aggagaaact caagattgac 300
tcatgaggac ctgaagggtg acatccagga ggggcctctg aaatttccca caccccagcg 360
cctgtgctga ggactcccgc catgtggccc caggtgccac caataaaaat cctaccg 417

<210> 13
<211> 753
<212> DNA

<213>  Homo sapiens

<400>  13
gttgtggctg tggagcggaa gtgggtctca accactataa atcctctctg tgcccgtccg      60
gagctggtga ggacagcctg ccagagtctg gtctctggac actatgggca cacgactcct     120
cccagctctg tttcttgtcc tcctggtatt gggatttgag gtccagggga cccaacagcc     180
ccagcaagat gagatgccta gcccgacctt cctcacccag gtgaaggaat ctctctccag     240
ttactgggag tcagcaaaga cagccgccca gaacctgtac gagaagacat acctgcccgc     300
tgtagatgag aaactcaggg acttgtacag caaaagcaca gcagccatga gcacttacac     360
aggcattttt actgaccaag ttctttctgt gctgaaggga gaggagtaac agccagaccc     420
cccatcagtg gacaagggga gagtccccta ctcccctgat cccccaggtt cagactgagc     480
tcccccttcc cagtagctct tgcatcctcc tcccaactct agcctgaatt cttttcaata     540
aaaaatacaa ttcaagttgc ttctcatgga tggcactgct tttctgagga ctcaagggcc     600
aagatggagg ggctgactca gtccagccaa catttaatga gcacctactt tatgtatgga     660
gctctaaccc atgggtccat gggaataaag cagtgaatag taacaataaa taatcgtaac     720
aaaaaaaaaa aaaaaaaaaa aaaaaaaaaa aaa                                  753

<210>  14
<211>  5950
<212>  DNA
<213>  Homo sapiens

<400>  14
gaattccagg ggacatctgt tgcgcaccta ctgtgctcac gctggggcct ctgggatgga      60
caggattctg ccaaggcaga catctgggtc aagacagtcc tgcacagttg ttcaggttgt     120
ggccaaggtt gcgtttgcag atttgccatg taaaaataca ggatgctcag ttacatttga     180
atttcagatt aatagcaaaa aaaacttttt ggtataattc tgaaatattt catgggacat     240
atttatacta aaacgtcatg cactgttgat ttgaaattca aatgtaattg ggcctcctct     300
atttcgtctg gcaagcgtag acaaaagaat ccagtccagg ccaggcgcag tggctcaagc     360
ctgtaatccc agcactttgg gaggccgagg cgggcggatc acgaggtcag gagatcgaga     420
ccatcctggc taacacggtg aaaccccgtc tctactaaaa atacaaaaaa ttagctgggc     480
gtggtggcgg gtgcctgtag tcccagctac tcgggaggct gaggcaggag aatggcgtga     540
acctgggagg cggagcttgc agtgagccga gatcgcgcca cagcactcca gcctgggcga     600
cagagccaga ctctgtctca aaaaaaaaaa aaagaatcca gtccatagtc ccctgagcca     660
tgtgccctgg ggtgcagctg ggtccttcag gagaaaaatg ctctatttct ggcactggga     720

ccgagcctga tgtgggtttt ttgttggttt ttgttgttgt tattgttttt gagacaaggt 780
ctcgctccac cacacccggc taatttttgt atttttagta gagacggggt ttcactacgt 840
tggccaggct ggtcttgaac tcctgacctc aagtgagccg cctgcctcgg cctcccaaag 900
tgctgggatt acaggtggga gccaccgccc tggccctggg cctgatgttg atgacctcct 960
actatgtgca cctgcagctc tcctgcatag gcctcagccg tcctgcatga ggacactggg 1020
aggcaggtgc tccctatcaa ccccgtgtta cagttgaaca aactgagccc cagaaaagaa 1080
aacgtatttg cccaggtcac acggtgaaga agtgagggat tcgaagccca ggtccatctg 1140
aagccagagt cacccagagg agaaagagtt ggaattgaga actcaaggaa tgcttggaag 1200
tgatcgggct cgagcccacc taggaagaaa cagaggctgg agacatgaga ctgtgttgct 1260
atttcctctc atcaaccctt gggccctatt gaggccctac cacaagcctg gccctgcagc 1320
ccagtgacta ggagaaatta gacacaagat aataataaca gcaatgatct tttttttttt 1380
tctgagacgg agtcttgctc tttcgcccag gctggactgc agtggcgcga tctcggctca 1440
atgcaagctc cacctcccag gttcacgcca ttctcctgcc tcagcctccc gagtagctag 1500
gactacaggc gcctgccacc acgcctggct aatttttcat atttttagta gagatggggt 1560
ttcaccgtgt tagccaagat ggtctcaatc tcctgacctc gtgatccgcc tgcctcggcc 1620
tcccaaagtg ttggggttac aggcatgagc caccgcgcct ggccaacagc aatgatcttt 1680
gagcacctat attgccagtc tccacggtaa gagctttctt cattttttgt tttgttttgt 1740
ttcaagacag agtcttgctc tgtcacccag gctggagtgc agtggtgtga tcgcggctca 1800
ctgcagcctt cacttcccgg gttcaagcca ttctcctgcc tcagcctccc aagtagctgg 1860
gattacaggc acgcatcact acttctggct aatttttgta tttttagtag ggacagggtt 1920
tttcaccatg ttggccaggt tggtctcaaa ctcctggcct catatgatct gcccacctcg 1980
gcctcccaaa gtgctgggat tacaggcgtg agccactgcg cctttctttg tatttgttca 2040
agtaatatac tgaaatatgt actgtgcctc ccactttatg gaggaggaaa ctgaggccag 2100
caaatgaggc tgtcatggga ggtggagaca ggatttgaac ctgcctcagt gcaggaggct 2160
caagagcctc tgtcttctct cagggcactg tgtgggaggg tgagaaggag ggaggcccac 2220
agaggcatga cctctgattg ccactgtcac ctgggccctg ctctctgaag tctctgccaa 2280
gcggggaggt ggccggggga gggccctgct ctgtgcagcc tcccctcccc cggcccgcag 2340
agttgagcac agagggacag aggcacggaa cccccagaaa tgtccctcct cagaaacagg 2400
ctccaggccc tgcctgccct gtgcctctgc gtgctggtcc tggcctgcat tgggggtgag 2460
aagaagtggg tggagggatg tggggcccac acctggtggg tgtgagtgtg gctgtgtgtc 2520
ctgtggctct gtagccacgt gagacatgag tacggagtgt gtgcgtttca tggcgtgcgt 2580
atgcatgtgc gtgtcgggga gtgtgtgtgt cggtggctga gagtgaagtg tgaatgtcac 2640
attggtacaa actgggatca tctgtgtgtg tgcacgtgcg tgcgtggaag tgggagtatg 2700
cagtcgtggt aaaaaagtgc atgtctgtgt gcatatgtgt atttgtgtgc acctgtctct 2760

ctgtggggta tgtgtgtgca aaatatttga gtgtgtggac atgtgtgagg gggtgagtgt 2820
gtgctggtgt gtacgtctgt gttttgcata tgcatttttt tttttttttt ttagacggag 2880
tctcactctg tcacccaggc tggagtgcag tggtagcagt ggtgcgatct tggctcactg 2940
catcatccgc ctacccgttt caagggattc tcctgcctca gtcttcagag tatttgggac 3000
tacagacaca cgccaccatg cctggctaat tttttttttt tgagacggag tctcgctctg 3060
ttacccaggc tggagtgcag tggcgtgatc ttggctcact gcaagctccg cctcccgggt 3120
tcacgccatt ctcctgcctc agcctcccga gtagctggga ctacaggagc ccaccaccac 3180
gcctggctaa ttttttgtat ttttactaga gacggggttt cgccgtgtta gccaggatgg 3240
tctccatatc ctgacctcgt gatccgcctg cctcggcctt ccaaagtgct aggattatag 3300
gcgtgagcca ctgcgcctgg ccaatgcctg gctaattttt ttatattttt ggtagagaca 3360
gggttttgcc atgttgccca ggctggtctt gaaatcctga cctcaggtga tccgcccgcc 3420
ttggcctccc aaagtgctgg gattacaggc atgagccacc acgcccggcc atgtacttta 3480
tgttaaaatg ggatcatatt ctagatcagc attatccagt agaaatttaa atttttaata 3540
cagggccagg cacggtggct catgcctgta atcccagcac tttcggaggc cgaggcgggt 3600
ggatcgcaag gtcaggagat ttgagatcat cctggctaac agatgggtaa aaacccatct 3660
ctactaaaaa tacaaaaaat tagccatgca tggtggcatg cgcctgtagt cccagctact 3720
cgggaggctg aggccggaga atcacttgaa cccgggaggc agaggttgca gtgagccgag 3780
atcgcgccac tgcattccaa cctgggtgac agagcgagac tccgtctgaa aaaaaaaaaa 3840
aatttaacac gtatgtagac aatgtgcaag gcaccattcc atgtgcatcg tatgtagtaa 3900
ctcttaattc tcacgataac cctgaggtag atattattac cccgttctac aaaaggagaa 3960
acagtcctgg ggagacagga taagtcaccg gccaaggcac acagctagct acatgtggcc 4020
cccgcgtgac ggctggtctc tgtaggcgag gctttgtcca gatgcgtggg tagaaggtct 4080
ggcccggaaa gaggaactga cagcaaggct aagccaatgt ctgcccctgg gggcagaaag 4140
tcacctcctg ctctccctcc actgtccaca gaggtagctc agacagggtg ggggtcacag 4200
gagaacgaag ggagaagggg gtagttcctg ggcagcaaaa tcaggtggtg aagggaggca 4260
tcagaggatg gcaattagag aggccattag aggggaacca caggcagaca gggtgacagg 4320
agggactact gacacaaggt gaagagatgg cccagccgga cggggtggct cacatctgta 4380
atcccagcat tttgggagcc cgaggtgggt ggatcacttg aggtcaggag ttcgaggccc 4440
caacatggca aaaccccatc tcttctaaaa atacaaaaat tagccgggca tgatggcaga 4500
tgcctgtaat ccctgctact cgggaggctg aggcaggaaa attgcctgaa tccaggaggt 4560
ggaggttgca atgagacgag atcatgacac tgcactccac cctgggcaac agagcaagag 4620
actgactctg tctcataaaa aaaaagaaaa aagaaaaaaa aaaagagatg gctgatggtt 4680
aaagaggggt tagcggtcag ggacacatat agggtaaagg caggaggcaa gaggactggc 4740
agggggctgc ccctgggcca ccgggagcga cacaggatga gcatggaggg aaagggagaa 4800

ggggattcta gggtcccagc ctacccaagt tgccctctgg ttccacctag catgccagcc 4860

agaggcccag gaaggaaccc tgagcccccc accaaagcta aagatgagtc gctggagcct 4920

ggtgaggggc aggatgaagg agctgctgga gacagtggtg aacaggacca gagacgggtg 4980

gcaatggttc tggtgagggt gtgctggcct gggtggtggg aggggactcc tgggtctgag 5040

ggaggagggg ctggggcctg gacccctgag tctcagggag gaggaaaggg tgggagtggg 5100

gctgtgaccc ctaggtctgg gaggagtgga gggttagagc tgagagcagg aactcctagg 5160

tcacagagag gagcggataa atggggcaga gaacacctgg ggagagctgg ggcctccact 5220

gtgatgtcct ctctcctgta ggagcccgag caccttccgg ggcttcatgc agacctacta 5280

tgacgaccac ctgagggacc tgggtccgct caccaaggcc tggttcctcg aatccaaaga 5340

cagcctcttg aagaagaccc acagcctgtg ccccaggctt gtctgtgggg acaaggacca 5400

gggttaaaat gttcataaaa gccaggtgtg gttgtggcgg gtgcctgtag tcccagctac 5460

tcaggaggct gaggtaggat gatggcttga gcccaggagt tcgagaccag cctgggcaac 5520

acagcgagat ctcttggggg taaaacaaaa agaaaaaaaa aagttcatac ttctccaata 5580

aataaagtct cacctgtgtc cctgtctgga tccttcccca gtgtggccag aaaaaaaccc 5640

accccactgc ctcccaggaa tcaatgagta gaagaggtga cacctgatgg ggaaggaaga 5700

gtagggaggt cgggaagggt atcaaggaat aacaccctat tgtgggcttg cggagaatgg 5760

gggacttcaa ggcgtgtcag tttcaggagg gtgagggcag gagcgtgggt ggagtcagca 5820

ggtccccatg atggccctca ctgagagctt cgcccttgtc tcctacaagc tctgactcca 5880

ttcccagtgg gcacccagca cctccaaccc ctccacagcc cccaacccag cctctgtcgg 5940

aggcgaattc 5950

<210> 15

<211> 3549

<212> DNA

<213> Homo sapiens

<400> 15
cccctcttcc tcctcctcaa gggaaagctg cccacttcta gctgccctgc catccccttt 60

aaagggcgac ttgctcagcg ccaaaccgcg gctccagccc tctccagcct ccggctcagc 120

cggctcatca gtcggtccgc gccttgcagc tcctccagag ggacgcgccc cgagatggag 180

agcaaagccc tgctcgtgct gactctggcc gtgtggctcc agagtctgac cgcctcccgc 240

ggaggggtgg ccgccgccga ccaaagaaga gattttatcg acatcgaaag taaatttgcc 300

ctaaggaccc ctgaagacac agctgaggac acttgccacc tcattcccgg agtagcagag 360

tccgtggcta cctgtcattt caatcacagc agcaaaacct tcatggtgat ccatggctgg 420

acggtaacag gaatgtatga gagttgggtg ccaaaacttg tggccgccct gtacaagaga 480

gaaccagact ccaatgtcat tgtggtggac tggctgtcac gggctcagga gcattaccca 540
gtgtccgcgg gctacaccaa actggtggga caggatgtgg cccggtttat caactggatg 600
gaggaggagt ttaactaccc tctggacaat gtccatctct tgggatacag ccttggagcc 660
catgctgctg gcattgcagg aagtctgacc aataagaaag tcaacagaat tactggcctc 720
gatccagctg gacctaactt tgagtatgca gaagccccga gtcgtctttc tcctgatgat 780
gcagattttg tagacgtctt acacacattc accagagggt cccctggtcg aagcattgga 840
atccagaaac cagttgggca tgttgacatt tacccgaatg gaggtacttt tcagccagga 900
tgtaacattg gagaagctat ccgcgtgatt gcagagagag gacttggaga tgtggaccag 960
ctagtgaagt gctcccacga gcgctccatt catctcttca tcgactctct gttgaatgaa 1020
gaaaatccaa gtaaggccta caggtgcagt tccaaggaag cctttgagaa agggctctgc 1080
ttgagttgta gaaagaaccg ctgcaacaat ctgggctatg agatcaataa agtcagagcc 1140
aaaagaagca gcaaaatgta cctgaagact cgttctcaga tgccctacaa agtcttccat 1200
taccaagtaa agattcattt ttctgggact gagagtgaaa cccataccaa tcaggccttt 1260
gagatttctc tgtatggcac cgtggccgag agtgagaaca tcccattcac tctgcctgaa 1320
gtttccacaa ataagaccta ctccttccta atttacacag aggtagatat tggagaacta 1380
ctcatgttga agctcaaatg gaagagtgat tcatacttta gctggtcaga ctggtggagc 1440
agtcccggct tcgccattca gaagatcaga gtaaaagcag gagagactca gaaaaaggtg 1500
atcttctgtt ctagggagaa agtgtctcat ttgcagaaag gaaaggcacc tgcggtattt 1560
gtgaaatgcc atgacaagtc tctgaataag aagtcaggct gaaactgggc gaatctacag 1620
aacaaagaac ggcatgtgaa ttctgtgaag aatgaagtgg aggaagtaac ttttacaaaa 1680
catacccagt gtttggggtg tttcaaaagt ggattttcct gaatattaat cccagcccta 1740
cccttgttag ttattttagg agacagtctc aagcactaaa aagtggctaa ttcaatttat 1800
ggggtatagt ggccaaatag cacatcctcc aacgttaaaa gacagtggat catgaaaagt 1860
gctgttttgt cctttgagaa agaaataatt gtttgagcgc agagtaaaat aaggctcctt 1920
catgtggcgt attgggccat agcctataat tggttagaac ctcctatttt aattggaatt 1980
ctggatcttt cggactgagg ccttctcaaa ctttactcta agtctccaag aatacagaaa 2040
atgcttttcc gcggcacgaa tcagactcat ctacacagca gtatgaatga tgttttagaa 2100
tgattccctc ttgctattgg aatgtggtcc agacgtcaac caggaacatg taacttggag 2160
agggacgaag aaagggtctg ataaacacag aggttttaaa cagtccctac cattggcctg 2220
catcatgaca aagttacaaa ttcaaggaga tataaaatct agatcaatta attcttaata 2280
ggctttatcg tttattgctt aatccctctc tcccccttct tttttgtctc aagattatat 2340
tataataatg ttctctgggt aggtgttgaa aatgagcctg taatcctcag ctgacacata 2400
atttgaatgg tgcagaaaaa aaaaagatac cgtaatttta ttattagatt ctccaaatga 2460
ttttcatcaa tttaaaatca ttcaatatct gacagttact cttcagtttt aggcttacct 2520

tggtcatgct tcagttgtac ttccagtgcg tctcttttgt tcctggcttt gacatgaaaa 2580

gataggtttg agttcaaatt ttgcattgtg tgagcttcta cagattttag acaaggaccg 2640

tttttactaa gtaaaagggt ggagaggttc ctggggtgga ttcctaagca gtgcttgtaa 2700

accatcgcgt gcaatgagcc agatggagta ccatgagggt tgttatttgt tgtttttaac 2760

aactaatcaa gagtgagtga acaactattt ataaactaga tctcctattt ttcagaatgc 2820

tcttctacgt ataaatatga aatgataaag atgtcaaata tctcagaggc tatagctggg 2880

aacccgactg tgaaagtatg tgatatctga acacatacta gaaagctctg catgtgtgtt 2940

gtccttcagc ataattcgga agggaaaaca gtcgatcaag ggatgtattg gaacatgtcg 3000

gagtagaaat tgttcctgat gtgccagaac ttcgaccctt tctctgagag agatgatcgt 3060

gcctataaat agtaggacca atgttgtgat taacatcatc aggcttggaa tgaattctct 3120

ctaaaaataa aatgatgtat gatttgttgt tggcatcccc tttattaatt cattaaattt 3180

ctggatttgg gttgtgaccc agggtgcatt aacttaaaag attcactaaa gcagcacata 3240

gcactgggaa ctctggctcc gaaaaacttt gttatatata tcaaggatgt tctggcttta 3300

cattttattt attagctgta aatacatgtg tggatgtgta aatggagctt gtacatattg 3360

gaaaggtcat tgtggctatc tgcatttata aatgtgtggt gctaactgta tgtgtcttta 3420

tcagtgatgg tctcacagag ccaactcact cttatgaaat gggctttaac aaaacaagaa 3480

agaaacgtac ttaactgtgt gaagaaatgg aatcagcttt taataaaatt gacaacattt 3540

tattaccac 3549

<210> 16

<211> 1790

<212> DNA

<213> Homo sapiens

<400> 16
gtgaatctct ggggccagga agaccctgct gcccggaaga gcctcatgtt ccgtgggggc 60

tgggcggaca tacatatacg ggctccaggc tgaacggctc gggccactta cacaccactg 120

cctgataacc atgctggctg ccacagtcct gaccctggcc ctgctgggca atgcccatgc 180

ctgctccaaa ggcacctcgc acgaggcagg catcgtgtgc cgcatcacca agcctgccct 240

cctggtgttg aaccacgaga ctgccaaggt gatccagacc gccttccagc gagccagcta 300

cccagatatc acgggcgaga aggccatgat gctccttggc caagtcaagt atgggttgca 360

caacatccag atcagccact tgtccatcgc cagcagccag gtggagctgg tggaagccaa 420

gtccattgat gtctccattc agaacgtgtc tgtggtcttc aaggggaccc tgaagtatgg 480

ctacaccact gcctggtggc tgggtattga tcagtccatt gacttcgaga tcgactctgc 540

cattgacctc cagatcaaca cacagctgac ctgtgactct ggtagagtgc ggaccgatgc 600

ccctgactgc tacctgtctt tccataagct gctcctgcat ctccaagggg agcgagagcc 660
tgggtggatc aagcagctgt tcacaaattt catctccttc accctgaagc tggtcctgaa 720
gggacagatc tgcaaagaga tcaacgtcat ctctaacatc atggccgatt ttgtccagac 780
aagggctgcc agcatccttt cagatggaga cattggggtg gacatttccc tgacaggtga 840
tcccgtcatc acagcctcct acctggagtc ccatcacaag ggtcatttca tctacaagaa 900
tgtctcagag gacctccccc tccccacctt ctcgcccaca ctgctggggg actcccgcat 960
gctgtacttc tggttctctg agcgagtctt ccactcgctg gccaaggtag ctttccagga 1020
tggccgcctc atgctcagcc tgatgggaga cgagttcaag gcagtgctgg agacctgggg 1080
cttcaacacc aaccaggaaa tcttccaaga ggttgtcggc ggcttcccca gccaggccca 1140
agtcaccgtc cactgcctca agatgcccaa gatctcctgc caaaacaagg gagtcgtggt 1200
caattcttca gtgatggtga aattcctctt tccacgccca gaccagcaac attctgtagc 1260
ttacacattt gaagaggata tcgtgactac cgtccaggcc tcctattcta agaaaaagct 1320
cttcttaagc ctcttggatt tccagattac accaaagact gtttccaact tgactgagag 1380
cagctccgag tccatccaga gcttcctgca gtcaatgatc accgctgtgg gcatccctga 1440
ggtcatgtct cggctcgagg tagtgtttac agccctcatg aacagcaaag gcgtgagcct 1500
cttcgacatc atcaaccctg agattatcac tcgagatggc ttcctgctgc tgcagatgga 1560
ctttggcttc cctgagcacc tgctggtgga tttcctccag agcttgagct agaagtctcc 1620
aaggaggtcg ggatggggct tgtagcagaa ggcaagcacc aggctcacag ctggaaccct 1680
ggtgtctcct ccagcgtggt ggaagttggg ttaggagtac ggagatggag attggctccc 1740
aactcctccc tatcctaaag gcccactggc attaaagtgc tgtatccaag 1790

<210> 17
<211> 2688
<212> DNA
<213> Homo sapiens

<400> 17
tttaaagctg ggaggttctg ccaccaagca cggccttccc actgggaaca caaacttgct 60
ggcgggaaga gcccggaaag aaacctgtgg atctcccttc gagatcatcc aaagagaaga 120
aaggtgacct cacattcgtg ccccttagca gcactctgca gaaatgcctc ctcagctgca 180
aaacggcctg aacctctcgg ccaaagttgt ccagggaagc ctggacagcc tgccccaggc 240
agtgagggag tttctcgaga ataacgctga gctgtgtcag cctgatcaca tccacatctg 300
tgacggctct gaggaggaga atgggcggct tctgggccag atggaggaag agggcatcct 360
caggcggctg aagaagtatg acaactgctg gttggctctc actgacccca gggatgtggc 420
caggatcgaa agcaagacgg ttatcgtcac ccaagagcaa agagacacag tgcccatccc 480

caaaacaggc ctcagccagc tcggtcgctg gatgtcagag gaggattttg agaaagcgtt 540
caatgccagg ttcccagggt gcatgaaagg tcgcaccatg tacgtcatcc cattcagcat 600
ggggccgctg ggctcgcctc tgtcaaagat cggcatcgag ctgacggatt caccctacgt 660
ggtggccagc atgcggatca tgacgcggat gggcacgccc gtcctggaag cactgggcga 720
tggggagttt gtcaaatgcc tccattctgt ggggtgccct ctgcctttac aaaagccttt 780
ggtcaacaac tggccctgca acccggagct gacgctcatc gcccacctgc ctgaccgcag 840
agagatcatc tcctttggca gtgggtacgg cgggaactcg ctgctcggga agaagtgctt 900
tgctctcagg atggccagcc ggctggccaa ggaggaaggg tggctggcag agcacatgct 960
ggttctgggt ataaccaacc ctgagggtga gaagaagtac ctggcggccg catttcccag 1020
cgcctgcggg aagaccaacc tggccatgat gaaccccagc ctccccgggt ggaaggttga 1080
gtgcgtcggg gatgacattg cctggatgaa gtttgacgca caaggtcatt taagggccat 1140
caacccagaa aatggctttt tcggtgtcgc tcctgggact tcagtgaaga ccaaccccaa 1200
tgccatcaag accatccaga agaacacaat ctttaccaat gtggccgaga ccagcgacgg 1260
gggcgtttac tgggaaggca ttgatgagcc gctagcttca ggtgtcacca tcacgtcctg 1320
gaagaataag gagtggagct cagaggatgg ggaaccttgt gcccacccca actcgaggtt 1380
ctgcacccct gccagccagt gccccatcat tgatgctgcc tgggagtctc cggaaggtgt 1440
tcccattgaa ggcattatct ttggaggccg tagacctgct ggtgtccctc tagtctatga 1500
agctctcagc tggcaacatg gagtctttgt gggggcggcc atgagatcag aggccacagc 1560
ggctgcagaa cataaaggca aaatcatcat gcatgacccc tttgccatgc ggcccttctt 1620
tggctacaac ttcggcaaat acctggccca ctggcttagc atggcccagc acccagcagc 1680
caaactgccc aagatcttcc atgtcaactg gttccggaag gacaaggaag gcaaattcct 1740
ctggccaggc tttggagaga actccagggt gctggagtgg atgttcaacc ggatcgatgg 1800
aaaagccagc accaagctca cgcccatagg ctacatcccc aaggaggatg ccctgaacct 1860
gaaaggcctg gggcacatca acatgatgga gcttttcagc atctccaagg aattctggga 1920
gaaggaggtg gaagacatcg agaagtatct ggaggatcaa gtcaatgccg acctcccctg 1980
tgaaatcgag agagagatcc ttgccttgaa gcaaagaata agccagatgt aatcagggcc 2040
tgagtgcttt acctttaaaa tcattccctt tcccatccat aaggtgcagt aggagcaaga 2100
gagggcaagt gttcccaaat tgacgccacc ataataatca tcaccacacc gtgagcagat 2160
ctgaaaggca cactttgatt tttttaagga taagaaccac agaacactgg gtagtagcta 2220
atgaaattga gaagggaaat cttagcatgc ctccaaaaat tcacatccaa tgcatagttt 2280
gttcaaattt aaggttactc aggcattgat cttttcagtg ttttttcact ttagctatgt 2340
ggattagcta gaatgcacac caaaaaaata cttgagctgt atatatatat gtgtgtgtgt 2400
gtgtgtgtgt gtgtgtgtgt gtgtgcatgt atgtgcacat gtgtctgtgt ggtatatttg 2460
tgtatgtgta tttgtatgta ctgttattga aaatatattt aatacctttg gaaaaatctt 2520

gggcaagatg acctactagt tttccttgaa aaaaagttgc tttgttatta atattgtgct 2580
taaattattt ttatacacca ttgttcctta cctttacata attgcaatat ttccccctta 2640
ctacttcttg gaaaaaaatt acaaaatgaa gttttataga aaagatgg 2688

<210> 18
<211> 3095
<212> DNA
<213> Homo sapiens

<400> 18
tagcagagca atcaccacca agcctggaat aactgcaagg gctctgctga catcttcctg 60
aggtgccaag gaaatgagga tggaggaagg aatgaatgtt ctccatgact ttgggatcca 120
gtcaacacat tacctccagg tgaattacca agactcccag gactggttca tcttggtgtc 180
cgtgatcgca gacctcagga atgccttcta cgtcctcttc cccatctggt tccatcttca 240
ggaagctgtg ggcattaaac tcctttgggt agctgtgatt ggagactggc tcaacctcgt 300
ctttaagtgg attctctttg gacagcgtcc atactggtgg gttttggata ctgactacta 360
cagcaacact tccgtgcccc tgataaagca gttccctgta acctgtgaga ctggaccagg 420
gagcccctct ggccatgcca tgggcacagc aggtgtatac tacgtgatgg tcacatctac 480
tctttccatc tttcagggaa agataaagcc gacctacaga tttcggtgct tgaatgtcat 540
tttgtggttg ggattctggg ctgtgcagct gaatgtctgt ctgtcacgaa tctaccttgc 600
tgctcatttt cctcatcaag ttgttgctgg agtcctgtca ggcattgctg ttacagaaac 660
tttcagccac atccacagca tctataatgc cagcctcaag aaatattttc tcattacctt 720
cttcctgttc agcttcgcca tcggatttta tctgctgctc aagggactgg gtgtagacct 780
cctgtggact ctggagaaag cccagaggtg gtgcgagcag ccagaatggg tccacattga 840
caccacaccc tttgccagcc tcctcaagaa cctgggcacg ctctttggcc tggggctggc 900
tctcaactcc agcatgtaca gggagagctg caaggggaaa ctcagcaagt ggctcccatt 960
ccgcctcagc tctattgtag cctccctcgt cctcctgcac gtctttgact ccttgaaacc 1020
cccatcccaa gtcgagctgg tcttctacgt cttgtccttc tgcaagagtg cggtagtgcc 1080
cctggcatcc gtcagtgtca tcccctactg cctcgcccag gtcctgggcc agccgcacaa 1140
gaagtcgttg taagagatgt ggagtcttcg gtgtttaaag tcaacaacca tgccagggat 1200
tgaggaggac tactatttga agcaatgggc actggtattt ggagcaagtg acatgccatc 1260
cattctgccg tcgtggaatt aaatcacgga tggcagattg gagggtcgcc tggcttattc 1320
ccatgtgtga ctccagcctg ccctcagcac agactctttc agatggaggt gccatatcac 1380
gtacaccata tgcaagtttc ccgccaggag gtcctcctct ctctacttga atactctcac 1440
aagtagggag ctcactccca ctggaacagc ccattttatc tttgaatggt cttctgccag 1500

cccattttga ggccagaggt gctgtcagct caggtggtcc tcttttacaa tcctaatcat 1560
attgggtaat gtttttgaaa agctaatgaa gctattgaga aagacctgtt gctagaagtt 1620
gggttgttct ggattttccc ctgaagactt acttattctt ccgtcacata tacaaaagca 1680
agacttccag gtagggccag ctcacaagcc caggctggag atcctaactg agaattttct 1740
acctgtgttc attcttaccg agaaaaggag aaaggagctc tgaatctgat aggaaaagaa 1800
ggctgcctaa ggaggagttt ttagtatgtg gcgtatcatg caagtgctat gccaagccat 1860
gtctaaatgg ctttaattat atagtaatgc actctcagta atgggggacc agcttaagta 1920
taattaatag atggttagtg gggtaattct gcttctagta ttttttttac tgtgcataca 1980
tgttcatcgt atttccttgg atttctgaat ggctgcagtg acccagatat tgcactaggt 2040
caaaacattc aggtatagct gacatctcct ctatcacatt acatcatcct ccttataagc 2100
ccagctctgc tttttccaga ttcttccact ggctccacat ccaccccact ggatcttcag 2160
aaggctagag ggcgactctg gtggtgcttt tgtatgtttc aattaggctc tgaaatcttg 2220
ggcaaaatga caaggggagg gccaggattc ctctctcagg tcactccagt gttactttta 2280
attcctagag ggtaaatatg actcctttct ctatcccaag ccaaccaaga gcacattctt 2340
aaaggaaaag tcaacatctt ctctcttttt tttttttttt gagacagggt ctcactatgt 2400
tgcccaggct gctcttgaat tcctgggctc aagcagtcct cccaccctac cacagcgtcc 2460
cgcgtagctg gcatacaggt gcaagccact atgtccagct agccaactcc tccttgcctg 2520
cttttctttt tttttctttt tttgagacgg cgcacctatc acccaggctg gagtggagtg 2580
gcacgatctt ggctcactgc aacctcttcc tcctggttca agcgattctc atgtctcagc 2640
ctcctcagta gctaggacta ccggcgtgca ccaccatgcc aggctaattt ttatattttt 2700
agaattttag aagagatggg atttcatcat gttggccagg ctggtctcga actcctgacc 2760
tcaagtgatc cacctgcctt ggcctcccaa ggtgctagga ttacaggcat gagccaccgc 2820
accgggccct ccttgcctgt tttcaatctc catctgatat gcagagtatt tctgccccac 2880
ccacctaccc cccaaaaaaa gctgaagcct atttatttga aagtccttgt ttttgctact 2940
aattatatag tataccatac attatcattc aaaacaacca tcctgctcat aacatctttg 3000
aaaagaaaaa tatatatgtg cagtatttta ttaaagcaac attttattta agaataaagt 3060
cttgttaatt actatatttt agatgcaatg tgatc 3095

<210> 19

<211> 2128

<212> DNA

<213> Homo sapiens


<400> 19
gggggtccca tcgggcccgc cctcgcacgt cactccggga cccccgcggc ctccgcaggt 60

tctgcgctcc aggccggagt cagagactcc aggatcggtt ctttcatctt cgccgcccct 120
gcgcgtccag ctcttctaag acgagatgcc gtcgggcttc caacagatag gctccgaaga 180
tggggaaccc cctcagcagc gagtgactgg gaccctggtc cttgctgtgt tctctgcggt 240
gcttggctcc ctgcagtttg ggtacaacat tggggtcatc aatgcccctc agaaggtgat 300
tgaacagagc tacaatgaga cgtggctggg gaggcagggg cctgagggac ccagctccat 360
ccctccaggc accctcacca ccctctgggc cctctccgtg gccatctttt ccgtgggcgg 420
catgatttcc tccttcctca ttggtatcat ctctcagtgg cttggaagga aaagggccat 480
gctggtcaac aatgtcctgg cggtgctggg gggcagcctc atgggcctgg ccaacgctgc 540
tgcctcctat gaaatgctca tccttggacg attcctcatt ggcgcctact cagggctgac 600
atcagggctg gtgcccatgt acgtggggga gattgctccc actcacctgc ggggcgccct 660
ggggacgctc aaccaactgg ccattgttat cggcattctg atcgcccagg tgctgggctt 720
ggagtccctc ctgggcactg ccagcctgtg gccactgctc ctgggcctca cagtgctacc 780
tgccctcctg cagctggtcc tgctgccctt ctgtcccgag agcccccgct acctctacat 840
catccagaat ctcgaggggc ctgccagaaa gagtctgaag cgcctgacag gctgggccga 900
tgtttctgga gtgctggctg agctgaagga tgagaagcgg aagctggagc gtgagcggcc 960
actgtccctg ctccagctcc tgggcagccg tacccaccgg cagcccctga tcattgcggt 1020
cgtgctgcag ctgagccagc agctctctgg catcaatgct gttttctatt attcgaccag 1080
catcttcgag acagcagggg taggccagcc tgcctatgcc accataggag ctggtgtggt 1140
caacacagtc ttcaccttgg tctcggtgtt gttggtggag cgggcggggc gccggacgct 1200
ccatctcctg ggcctggcgg gcatgtgtgg ctgtgccatc ctgatgactg tggctctgct 1260
cctgctggag cgagttccag ccatgagcta cgtctccatt gtggccatct ttggcttcgt 1320
ggcatttttt gagattggcc ctggccccat tccttggttc atcgtggccg agctcttcag 1380
ccagggaccc cgcccggcag ccatggctgt ggctggtttc tccaactgga cgagcaactt 1440
catcattggc atgggtttcc agtatgttgc ggaggctatg gggccctacg tcttccttct 1500
atttgcggtc ctcctgctgg gcttcttcat cttcaccttc ttaagagtac ctgaaactcg 1560
aggccggacg tttgaccaga tctcagctgc cttccaccgg acaccctctc ttttagagca 1620
ggaggtgaaa cccagcacag aacttgagta tttagggcca gatgagaacg actgaggggc 1680
caggcagggg tgggagagcc agctctctct acccggccca gagacccctt cctttcctct 1740
gcagcacttt aaccctctct tccctattat ttccgggtgg aaaagaatcc ctgcagcctg 1800
gtagaattgg gaagctgggg gaagggtggt ctgagcaccc cctcattccc ctcgtgtgac 1860
tctcttggat tatttatgtg ttgtggtttg gccgtggcca tcagggtggg ccactctccc 1920
ctccctcttc cttcccccat cccctttcct ccccaccttc cccagactca gctccagaat 1980
accttcttcg ctgctagaga agggggattg gagggaagac aggtctagac tttctcagtg 2040
ggacaaacca gagcagagag caggacagga gacaagaaat ccagtttccc accaccttgg 2100

actcctccca caatctggga ctttcact 2128

<210> 20

<211> 1496

<212> DNA

<213> Homo sapiens

<400> 20
ggcacgagga aaatcaagat aaaaatgttc acaattaagc tccttctttt tattgttcct 60

ctagttattt cctccagaat tgatcaagac aattcatcat ttgattctct atctccagag 120

ccaaaatcaa gatttgctat gttagacgat gtaaaaattt tagccaatgg cctccttcag 180

ttgggacatg gtcttaaaga ctttgtccat aagacgaagg gccaaattaa tgacatattt 240

caaaaactca acatatttga tcagtctttt tatgatctat cgctgcaaac cagtgaaatc 300

aaagaagaag aaaaggaact gagaagaact acatataaac tacaagtcaa aaatgaagag 360

gtaaagaata tgtcacttga actcaactca aaacttgaaa gcctcctaga agaaaaaatt 420

ctacttcaac aaaaagtgaa atatttagaa gagcaactaa ctaacttaat tcaaaatcaa 480

cctgaaactc cagaacaccc agaagtaact tcacttaaaa cttttgtaga aaaacaagat 540

aatagcatca aagaccttct ccagaccgtg gaagaccaat ataaacaatt aaaccaacag 600

catagtcaaa taaaagaaat agaaaatcag ctcagaagga ctagtattca agaacccaca 660

gaaatttctc tatcttccaa gccaagagca ccaagaacta ctccctttct tcagttgaat 720

gaaataagaa atgtaaaaca tgatggcatt cctgctgaat gtaccaccat ttataacaga 780

ggtgaacata caagtggcat gtatgccatc agacccagca actctcaagt ttttcatgtc 840

tactgtgatg ttatatcagg tagtccatgg acattaattc aacatcgaat agatggatca 900

caaaacttca atgaaacgtg ggagaactac aaatatggtt ttgggaggct tgatggagaa 960

ttttggttgg gcctagagaa gatatactcc atagtgaagc aatctaatta tgttttacga 1020

attgagttgg aagactggaa agacaacaaa cattatattg aatattcttt ttacttggga 1080

aatcacgaaa ccaactatac gctacatcta gttgcgatta ctggcaatgt ccccaatgca 1140

atcccggaaa acaaagattt ggtgttttct acttgggatc acaaagcaaa aggacacttc 1200

aactgtccag agggttattc aggaggctgg tggtggcatg atgagtgtgg agaaaacaac 1260

ctaaatggta aatataacaa accaagagca aaatctaagc cagagaggag aagaggatta 1320

tcttggaagt ctcaaaatgg aaggttatac tctataaaat caaccaaaat gttgatccat 1380

ccaacagatt cagaaagctt tgaatgaact gaggcaaatt taaaaggcaa taatttaaac 1440

attaacctca ttccaagtta atgtggtcta ataatctggt attaaatcct taagag 1496

<210> 21

<211> 1415

<212>  DNA

<213>  Homo sapiens

<400>  21
ggaggaggga gagagagaga agagaagaaa aagaaaaaag aacatcaata aaaagaagtc      60
agatttgttc gaaatcttga ggagtcttca ggccagctcc ctgtcggatg gcttttatga     120
aaaaatatct cctccccatt ctggggctct tcatggccta ctactactat tctgcaaacg     180
aggaattcag accagagatg ctccaaggaa agaaagtgat tgtcacaggg gccagcaaag     240
ggatcggaag agagatggct tatcatctgg cgaagatggg agcccatgtg gtggtgacag     300
cgaggtcaaa agaaactcta cagaaggtgg tatcccactg cctggagctt ggagcagcct     360
cagcacacta cattgctggc accatggaag acatgacctt cgcagagcaa tttgttgccc     420
aagcaggaaa gctcatggga ggactagaca tgctcattct caaccacatc accaacactt     480
ctttgaatct ttttcatgat gatattcacc atgtgcgcaa aagcatggaa gtcaacttcc     540
tcagttacgt ggtcctgact gtagctgcct tgcccatgct gaagcagagc aatggaagca     600
ttgttgtcgt ctcctctctg gctgggaaag tggcttatcc aatggttgct gcctattctg     660
caagcaagtt tgctttggat gggttcttct cctccatcag aaaggaatat tcagtgtcca     720
gggtcaatgt atcaatcact ctctgtgttc ttggcctcat agacacagaa acagccatga     780
aggcagtttc tgggatagtc catatgcaag cagctccaaa ggaggaatgt gccctggaga     840
tcatcaaagg gggagctctg cgccaagaag aagtgtatta tgacagctca ctctggacca     900
ctcttctgat cagaaatcca tgcaggaaga tcctggaatt tctctactca acgagctata     960
atatggacag attcataaac aagtaggaac tccctgaggg ctgggcatgc tgagggattt    1020
tgggactgtt ctgtctcatg tttatctgag ctcttatcta tgaagacatc ttcccagagt    1080
gtccccagag acatgcaagt catgggtcac acctgacaaa tggaaggagt tcctctaaca    1140
tttgcaaaat ggaaatgtaa taataatgaa tgtcatgcac cgctgcagcc agcagttgta    1200
aaattgttag taaacatagg tataattacc agatagttat attaaattta tatcttatat    1260
ataataatat gtgatgatta atacaatatt aattataata aaggtcacat aaactttata    1320
aattcataac tggtagctat aacttgagct tattcaggat ggtttcttta aaaccataaa    1380
ctgtacaaat gaaatttttc aatatttgtt tctta                                1415

<210>  22

<211>  1405

<212>  DNA

<213>  Homo sapiens

<400>  22

acaattcaga ggctgctgcc tgcttaggag gttgtagaaa gctctgtagg ttctctctgt 60
gtgtcctaca ggagtcttca ggccagctcc ctgtcggatg gcttttatga aaaaatatct 120
cctccccatt ctggggctct tcatggccta ctactactat tctgcaaacg aggaattcag 180
accagagatg ctccaaggaa agaaagtgat tgtcacaggg gccagcaaag ggatcggaag 240
agagatggct tatcatctgg cgaagatggg agcccatgtg gtggtgacag cgaggtcaaa 300
agaaactcta cagaaggtgg tatcccactg cctggagctt ggagcagcct cagcacacta 360
cattgctggc accatggaag acatgacctt cgcagagcaa tttgttgccc aagcaggaaa 420
gctcatggga ggactagaca tgctcattct caaccacatc accaacactt ctttgaatct 480
ttttcatgat gatattcacc atgtgcgcaa aagcatggaa gtcaacttcc tcagttacgt 540
ggtcctgact gtagctgcct tgcccatgct gaagcagagc aatggaagca ttgttgtcgt 600
ctcctctctg gctgggaaag tggcttatcc aatggttgct gcctattctg caagcaagtt 660
tgctttggat gggttcttct cctccatcag aaaggaatat tcagtgtcca gggtcaatgt 720
atcaatcact ctctgtgttc ttggcctcat agacacagaa acagccatga aggcagtttc 780
tgggatagtc catatgcaag cagctccaaa ggaggaatgt gccctggaga tcatcaaagg 840
gggagctctg cgccaagaag aagtgtatta tgacagctca ctctggacca ctcttctgat 900
cagaaatcca tgcaggaaga tcctggaatt tctctactca acgagctata atatggacag 960
attcataaac aagtaggaac tccctgaggg ctgggcatgc tgagggattt tgggactgtt 1020
ctgtctcatg tttatctgag ctcttatcta tgaagacatc ttcccagagt gtccccagag 1080
acatgcaagt catgggtcac acctgacaaa tggaaggagt tcctctaaca tttgcaaaat 1140
ggaaatgtaa taataatgaa tgtcatgcac cgctgcagcc agcagttgta aaattgttag 1200
taaacatagg tataattacc agatagttat attaaattta tatcttatat ataataatat 1260
gtgatgatta atacaatatt aattataata aaggtcacat aaactttata aattcataac 1320
tggtagctat aacttgagct tattcaggat ggtttcttta aaaccataaa ctgtacaaat 1380
gaaattttc aatatttgtt tctta 1405